# EUROPEAN PATENT SPECIFICATION

(11) **EP 1 160 233 B1**
(45) Date of publication and mention of the grant of the patent: **21.07.2004**
(21) Application number: 01304815.2
(22) Date of filing: 31.05.2001
(51) Int. Cl.: C07C 43/225, C07C 25/18, C09K 19/12

(54) **Novel terphenyl compounds**
Terphenylverbindungen
Composés de terphényle

(30) Priority: 02.06.2000 GB 0013312
(43) Date of publication of application: 05.12.2001
(73) Proprietor: SHARP KABUSHIKI KAISHA, Osaka 545-8522 (JP)
(72) Inventor: Booth, Christopher James, Reading, Berkshire RG4 6RW (GB); Walton, Emma Jaine, Cowley, Oxford OX4 3NE (GB)
(74) Representative: Robinson, John Stuart

(56) References cited:
- EP-A- 0 360 042
- EP-A- 0 360 043
- DATABASE CA [Online] CHEMICAL ABSTRACTS SERVICE, COLUMBUS, OHIO, US; BOOSE, CHRISTOPHER JAMES ET AL: "Antiferroelectric liquid crystalline display devices, the antiferroelectric substances, their preparation, and compositions thereof" retrieved from STN Database accession no. 132:144480 XP002182660 & JP 2000 035596 A (SHARP CORP., UK) 2 February 2000 (2000-02-02)
- BOOTH C J ET AL: "NOVEL CHIRAL LIQUID CRYSTALS DERIVED FROM (R)-2-(4-HYDROXYPHENOXY)PRO PAN-1-OL" JOURNAL OF MATERIALS CHEMISTRY, CAMBRIDGE, GB, vol. 3, no. 8, 1993, pages 821-832, XP000575417
- DATABASE CA [Online] CHEMICAL ABSTRACTS SERVICE, COLUMBUS, OHIO, US; AZUMAI, TAKAYUKI ET AL: "Preparation of optically active alcohol derivatives" retrieved from STN Database accession no. 124:29778 XP002182661 & JP 07 179380 A (SUMITOMO CHEMICAL CO, JAPAN) 18 July 1995 (1995-07-18)

## Description

This invention relates to novel terphenyl compounds and is more particularly concerned with novel terphenyl compounds which have useful chiral liquid crystalline properties.

Terphenyls and latterly fluoro-substituted terphenyls are known to be effective smectic C materials and are therefore useful components in ferroelectric liquid crystal mixtures.

C. J. Booth et al (J. Mater. Chem. 1993, 3(8), 821) discloses, inter alia, (R)-2-(4"-pentyl-p-terphenyl-4-oxy)-1-methoxypropane), a terphenyl-based compound having at one end of the terphenyl ring system a branched chiral moiety. The compound exhibits liquid crystal behaviour.

EP 0360043 discloses a number of di-fluorinated terphenyl based compounds, only two of which show chiral liquid crystal phases. (R)-4-(1-methylhexyl)oxy-3,3'-difluoro-4"-octyl-*p*-terphenyl exhibits monotropic liquid crystal behaviour whereas (R)-4-(1-methylhexyl)oxy-3,3'-difluoro-4"-octyloxy-*p*-terphenyl exhibits enatiotropic liquid crystal behaviour.

EP 036042 discloses a number of mono-fluorinated terphenyl based compounds having at one end of the terphenyl ring system an alkoxy moiety, and at the other end a chiral methylalkoxy moiety. Several of the compounds exhibit choleteric liquid crystal phases.

It is an object of the present invention to provide novel terphenyl compounds which preferably have useful chiral liquid crystalline properties.

According to the present invention there is provided a terphenyl compound having the general formula [1]:- 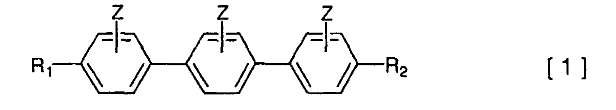 where each of R₁ and R₂ is the same or different and is selected from the group consisting of:- alkyl, alkyloxy, alkenyl, alkenyloxy, alkynyl, alkynyloxy, alkylthio, alkenylthio and alkynylthio 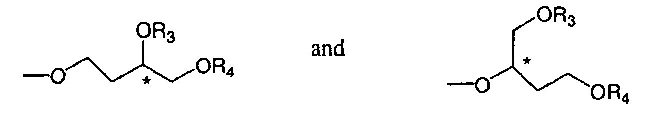 where * indicates an asymmetric carbon atom,
provided that at least one of R₁ and R₂ is 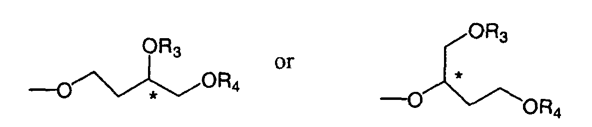 and where each of R₃ and R₄ is the same or different and is an alkyl, alkenyl or alkynyl group; and Z is independently selected from hydrogen and fluorine at each terphenyl carbon.

The group represented by R₁ or R₂ is a C₁- to C₁₆-alkyl or alkyloxy group or a C₂- to C₁₆-alkenyl, alkynyl, alkenyloxy or alkynyloxy group, preferably a C₁- to C₁₆ n-alkyl group, more preferably a C₇- to C₁₂ n-alkyl group.

The group represented by R₃ or R₄ is a C₁- to C₁₆-alkyl group or a C₂- to C₁₆-alkenyl or alkynyl group, and is preferably a C₁- to C₁₆ n-alkyl group, more preferably a C₁- to C₅ n-alkyl group.

Preferably, said compound has the general formula [2] or the general formula [3]:- 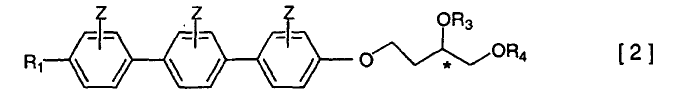 wherein R₁, R₃, R₄ and Z are as defined above. 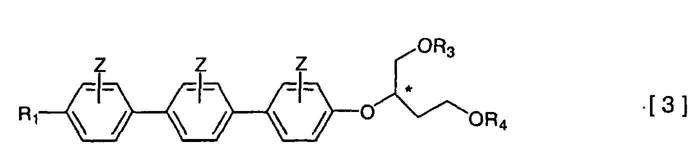 It will be understood that each of the three aromatic rings of the terphenyl moiety may be independently unsubstituted, mono-, di-, tri- or tetra-fluoro substituted. Preferably, each of the three aromatic rings is independently mono-fluoro substituted, di-fluoro substituted or unsubstituted.

Preferably, the compound has the general formula [4] or the general formula [5]:- 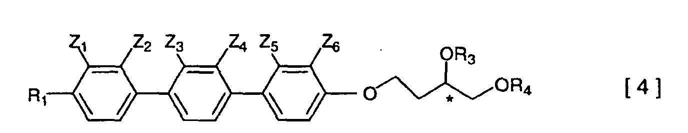 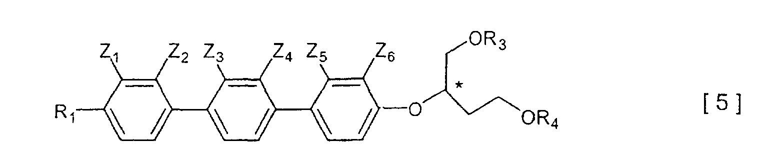 wherein R₁, R₃ and R₄ are as defined above, and either
(i) one of Z₁ to Z₆ is F, the remaining Z₁ to Z₆ being H, or
(ii) Z₁ and Z₂, or Z₃ and Z₄, or Z₅ and Z₆ are F, the remaining Z₁ to Z₆ being H.

In particularly preferred examples of the compound, R₁ is n-octyl and R₃ and R₄ are independently selected from methyl and n-pentyl.

According to a second aspect of the present invention, there is provided a liquid crystal composition containing a compound according to said first aspect of the present invention.

According to a third aspect of the present invention, there is provided an electro-optical liquid crystal device including a liquid crystal cell have a layer of a liquid crystal composition according to said second aspect of the present invention, and means for applying an electrical field across said layer.

### Examples

### (S)-1,2,4-butanetriol as starting material

Successful manipulation of the triols **1a** and **b** involves the use of a suitable protection (and deprotection) step which masks two of the hydroxy groups and thereby allows selective reaction of the remaining unprotected hydroxy moiety. This may be achieved using the acetonide (or isopropylidene ketal) protection group, although this leads to the possibility of two isomeric acetonides being formed, namely the 1,2- (5-membered ring) or 1,3- (6-membered ring), as is shown in **scheme 1.** It is normally the case that the 1,2- acetonide is favoured over the 1,3-acetonide (kinetic and thermodynamic control). Treatment by a known method (H. Hayashi et. al., **J. Am. Chem. Soc.**, 1973, 95, 8749) of *(S)*-1,2,4-butanetriol **(1a)** with anhydrous acetone in the presence of p-toluene sulphonic acid (p-TSA) gave a mixture of the 1,2- (**2b**) and 1,3-acetonide (**2a**) in good yield (73%). Analysis by ¹H NMR revealed a ratio of 96:4 of **(2b)** to (**2a**) by integration of the (CH₃)₂C signals. A similar result was obtained for the racemate (**3a** and **b**). The presence of the 1,3-acetonide proved to be no hindrance as it was easily removed by recrystallisation or column chromatography of later derivatives. Isolation of pure *(S)*-1,2-isopropylidene-1,2,4-butanetriol was not necessary.

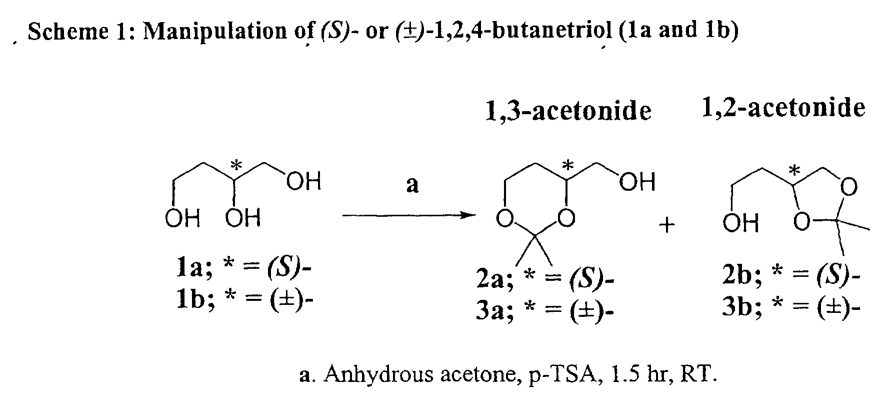

### A. 1,(S)-2-(4"-Octyloxyterphenyl-4-oxy)dialkoxy-butanes (Examples 1 to 3)

### Summary

The route employed is outlined in **scheme 2.** Here 4-bromophenol (**4**) was alkylated with acetonide **2a/b** or **3a/b** using the Mitsunobu reaction (O. Mitsunobu, **Synthesis,** 1981, 1) to give compound **5a** or **b** in 74 % yield (purification by flash chromatography removed all trace of the six membered ring side product). Deprotection of **5a** or **b** using p-TSA in methanol at room temperature gave 4-bromo-1-(1,2-dihydroxybutoxy)benzenes **6a** or **b** in 87 % yield. Compound **6a** or **b** was then alkylated with methyl iodide or 1-bromopentane and excess sodium hydride (D.M. Walba et. al., **J. Am. Chem. Soc.,** 1986, **108**, 5211) to give compounds **7, 8a, 8b, 9** and **10** in yields of between 62-88%. Compound **10** was synthesised from the monopentylated side product **(9)** of the reaction of compounds **6a** to **8a** (showing the greater reactivity of the 1° alcohol site over the 2° alcohol). The final step involved the Suzuki coupling of compounds **7, 8a, 8b** and **10** with 4-(4-octyloxyphenyl)phenylboronic acid **(11)** using palladium(0) *tetrakis*(triphenylphosphine) catalyst, 2M sodium carbonate and 1,2-dimethoxyethane (R.B. Miller et. al., **Organometallics,** 1984, **3**, 1261; M. Hird et. al., **Mol. Cryst. Liq. Cryst.,** 1991, 206, 187; L.K.M. Chan et. al., **Mol.Cryst. Liq. Cryst.,** 1985, **123**, 185; L.K.M. Chan et. al., **Mol. Cryst. Liq. Cryst.,** 1987, **150B**, 335 and **Mol. Cryst. Liq. Cryst.,** 1988, **158B**, 209). Compounds **12 (Example 1), 13a (Example 2), 13b** and **14 (Example 3)** were obtained in 20-55 % yields after rigorous flash chromatography and repeated recrystallisation.

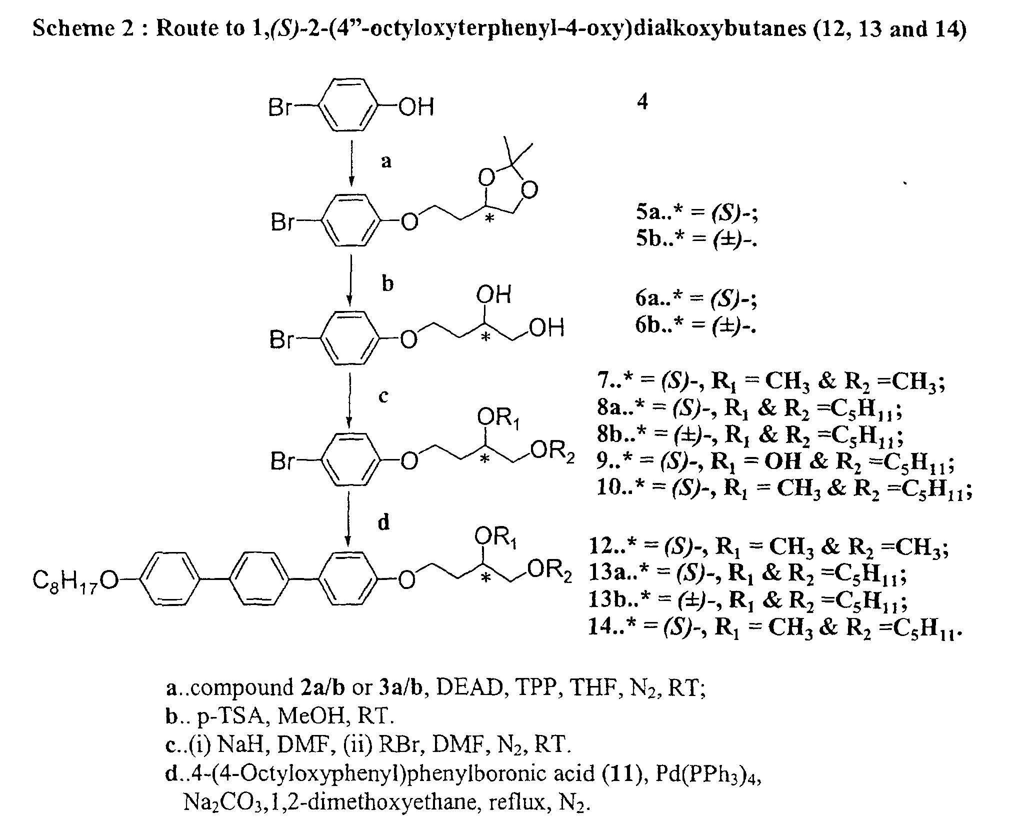

### Experimental details

The structures and purities of all intermediates and final products were confirmed by a combination of NMR spectroscopy (¹H, ¹³C, ¹³C DEPT, ¹⁹F NMR, 2D COSY, CHCORR), FT-IR spectroscopy (ATR or DRIFT sampling), UV-Vis spectroscopy (5-10 mg of sample is dissolved in 50 cm³ of solvent (e.g. CHCl₃), a 1 ml aliquot is then removed and made up to 10 ml), gas chromatography and high performance liquid chromatography. The transition temperatures and associated enthalpies were determined using a combination of optical microscopy and differential scanning calorimetry.

***(S)-1,2,4-Butanetriol (1a)*** ¹H NMR (300MHz; acetone, TMS) 8: 1.66 (2H,m, CH₂CH₂OH), 3.47 (2H,m, diastereotopic protons), 3.72 (2H,m, CH₂OH), 3.80 (1H,m, C*H), 4.12 (3H,m, OH); ¹³C NMR (75MHz; acetone, TMS) δ: 36.92 (CH₂CH₂OH, -ve DEPT), 59.95 (CH₂OH, -ve DEPT), 67.34 (CH₂C*H, -ve DEPT), 70.96 (C*H, +ve DEPT); GC analysis (det 300°C, inj 280 °C, oven 80 °C 5 min 10 °C/min to 280 °C hold 1 min): 98.94% (R_{T} = 10.90 min).

***(S)-1,2-Isopropylidene-1,2,4-butanetriol (2a*/*b)*** *(S)*-1,2,4-Butanetriol (1) (9.02 g, 85.1 mmol) was stirred in acetone (500 ml) with *p*-toluenesulfonic acid (400 mg) for 1½ hours at room temperature. Sodium bicarbonate was added to the mixture and stirred for a further 10 minutes. Acetone was removed by evaporation under reduced pressure. Ethyl acetate (100 ml) was added to the residue, and then the mixture was washed with aqueous sodium bicarbonate (50 ml) and aqueous sodium chloride (50 ml). The mixture was then dried with anhydrous magnesium sulphate, filtered and the solvent removed by evaporation. Kugelrohr distillation (90 °C, 1 mbar) yielded a colourless liquid. Yield = 6.84 g (55%); ¹H NMR (300MHz; CDCl₃, TMS) δ: 1.37 (3H,s, CH₃), 1.43 (3H,s, CH₃), 1.83 (2H,q, CH₂CH₂OH), 2.68 (1H,br t, OH), 3.60 (1H,pseudo t, diastereotopic proton), 3.80 (2H,q, CH₂OH), 4.09 (1H,dd, diastereotopic proton), 4.27 (1H,quintet, C*H) Analysis of peak heights from ¹H NMR showed that the product contained 96% (S)-1,2-isopropylidene-1,2,4-butanetriol and 4% *(S)*-2,4-isopropylidene-1,2,4-butanetriol; ¹³C NMR (75MHz; CDCl₃, TMS) δ: 25.69 (CH₃, +ve DEPT), 26.89 (CH₃, +ve DEPT), 35.65 (CH₂CH₂OH, -ve DEPT), 60.48 (CH₂C*H, -ve DEPT), 69.46 (CH₂OH, -ve DEPT), 75.03 (C*H, +ve DEPT), 109.08 (C(CH₃)₂, no DEPT); FT-IR (film; ATR/ZnSe) νₘₐₓ: 3417, 2992, 2939, 2873, 1381, 1209, 1156, 1050, 871cm⁻¹.

***(±)-1,2-Isopropylidene-1,2,4-butanetriol (3a*/*b)*** Was obtained in exactly the same way as **2a/b** using (+)-1,2,4-butanetriol **1b** as starting material. Yield = 9.08 g (73%); ¹H NMR (300MHz; CDCl₃, TMS) δ: 1.36 (3H,s, CH₃), 1.42 (3H,s, CH₃), 1.81 (2H,q, CH₂CH₂OH), 2.94 (1H,br t, OH), 3.59 (1H,pseudo t, diastereotopic proton), 3.76 (2H,q, CH₂OH), 4.09 (1H,dd, diastereotopic proton), 4.26 (1H,quintet, CH) Analysis of peak heights from ¹H NMR showed that the product contained 96% (±)-1,2-isopropylidene-1,2,4-butanetriol and 4% (+)-2,4-isopropylidene-1,2,4-butanetriol; FT-IR (film; ATR/ZnSe) νₘₐₓ: 3423, 2985, 2938, 2878, 1370, 1244, 1214, 1157, 1052, 854 cm⁻¹.

***1-{(2,2-Dimethyl)-1,(S)-2-dioxolan-4-yl}ethoxy-4-bromobenzene (5a)*** Compound **2a/b** (9.07 g, 62.1 mmol), triphenylphoshine (10.83 g, 41.3 mmol) and dry tetrahydrofuran (100 ml) was added to a stirred mixture of 4-bromophenol (4) (7.16 g, 41.3 mmol), diethylazodicarboxylate (7.20 g, 41.3 mmol) and dry tetrahydrofuran (45 ml) at RT under dry nitrogen, stirred for 18 h, before being diluted with diethyl ether (150 ml) and then washed with brine (50 ml). The organic phase was dried (MgSO₄), filtered and evaporated to give a colourless oil. Purification by flash chromatography [fine mesh silica gel: 8% ethyl acetate in hexanes] gave a colourless oil. Yield = 9.27 g (74%); ¹H NMR (300MHz; CDCl₃, TMS) δ: 1.36 (3H,s. CH₃CH), 1.42 (3H,s, CH₃CH), 2.03 (2H,m, OCH₂CH₂CH), 3.64 (1H,dd, CHCH₂O), 4.05 (2H,m, OCH₂CH₂), 4.11 (1H,dd, CHCH₂O), 4.28 (1H,quint, CH₂C*HCH₂), 6.77 (2H,d, Ar-H₂, ³J_{HH} = 9.05 Hz), 7.36 (2H, d, Ar-H₃, ³J_{HH} = 7.36 Hz); ¹³C NMR (75MHz; CDCl₃, TMS) δ: 25.70, 26.95 (CH₃CH, +ve DEPT), 33.38 (OCH₂CH₂CH, -ve DEPT), 64.85 (OCH₂CH₂CH, -ve DEPT), 69.48 (CHCH₂O,-ve DEPT), 73.27 (C*HCH₂O, +ve DEPT), 108.83 ((CH₃)₂C, no DEPT), 112.87 (Ar-C₄, no DEPT), 116.20 (Ar-C₂, +ve DEPT), 132.22 (Ar-C₃, +ve DEPT), 157.83 (Ar-C₁, no DEPT); FT-IR (film; ATR/ZnSe) νₘₐₓ: 2986, 2939, 2873, 1488, 1472, 1369, 1240, 1171, 1064, 820 cm⁻¹. GC Analysis (inj 250 °C; det 300 °C; oven 80 °C hold 1 min, 30 °C/min to 280 °C hold 2 min) R_{T} = 7.21 (99.75 %) min.

***1-{(2,2-Dimethyl)-1,(R,S)-2-dioxolan-4-yl}ethoxy-4-bromobenzene (5b)*** A similar procedure to that used for compound **5a** was used. Quantities used: compound **3a/b** (9.00 g, 61.6 mmol), triphenylphosphine (10.78 g, 41.4 mmol), 4-bromophenol (**4**) (7.11 g, 41.4 mmol), diethylazodicarboxylate (7.16 g, 41.4 mmol) and tetrahydrofuran (160 ml). Purification by flash chromatography [fine mesh silica gel: 8% ethyl acetate in hexane] gave a colourless oil which was dried *in vacuo* (P₂O₅, 0.5 mbar, RT, 16 h). Yield = 4.55 g (37%); ¹H NMR (300MHz, CDCl₃, TMS) δ: 1.36 (3H, s, O₂CCH₃), 1.42 (3H, s, O₂CCH₃), 2.03 (2H, dd, ArOCH₂CH₂^{(±)}CH), 3.63 (1H, t, ^{(±)}CHCH₂OC), 4.03 (2H, m, ArOCH₂CH₂^{(±)}CH), 4.11 (1H, dd, ^{(±)}CHCH₂OC), 4.28 (1H, q, ArOCH₂CH₂^{(±)}CH), 6.76 (2H, dd, ArH₂/H₆, ³J_{HH} = 6.80 Hz), 7.35 (2H, dd, ArH₃/H₅, ³J_{HH} = 6.76 Hz). ¹³C NMR (75MHz, CDCl₃, TMS) δ: 25.8 (O₂CCH₃, +ve DEPT), 27.0 (O₂CCH₃, +ve DEPT), 33.4 (ArOCH₂CH₂^{(±)}CH, -ve DEPT), 64.9 (ArOCH₂CH₂^{(±)}CH, -ve DEPT), 69.5 (^{(±)}CHCH₂OC, -ve DEPT), 73.3 (ArOCH₂CH₂^{(±)}CH, +ve DEPT), 108.8 (O₂C(CH₃)₂, no DEPT), 112.9 (Ar-C₄, no DEPT), 116.3 (Ar-C₂/C₆, no DEPT), 132.3 (Ar-C₃/C₅, no DEPT), 157.8 (Ar-C₁, no DEPT). FT/IR (film/ATR/ZnSe) µₘₐₓ: 2984, 2933, 2877, 1488, 1472, 1369, 1285, 1240, 1171, 1158, 1059, 1002, 820 cm⁻¹.

***4-Bromo-1-(1,(S)-2-dihydroxybutoxy)benzene (6a)*** p-Toluenesulfonic acid (500 mg) was added to a stirred solution of compound **5a** (9.16 g, 30.4 mmol) in methanol (460 ml) and left to stir for 18 h at room temperature. The reaction was quenched by addition of sodium bicarbonate (3.00 g) and stirred for a further 10 mins. The methanol was removed by evaporation under reduced pressure and the residue redissolved in ethyl acetate (200 ml). The organic solution was then washed with water (50 ml), dried (MgSO₄), filtered and evaporated to give a colourless solid, recrystallised (ethanol) and dried *in vacuo* (P₂O₅, 20 mbar, 50 °C, 18 h). Yield = 6.88 g (87 %). ¹H NMR (300MHz; CDCl₃, TMS) δ: 1.92 (2H,m, OCH₂CH₂CH), 2.47 (1H,s broad, CHCH₂OH), 2.88 (1H,s broad, CH₂CH(OH)CH₂), 3.53 (1H,dd, CHCH₂OH, J = 11.15 and 7.10 Hz), 3.71 (1H,dd, CHCH₂OH, J = 11.15 and 2,72 Hz), 3.99 (1H,m CH₂C*HCH₂), 4.09 (2H,m, OCH₂CH₂CH), 6.78 (2H,d, Ar-H₂, ³J_{HH} = 9.05 Hz), 7.37 (2H,d, Ar-H₃, ³J_{HH} = 8.98 Hz). ¹³C NMR (75MHz; CDCl₃, TMS) δ: 32.41 (OCH₂CH₂CH, -ve DEPT), 65.22 (OCH₂CH₂CH, -ve DEPT), 66.69 (CHCH₂OH, -ve DEPT), 69.93 (CH₂C*HCH₂, +ve DEPT), 113.14 (Ar-C₄, no DEPT), 116.25 (Ar-C₂, +ve DEPT), 132.31 (Ar-C₃, +ve DEPT), 157.66 (Ar-C₁, no DEPT). FT-IR(KBr/DRIFT) νₘₐₓ: 3390, 3304, 2959, 2926, 2873, 1490, 1458, 1289, 1248, 1239, 1121, 1060, 971, 822 cm⁻¹.

***4-Bromo-1-(1,(R,S)-2-dihydroxybutoxy)benzene (6b)*** This compound was prepared in a manner similar to compound **6a.** Quantities used: p-toluenesulfonic acid (500 mg), compound **5b** (4.30 g, 14.3 mmol), in methanol (460 ml). Purified by flash chromatography [fine mesh silica gel: dichloromethane (initially) then 1:1 dichloromethane to ethyl acetate]. Dried *in vacuo* (P₂O₅, 0.5 mbar, RT, 16 h), to give a white crystalline solid. Yield = 2.75 g (74%); ¹H NMR (300MHz, CDCl₃, TMS) δ: 1.87 (2H, m, ArOCH₂CH₂^{(±)}CH), 3.01 (1H, broad s, CHOH), 3.31 (1H, broad s, CHCH₂OH), 3.5 (1H, t, ^{(±)}CHCH₂OH), 3.63 (1H, d, ^{(±)}CHCH₂OH), 3.96 (1H, m, ArOCH₂CH₂^{(±)}CH), 4.06 (2H, m, ArOCH₂CH₂^{(±)}CH), 6.76 (2H, dd, ArH₂/H₆, ³J_{HH} = 6.74 Hz), 7.35 (2H, dd, ArH₃/H₅, ³J_{HH} = 6.9 Hz). FT/IR (KBr/Drift) µₘₐₓ: 3277, 2939, 2880, 1593, 1492, 1471, 1291, 1249, 1102, 1079, 1052, 984, 971, 827, 812, 647cm⁻¹.

***4-Bromo-1-(1,(S)-2-dimethoxybutoxy)benzene (7)*** A solution of methyl iodide (3.21 g, 22.6 mmol) in dry DMF (5 ml) was added dropwise to a stirred mixture of compound **6a** (2.03 g, 7.8 mmol), sodium hydride (1.19 g, *ca.* 29 mmol of a 60% dispersion in mineral oil) and dry DMF (20 ml) at room temperature under dry nitrogen. After a slight exotherm had subsided, the reaction was left to stir at RT for a further 18 h, after which TLC showed no starting material to have remained. The excess sodium hydride was destroyed by the cautious dropwise addition of water (50 ml). The product was then extracted using diethyl ether (5 x 50 ml). The combined ether extracts were then washed successively with sat. sodium bicarbonate (50 ml), 10 % v/v hydrochloric acid (50 ml) and water (50 ml) before being dried (MgSO₄), filtered and evaporated to give a colourless oil. Purification by flash chromatography [fine mesh silica gel: dichloromethane] gave a colourless oil which was dried *in vacuo* (P₂O₅, 10 mbar, RT, 18h), Yield = 1.97 g (88%). ¹H NMR (300MHz; CDCl₃, TMS) δ: 1.98 (2H,m, OCH₂CH₂CH), 3.39 (3H,s, CH₂OCH₃), 3.41 (3H,s, CHOCH₃), 3.47 (2H,m, CHCH₂OCH₃), 3.57 (1H,m, C*H), 4.02 (2H,m, OCH₂CH₂CH), 6.78 (2H,d, Ar-H₂, ³J_{HH} = 8.96 Hz), 7.36 (2H,d, Ar-H₃, ³J_{HH} = 8.88 Hz). ¹³C NMR (75MHz; CDCl₃, TMS) δ: 31.26 (OCH₂CH₂CH, -ve DEPT), 57.79 (CH₂OCH₃, +ve DEPT), 59.29 (CHOCH₃, +ve DEPT), 64.47 (OCH₂CH₂CH, -ve DEPT), 74.28 (CH₂OCH₃, -ve DEPT), 76.76 (C*H, +ve DEPT), 112.72 (Ar-C₄, no DEPT), 116.28 (Ar-C₂, +ve DEPT), 132.21 (Ar-C₃, +ve DEPT), 158.01 (Ar-C₁, no DEPT). FT-IR (film; ZnSe/ATR) νₘₐₓ: 2927,2879, 2833, 1488, 1473, 1240, 1131, 1101, 1071, 1044, 1001, 820 cm⁻¹. GC Analysis (inj 250 °C; det 300 °; oven 80 °C hold 1 min, 30 °C min⁻¹ to 280 °C hold 2 min) R_{T} = 6.85 (100 %) min.

***4-Bromo-1-(1,(S)-2-dipentoxybutoxy)benzene (8a)*** A similar procedure to that employed for compound **7** was used. Quantities used: compound **6a** (2.01 g, 7.7 mmol), 1-bromopentane (3.42 g, 22.6 mmol), sodium hydride (1.36 g, *ca.* 34 mmol of 60 % dispersion in oil) and dry DMF (25 ml). Worked up reaction mixture was purified by flash chromatography [fine mesh silica gel: dichloromethane] to give two fractions as colourless oils. **8a** (R_{f} CH₂Cl₂ = 0.64) Yield = 1.90 g (62%) and **9** (R_{f}CH₂Cl₂ = 0.19) Yield = 0.50 g. Both dried *in vacuo* (P₂O₅, 10 mbar, 20 °C, 12 h). Compound **8a** ¹H NMR (300MHz; CDCl₃, TMS) δ: 0.85-0.89 (6H,t overlapping, CH₃C₄O), 1.29 (8H,m, CH₃CH₂CH₂C₂O), 1.55 (4H,m , C₃CH₂CH₂O), 1.96 (2H,m, OCH₂CH₂CH), 3.41 (1H,m, CHCH₂OC₅), 3.46 (4H,m, OCH₂C₄), 3.64 (2H,m, CH₂C*HCH₂ & CHCH₂O), 4.05 (2H,m, ArOCH₂CH₂), 6.78 (2H,d, Ar-H₂, ³J_{HH} = 9.07 Hz), 7.36 (2H, d, Ar-H₃, ³J_{HH} = 8.93 Hz). ¹³C NMR (75MHz; CDCl₃, TMS) δ: 14.03 (CH₃C₄OCH₂, +ve DEPT), 14.07 (CH₃C₄OCH, +ve DEPT), 22.49, 22.53 (CH₃CH₂C₃O, -ve DEPT), 28.32, 29.37, 29.80 (CH₂'s, ve DEPT), 31.94 (OCH₂CH₂CH, -ve DEPT), 64.59 (OCH₂CH₂CH, -ve DEPT), 70.45, CHCH₂OC₅, -ve DEPT), 71.68 (CHCH₂OCH₂C₄, -ve DEPT), 73.12 (CHOCH₂C₄, -ve DEPT), 75.11 (C*H, +ve DEPT), 112.61 (Ar-C₄, no DEPT), 116.26 (Ar-C₂, +ve DEPT), 132.18 (Ar-C₃, +ve DEPT), 158.11 (Ar-C₁, no DEPT). FT-IR (film; ZnSe/ATR) νₘₐₓ: 2954, 2929, 2858, 1489, 1467, 1243, 1109, 1102, 820 cm⁻¹. GC Analysis (inj 250 °C; det 300 °C; oven 80 °C hold 1 min, 30 °C min⁻¹ to 280 °C hold 5 min) R_{T} = 9.60 (99.42 %) min. ***4-Bromo-1-(1-pentoxy-(S)-2-hydroxybutoxy)benzene (9)*** ¹H NMR (300MHz; CDCl₃, TMS) δ: 0.90 (3H,t, CH₃C₄O), 1.32 (4H,m, CH₃CH₂CH₂C₂O), 1.59 (2H,m, OCH₂CH₂C₃), 1.90 (2H,m, OCH₂CH₂CH), 2.56 (1H,s broad, CH₂CH(OH)CH₂), 3.34 (1H,m, CHCH₂OC₅), 3.49 (3H,m, CHCH₂OC₅), 4.06 (1H,m, C*HOH), 4.09 (2H,m, OCH₂CH₂CH), 6.79 (2H,d, Ar-H₂, ³J_{HH} = 8.91 Hz), 7.36 (2H,d, Ar-H₃, ³J_{HH} = 8.98 Hz). ¹³C NMR (75MHz; CDCl₃, TMS) δ: 14.05 (CH₃C₄O, +ve DEPT), 22.52, 28.28 (CH₃CH₂CH₂, -ve DEPT), 29.32 (CH₃CH₂CH₂CH₂, -ve DEPT), 32.70 (OCH₂CH₂CH, -ve DEPT), 64.94 (OCH₂CH₂CH, -ve DEPT), 67.69 (C*HOH, +ve DEPT), 71.57 (CH₂OCH₂C₄, -ve DEPT), 74.78 (CH₂OC₅, -ve DEPT), 112.83 (Ar-C₄, no DEPT), 116.27 (Ar-C₂, +ve DEPT), 132.27 (Ar-C₃, +ve DEPT), 157.90 (Ar-C₁, no DEPT). FT-IR (film; ZnSe/ATR) νₘₐₓ: 3430, 2954, 2930, 2868, 2861, 1488, 1473, 1241, 1112, 1102, 1071, 1003, 820 cm⁻¹. GC Analysis (inj 250 °C; det 300 °C; oven 80 °C hold 1 min, 30 °C min⁻¹ to 280 °C hold 5 min) R_{T} = 8.46,(100 %) min.

***4-Bromo-1-(1,(R,S)-2-dipentoxybutoxy)benzene (8b)*** This compound was prepared in a manner similar to compound **8a.** Quantities used: 1-bromopentane (4.34 g, 9.6 mmol), compound **7b** (2.50 g, 9.6 mmol), sodium hydride (1.92 g, 60% dispersion in oil, *ca.,* 48 mmol) and dimethylformamide (40 ml). This was purified by flash chromatography [fine mesh silica gel: dichloromethane] to give a colourless oil. Dried *in vacuo* (P₂O₅, 0.6 mbar, RT, 16 h). Yield = 2.35 g (61 %); ¹H NMR (300 MHz, CDCl₃, TMS) δ: 0.87 (6H, m, OC₄CH₃'s), 1.30 (8H, m, OC₂CH₂CH₂CH₃'s), 1.55 (4H, m, OCH₂CH₂C₃'s), 1.90 (1H, m, ArOCH₂CH₂^{(±)}CH), 1.99 (1H, m, ArOCH₂CH₂^{(±)}CH), 3.44 (4H + 1H, m, OCH₂C₄'s + ^{(±)}CHCH₂O), 3.63 (1H + 1H, m, ArOCH₂CH₂^{(±)}CH + ^{(±)}CHCH₂O), 4.05 (2H, m, ArOCH₂CH₂^{(±)}CH), 6.78 (2H, dd, ArH₂/H₆, ³J_{HH} = 6.78 Hz), 7.36 (2H, dd, ArH₃/H₅, ³J_{HH} = 6.8 Hz). FT/IR (film/ATR/ZnSe) µₘₐₓ: 2954, 2929, 2858, 1591, 1489, 1467, 1285, 1243, 1171, 1105, 1072, 1002, 820 cm⁻¹.

***4-Bromo-1-(1-pentoxy-(S)-2-methoxybutoxy)benzene (10)*** A similar procedure to that employed in the preparation of compound **7** was used. Quantities used: compound **6a** (0.44 g, 1.33 mmol), methyl iodide (1.14 g, 8.1 mmol), sodium hydride (0.24 g, *ca.* 6 mmol of a 60 % dispersion in oil) and dry DMF (10 ml). The crude product was purified by flash chromatography [fine mesh silica gel: dichloromethane] to give a colourless oil which was dried *in vacuo* (P₂O₅, 10 mbar, RT, 18h). Yield = 0.38 g (83%). ¹H NMR (300MHz; CDCl₃, TMS) δ: 0.89 (3H,t, CH₃C₄O), 1.31 (4H,m, CH₃CH₂CH₂C₂O), 1.58 (2H,m, C₃CH₂CH₂O), 1.97 (2H,m, OCH₂CH₂CH), 3.41 (3H,s, OCH₃), 3.47 (4H,m, CH₂OCH₂C₄), 3.56 (1H,m, C*H), 4.02 (2H,m, OCH₂CH₂CH), 6.79 (2H,d, Ar-H₂, ³J_{HH} = 8.96 Hz), 7.36 (2H,d, Ar-H₃, ³J_{HH} = 8.99 Hz). ¹³C NMR (75MHz; CDCl₃, TMS) δ: 14.06 (CH₃C₄O, +ve DEPT), 22.53, 28.30, 29.34 (CH₂'s, -ve DEPT), 31.50 (OCH₂CH₂CH, -ve DEPT), 57.90 (CH₃O, +ve DEPT), 64.57 (OCH₂CH₂CH, -ve DEPT), 71.74. 72.54 (CH₂OCH₂C₄, -ve DEPT), 76.61 (C*H, +ve DEPT), 112.69 (Ar-C₄, no DEPT), 116.30 (Ar-C₂, +ve DEPT), 132.21 (Ar-C₃, +ve DEPT), 158.05 (Ar-C₁, no DEPT). FT-IR (film; ZnSe/ATR) νₘₐₓ: 2954, 2930, 2861, 1488, 1473, 1466, 1242, 1101, 1072, 820 cm⁻¹. GC Analysis (inj 250 °C; det 300 °C; oven 80 °C hold 1 min, 30 °C min⁻¹ to 280 °C hold 5 min) R_{T} = 8.26 (100 %) min.

### EXAMPLE 1

***1,2-(S)-Dimethoxy-(4"-octyloxyterphenyl-4-oxy)butane (12)*** 4-(4-Octyloxyphenyl)phenylboronic acid (**11**) (1.47 g, 4.5 mmol) was added to a stirred mixture of compound **7** (1.00 g, 3.5 mmol), palladium(0) *tetrakis*(triphenylphosphine) (0.12 g, 0.1 mmol), 2M sodium carbonate (25 ml) and 1,2-dimethoxyethane (25 ml). The reaction was then heated under gentle reflux for 6 h under a stream of dry nitrogen gas. The cooled reaction mixture was then poured onto water (150 ml) and the products extracted using diethyl ether (4 x 70 ml). The combined ether extracts were then washed with brine (50 ml), dried (MgSO₄), filtered and evaporated to give a dark solid. This was then absorbed on silica gel and passed through a short silica gel column using 4:1 dichloromethane-ethyl acetate to remove front and baseline material. The colourless solid obtained was then purified rigorously by flash chromatography [fine mesh silica gel: dichloromethane] and repeated recrystallisation (toluene x 5). The colourless solid was dried *in vacuo* (P₂O₅, 10 mbar, 50 °C, 8 h). Yield = 0.33 g (15%). ¹H NMR (300MHz; CDCl₃, TMS) δ: 0.89 (3H,t, CH₃C₇O), 1.32 (8H,m CH₂'s), 1.48 (2H,m, CH₂CH₂CH₂O), 1.81 (2H,m, CH₂CH₂O), 2.03 (2H,m, OCH₂CH₂CH), 3.41 (3H,s, CH₃O), 3.44 (3H,s, CH₂OCH₃), 3.50 (2H,m, CH₂OCH₃), 3.63 (1H,m, C*H), 4.00 (2H,t, OCH₂C₇), 4.11 (2H,m, OCH₂CH₂CH), 6.98, 6.99 (4H,d, Ar-H_{3' or 3"}, ³J_{HH} = 8.75 and 8.79 Hz), 7.55, 7.56 (4H,2d, Ar-H_{2' or 2"}, ³J_{HH} = 8.74 Hz and 8.72 Hz), 7.60 (4H,s, Ar-H_{2/3}). ¹³C NMR (75MHz; CDCl₃, TMS) δ: 14.13 (CH₃C₇O, +ve DEPT), 22.68, 26.08, 29.27, 29.30, 29.38, (CH₂'s, -ve DEPT), 31.36 (OCH₂CH₂CH, -ve DEPT), 31.83 (CH₂, -ve DEPT), 57.83 (CH₃OCH, +ve DEPT), 59.31 (CH₃OCH₂, +ve DEPT), 64.31 (OCH₂CH₂CH, -ve DEPT), 68.10 (C₇CH₂O, -ve DEPT), 74.40 (CH₂OCH₃, -ve DEPT), 76.91 (C*H, +ve DEPT), 114.81 (d, Ar-C_{3' or 3",} +ve DEPT), 126.97 (s, Ar-C_{2/3}, +ve DEPT), 127.94, 127.98 (Ar-C₂_{'or2",} +ve DEPT), 133.04, 133.27 (Ar-C _{1' or 1"}, no DEPT), 139.05, 139.17 (Ar-C_{1 or} _{4,} no dept), 158.47, 158.72 (Ar-C_{4'} or _{4"}, no DEPT). FT-IR (KBr/DRIFT) νₘₐₓ: 2926, 2860, 1605, 1493, 1468, 1288, 1249, 1186, 1139, 1113, 970, 819, 809, 506 cm⁻¹. UV-Vis (6.43 mg, CHCl₃) λₘₐₓ: 294.00 (1.1201) nm. HPLC analysis (C18 column; 1 ml min⁻¹ 99.5:0.5 acetonitrile-tetrahydrofuran) RT = 10.05 (99.78%) min.

### EXAMPLE 2

***1,2-(S)-Dipentoxy(4"-octyloxyterphenyl-4-oxy)butane (13a)*** A similar method to that employed for compound **12** was used. Quantities used: compound **11** (1.49 g, 4.6 mmol), compound **8a** (1.40 g, 3.5 mmol), palladium(0) *tetrakis*(triphenylphosphine) (0.18 g, 0.18 mmol), 2M sodium carbonate (30 ml) and 1,2-dimethoxyethane (30 ml). Crude product was first passed through a short column [silica gel: 9:1 dichloromethane-ethyl acetate], before being purified by flash chromatography (twice) [fine mesh silica gel: 1:1 dichloromethane-hexanes] to give a colourless solid which was recrystallised (toluene x 3). Dried *in vacuo* (P₂O₅, 10 mbar, 50 °C, 6 h). Yield = 0.56 g (27%). ¹H NMR (300MHz; CDCl₃, TMS) δ: 0.88 (9H,m, CH₃'s), 1.31 (16H,m, CH₂'s), 1.47 (2H,m, C₅CH₂CH₂CH₂O), 1.55 (4H,m, OCH₂CH₂C₃), 1.81 (2H,m, OCH₂CH₂C₆), 2.00 (2H,m, OCH₂CH₂CH), 3.43 (1H,m, CHCH₂O), 3.49 (4H,m, OCH₂C₄), 3.65 (1H,m, CHCH₂O), 3.71 (1H,m, C*H), 3.99 (2H,t, ArOCH₂C₇), 4.12 (2H,m, OCH₂CH₂CH), 6.98 (2H,d, Ar-H_{3'/3"}, ³J_{HH} = 8.78 Hz), 6.99 (2H,d, Ar-H_{3'/3"}, ³J_{HH} = 8.77 Hz), 7.55 (4H,d, Ar-H_{2'/2"}, ³J_{HH} = 8.75 Hz), 7.60 (4H,s, Ar-H_{2/3}). ¹³C NMR (75MHz; CDCl₃, TMS) δ: 14.08, 14.06 (CH₃C₄O, +ve DEPT), 14.13 (CH₃C₇O, +ve DEPT), 22.52, 22.67 (CH₂'s, -ve DEPT), 26.07 (ArOCH₂CH₂CH₂C₅, -ve DEPT), 28.34, 29.27, 29.30, 29.39, 29.84, 31.83, 32.05 (CH₂'s, -ve DEPT), 64.43 (OCH₂CH₂CH, -ve DEPT), 68.08 (ArOCH₂C₇, -ve DEPT), 70.49 (CH₂OC₅, -ve DEPT), 71.67, 73.22 (OCH₂C₄, -ve DEPT), 75.25 (C*H, +ve DEPT), 114.79 (Ar-C_{3'/3"}, +ve DEPT), 126.96 (Ar-C_{2/3}, +ve DEPT), 127.93 (Ar-C_{2'/2"}, +ve DEPT), 133.04, 133.13 (Ar-C_{1'/1"}, no DEPT), 139.10 (Ar-C_{1/4}, no DEPT), 158.58, 158.70 (Ar-C_{4'/4"}, no DEPT). FT-IR (KBr/DRIFT) νₘₐₓ: 2955, 2934, 2856, 1607, 1494, 1475, 1468, 1293, 1261, 1252, 1188, 1117, 1096, 998, 822, 813, 503 cm⁻¹. UV-Vis (7.34 mg, CHCl₃) λₘₐₓ: 290.50 (1.0240) nm. HPLC analysis (C₁₈ column; 1 ml min⁻¹ 90:10 acetonitrile-tetrahydrofuran) R_{T} = 19.27 (99.84%) min.

***1,2-(R,S)-Dipentoxy(4"-octyloxyterphenyl-4-oxy)butane (13b)*** A similar method to that employed for compound **12** was used. Quantities used: compound **11** (2.00 g, 6.1 mmol), compound **8b** (1.64 g, 4.09 mmol), palladium(0) *tetrakis*(triphenylphosphine) (0.16 g, 0.14 mmol), 2M sodium carbonate (20 ml) and 1,2-dimethoxyethane (30 ml). Yield = 0.56 g (27%). ¹H NMR (300MHz; CDCl₃, TMS) δ: 0.88 (9H,m, CH₃'s), 1.31 (16H,m, CH₂'s), 1.46 (2H,m, C₅CH₂CH₂CH₂O), 1.57 (4H,m, OCH₂CH₂C₃), 1.80 (2H,m, OCH₂CH₂C₆), 1.95, 2.03 (2H,m, OCH₂CH₂CH), 3.46 (1H,m, CHCH₂O), 3.46 (4H,m, OCH₂C₄), 3.64 (1H,m, CHCH₂O), 3.70 (1H,m, C*H), 3.99 (2H,t, ArOCH₂C₇), 4.14 (2H,m, OCH₂CH₂CH), 6.97 (2H,d, Ar-H_{3'/3"}, ³J_{HH} = 8.79 Hz), 6.98 (2H,d, Ar-H_{3'/3"}, ³J_{HH} = 8.78 Hz), 7.55 (4H,d, Ar-H_{2'/2"}, ³J_{HH} = 8.74 Hz), 7.59 (4H,s, Ar-H_{2/3}). ¹³C NMR (75MHz; CDCl₃, TMS) δ: 14.05, 14.07 (CH₃C₄O, +ve DEPT), 14.12 (CH₃C₇O, +ve DEPT), 22.52, 22.55, 22.68 (CH₂'s, -ve DEPT), 26.08 (ArOCH₂CH₂CH₂C₅, -ve DEPT), 28.34, 28.35, 29.27, 29.31, 29.39, 29.85, 31.83, 32.06 (CH₂'s, -ve DEPT), 64.44 (OCH₂CH₂CH, -ve DEPT), 68.08 (ArOCH₂C₇, -ve DEPT), 70.48 (CH₂OC₅, -ve DEPT), 71.68 (OCH₂C₄, -ve DEPT), 75.26 (C*H, +ve DEPT), 114.80 (Ar-C_{3'/3"}, +ve DEPT), 126.95 (Ar-C_{2/3}, +ve DEPT), 127.93 (Ar-C_{2'/2"}, +ve DEPT), 133.04, 133.14 (Ar-C_{1'/1"}, no DEPT), 139.10 (Ar-C_{1/4}, no DEPT), 158.58, 158.71 (Ar-C_{4'/4"}, no DEPT). FT-IR (KBr/DRIFT) νₘₐₓ: 3039, 2955, 2933, 2858, 1607, 1491, 1473, 1468, 1292, 1252, 1186, 1116, 821, 811, 738, 672, 505 cm⁻¹. UV-Vis (5.00 mg, CHCl₃) λₘₐₓ: 294.50 (0.7719) nm. HPLC analysis (C₁₈ column; 1 ml min⁻¹ 90:10 acetonitrile-tetrahydrofuran) R_{T} = 18.75 (99.86%) min.

### EXAMPLE 3

***1-Pentoxy-2-(S)-methoxy-(4"-octyloxyterphenyl-4-oxy)butane (14)*** A similar method to that employed for compound **12** was used. Quantities used: compound **11** (0.45g, 1.4 mmol), compound **10** (0.34 g, 1.0 mmol), palladium(0) *tetrakis*(triphenylphosphine) (0.04 g, 0.03 mmol), 2M sodium carbonate (10 ml) and 1,2-dimethoxyethane (10 ml). The crude product was passed through a short column [silica gel: dichloromethane] before being purified by flash chromatography [fine mesh silica gel: 7:3 dichloromethane-hexanes] to give a colourless solid which was recrystallised (toluene x 2) and dried *in vacuo* (P₂O₅, 10 mbar, 50 °C, 6 h). Yield = 0.30 g (55 %). ¹H NMR (300MHz; CDCl₃, TMS) δ: 0.89 (3H,m, CH₃), 0.90 (3H,m, CH3), 1.33 (12H,m, CH₂'s), 1.48 (2H,m, C₅CH₂CH₂CH₂O), 1.60 (2H,m, OCH₂CH₂C₃), 1.81 (2H,m, OCH₂CH₂C₆), 2.02 (2H,m, OCH₂CH₂CH), 3.44 (3H,s, CH₃O), 3.53 (4H,m, CH₂OCH₂C₄), 3.62 (1H,m, CH₂CHCH₂O), 4.00 (2H,t, OCH₂C₇), 4.12 (2H,m, OCH₂CH₂CH), 6.98, 6.99 (4H,dd, Ar-H_{3'/3"}, ³J_{HH} = 8.77 and 8.85 Hz), 7.55, 7.57 (4H,dd, Ar-H_{2'/2"}, ³J_{HH} = 8.78 and 8.78 Hz), 7.60 (4H,s, Ar-H_{2/3}). ¹³C NMR (75MHz; CDCl₃, TMS) δ: 14.07 (CH₃C₄O, +ve DEPT), 14.13 (CH₃C₇O, +ve DEPT), 22.54, 22.68 (CH₂'s, -ve DEPT), 26.07 (CH₂, -ve DEPT), 28.31, 29.26, 29.30, 29.36, 29.34 (CH₂'s, -ve DEPT), 31.59 (OCH₂CH₂CH, -ve DEPT), 31.83 (CH₂, -ve DEPT), 57.95 (CH₃O, +ve DEPT), 64.39 (OCH₂CH₂CH, -ve DEPT), 68.09 (ArOCH₂C₇, -ve DEPT), 71.74 (OCH₂C₄, -ve DEPT), 72.63 (CH₂OC₅, -ve DEPT), 77.02 (C*H, +ve DEPT), 114.79, 114.81 (Ar-C_{3'/3"}, +ve DEPT), 126.96 (Ar-C_{2/3}, +ve DEPT), 127.93, 127.95 (Ar-C_{2'/2"}, +ve DEPT), 133.03, 133.21 (Ar-C_{1/4}, no DEPT), 139.05, 139.14 (Ar-C_{1'/1"}, no DEPT), 158.51 (Ar-C_{4",} no DEPT), 158.71 (Ar-C_{4'}, no DEPT). FT-IR (KBr/DRIFT) νₘₐₓ: 2966, 2926, 2860, 1613, 1493, 1288, 1250, 1186, 1115, 819, 667, 505 cm⁻¹. UV-Vis (4.89 mg, CHCl₃) λₘₐₓ: 295.50 (0.7500) nm. HPLC analysis (C18 column; 1 ml min⁻¹ 90:10 acetonitrile-tetrahydrofuran) R_{T} = 10.02 (100%) min.

### B. 1,(S)-2-(4"-Octyloxy-mono-fluoro-terphenyl-4-oxy)dialkoxy-butanes (Examples 4 to 9)

### Summary

Data generated from Examples 1 to 3 (see below) showed that the 1,*(S)*-2-dialkyloxybutane terminal unit was capable of generating an interesting mixture of novel chiral phases (N*, TGB A* and TGB C*) as well as ferroelectric phases (S_{C}*), albeit at relatively high melting (65-162 °C) and thermal stabilities (118-180 °C). It was hoped that fluorine substitution on the terphenyl moiety would lower the thermal stability of the various phases as well as the melting point, while maintaining the interesting phase behaviour.

The routes employed are shown in schemes 3 to 6. **Scheme 3** charts the route used to synthesise mono-fluoro-4-bromo-1-(1-pentoxy-2-pentoxybutoxy)benzenes **(21** and **22)** and is essentially synthetically identical to that described in first three steps of scheme 2. Schemes 4 and 5 describe the routes to various phenyl, biphenylene and mono-fluorobiphenylene units which are used in the final steps of synthesis (Scheme 6). **Scheme 4** shows the routes used for the synthesis of 4-(2'- or 3'-fluoro-4'-octyloxyphenyl)phenylboronic acids **30** and **31**. Here the appropriately substituted bromophenols **15** or **16** were alkylated using 1-bromooctane and potassium carbonate in butanone to give compounds **23** and **24** as colourless liquids in quantitative yields. These were then converted to their respective boronic acids **25** and **26** using butyllithium and trimethyl borate at -78 °C. Compounds **25** and **26** then underwent Suzuki cross-coupling with 1-bromo-4-iodobenzene **(27)** using palladium(0) *tetrakis*(triphenylphosphine) catalyst to give the biphenyls **28** and **29** in low to moderate yields (26-51%) (R.B. Miller et. al., *supra;* M. Hird et. al., *supra;* L.K.M. Chan et. al., *supra;* L.K.M. Chan et. al., *supra*). Compounds **28** and **29** were then converted to 4-(2'-fluoro-4'-octyloxyphenyl)phenyl boronic acid **30** and 4-(3'-fluoro-4'-octyloxyphenyl)phenyl boronic acid **31** respectively in high yields. **Scheme 5** outlines the three step route to 4-(4'-octyloxyphenyl-2-fluorophenylboronic acid (**36**) and 4-(4'-Octyloxyphenyl)-3-fluorophenylboronic acid (**39**) starting from 1-bromo-4-octyloxybenzene (**32**) using and identical synthetic approach to scheme 4.

Finally, the relevant fragments drawn from schemes 3, 4 to 5 were brought together in **scheme 6** which illustrates the final steps in the synthesis of the target materials **40, 41, 42, 43,** and **45.** All compounds were simply formed in one step reaction from the chiral ethers **13a, 21** and **22** with the appropriate boronic acids **11, 30, 31, 36** and **39** and palladium(0) *tetrakis*(triphenylphosphine) under Suzuki cross-couplings.

All final materials were purified rigorously by flash chromatography (often repeatedly) and by repeated recrystallisation until HPLC analysis revealed purities in excess of at least 99.8%. This route failed for the synthesis of 1,2-(S)-dipentoxy-(3'-fluoro-4"-octyloxyterphenyl-4-oxy)butane, only starting material **13a** and the hydrodeboronated 3-fluorobiphenyl being recovered. Presumably, the α-fluor substituent of compound (**39**) causing activation of the boronic acid which results in facile hydro-deboronation.

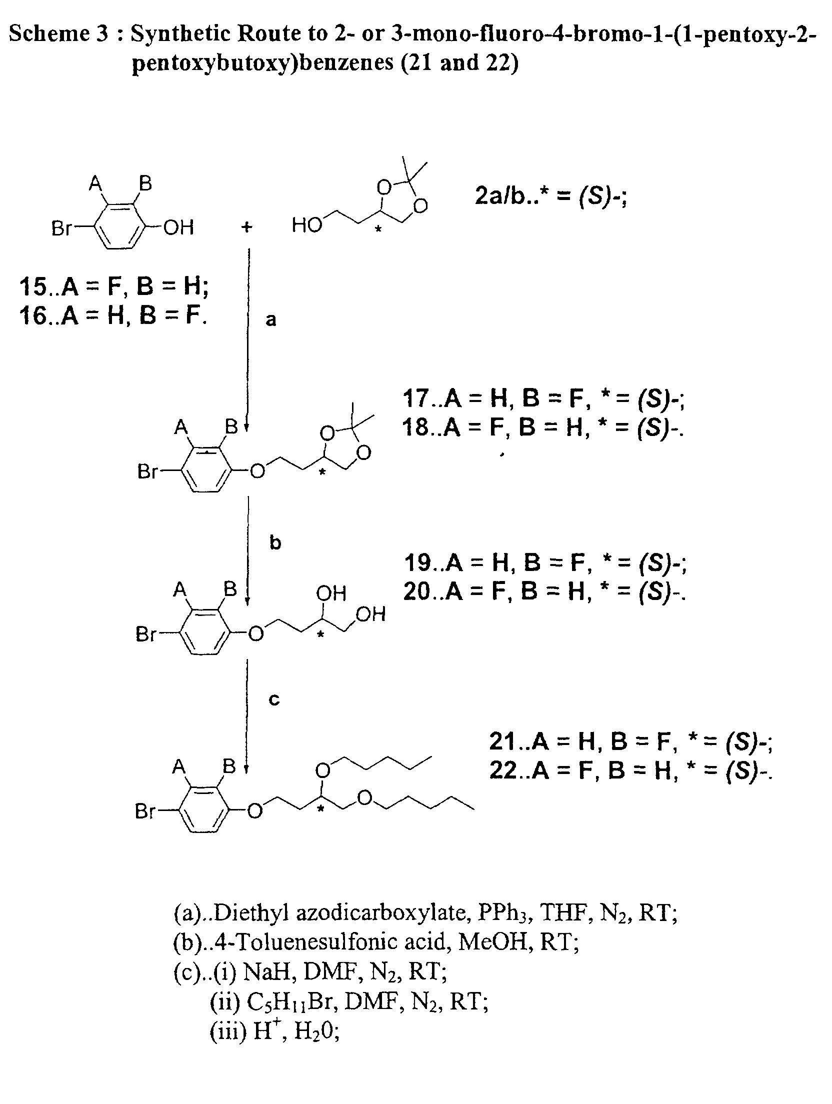

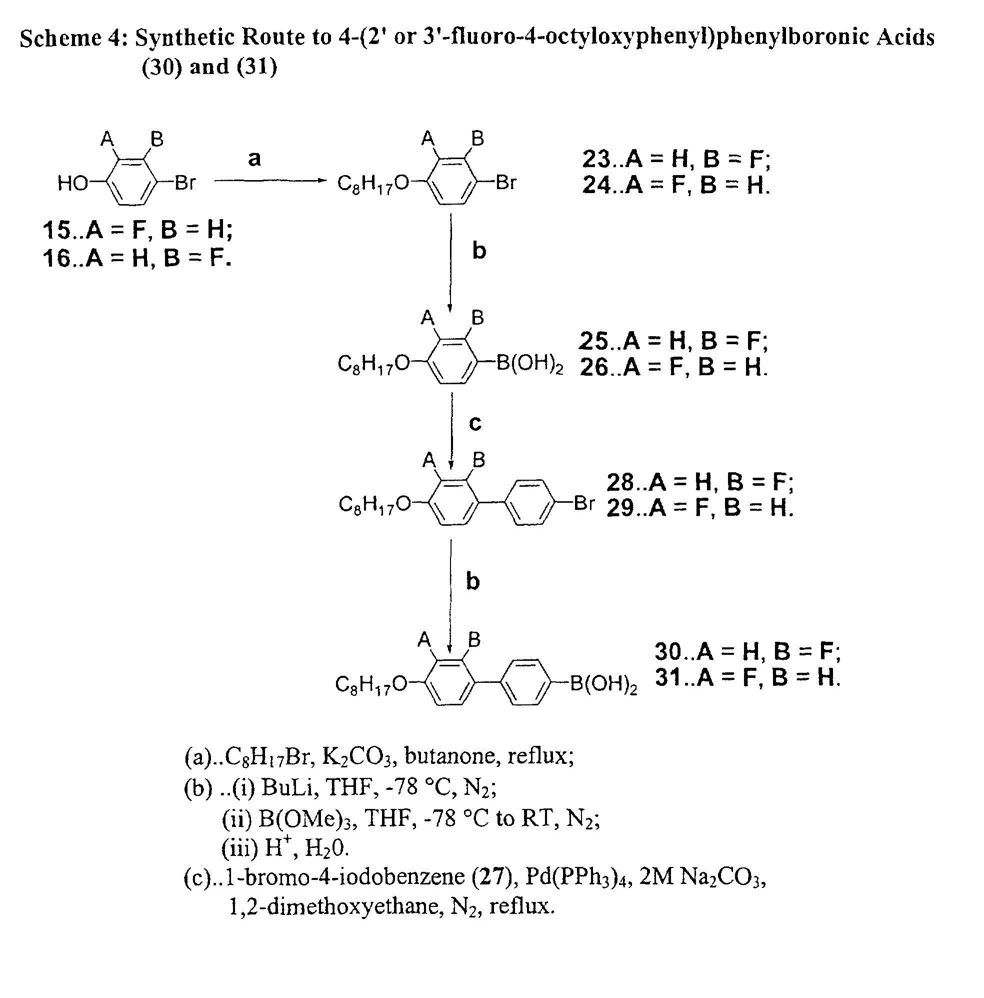

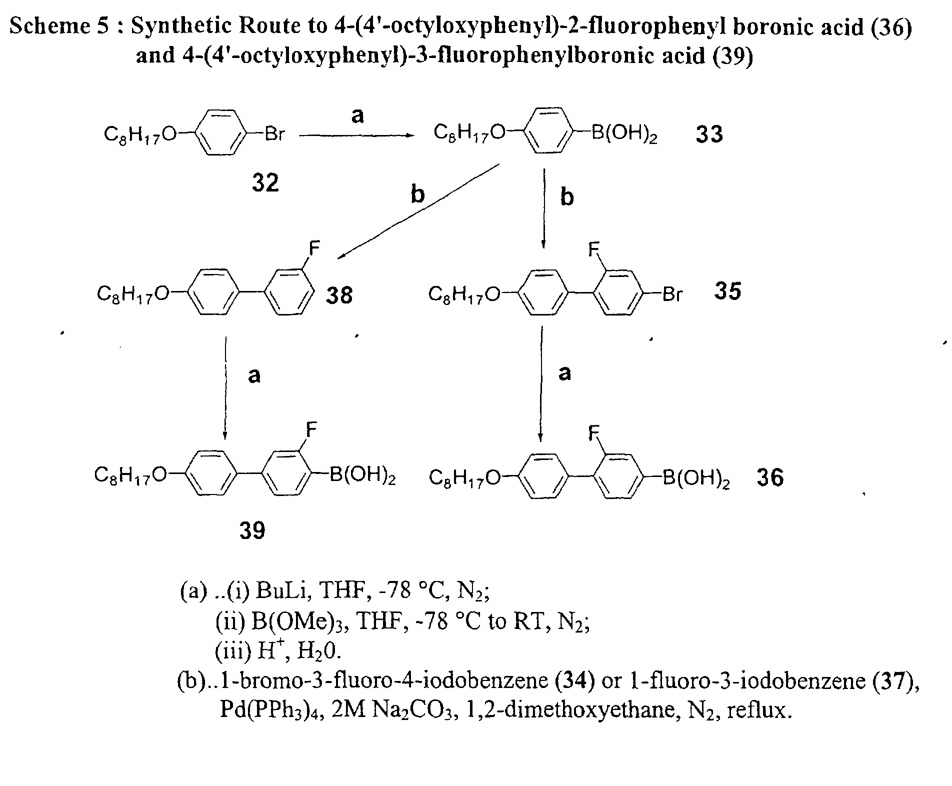

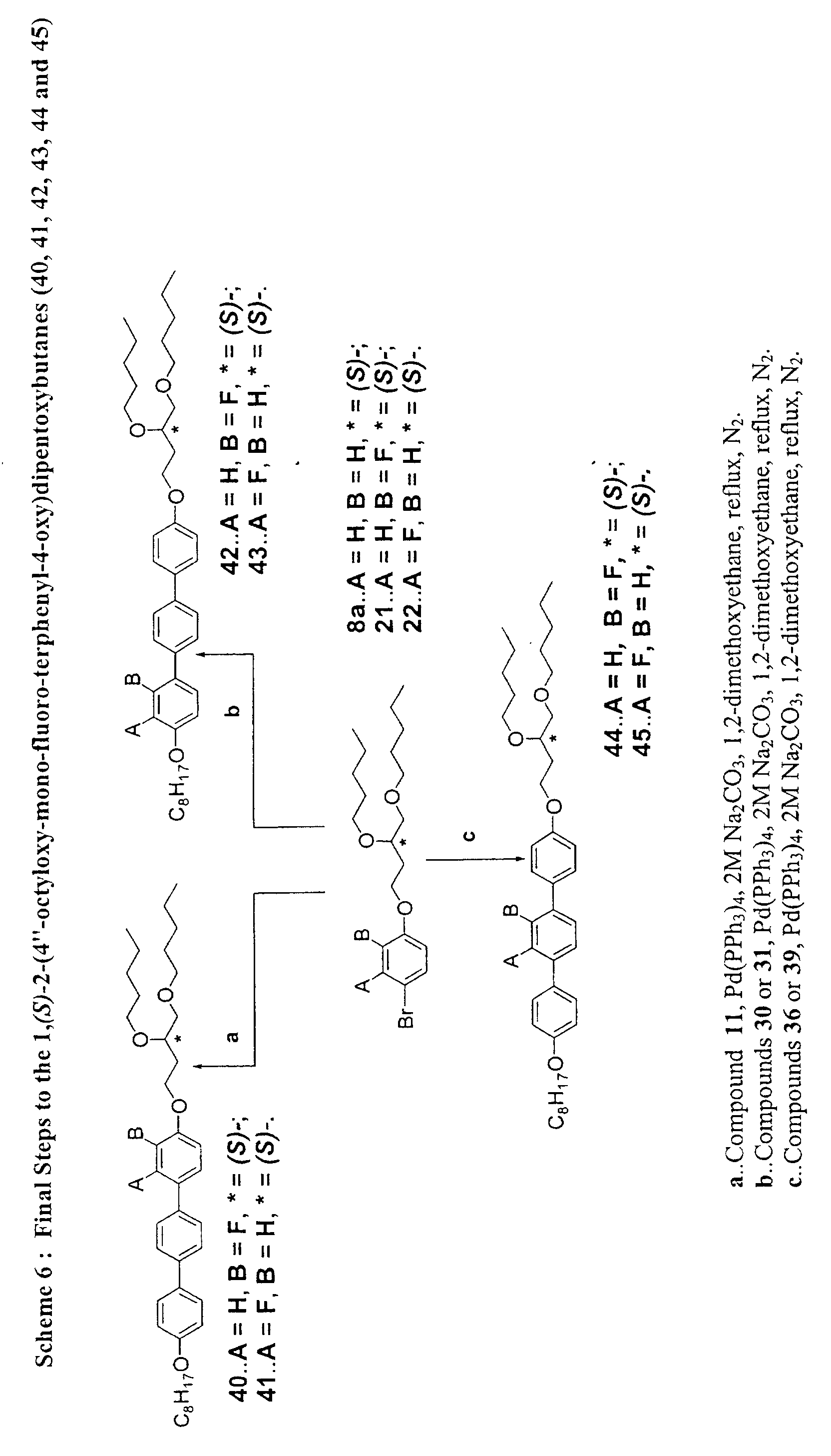

### Experimental details

***1-{(2,2-dimethyl)-1,(S)-2-dioxolan-4-yl}ethoxy-4-bromo-2-fluorobenzene (17)*** A similar procedure to that used for compound **5a** was used. Quantities used: compound **2a/b** (9.00 g, 61.6 mmol), triphenylphosphine (10.78 g, 41.1 mmol), 2-fluoro-4-bromophenol **(16)** (7.85 g, 41.1 mmol), diethylazodicarboxylate (7.16 g, 41.1 mmol) and tetrahydrofuran (150 ml). Purified by flash chromatography [fine mesh silica gel: 10% ethyl acetate in hexane] to give a colourless oil. Dried *in vacuo* (P₂O₅, RT, 5.0 mbar, 36 h). Yield = 8.59 g (66%). ¹H NMR (300MHz, CDCl₃, TMS) δ: 1.36 (3H, s, CCH₃), 1.42 (3H, s, CCH₃), 2.06 (2H, dd, OCH₂CH₂CH*), 3.65 (1H, t, *CHCH₂O), 4.01 (2H + 1H, m, OCH₂CH₂CH* + *CHCH₂O), 4.30 (1H, q, OCH₂CH₂CH*), 6.83 (1H, t, Ar-H₅, ³J_{HH} = 8.73 Hz), 7.16 (1H, m, Ar-H₆, ³J_{HH} = 8.65 Hz), 7.21 (1H, m, Ar-H₃, ³J_{HF} = 10.46 Hz). ¹³C NMR (75MHz, CDCl₃, TMS) δ: 26.29 (CCH₃, +ve DEPT), 33.39 (OCH₂CH₂CH*, -ve DEPT), 66.24 (OCH₂CH₂CH*, -ve DEPT), 69.45 (*CHCH₂O, -ve DEPT) 73.16 (OCH₂CH₂CH*, +ve DEPT), 103.86 (CCH₃, no DEPT), 112.11 (Ar-C₄, d, J_{CF} = 8.164 Hz, no DEPT), 115.82 (Ar-C₆, d, J_{CF} = 2.123 Hz, +ve DEPT), 119.67 (ArC₃, d, J_{CF} = 21.35 Hz, +ve DEPT), 127.17 (Ar-C₅, d, J_{CF} = 4.12 Hz, +ve DEPT), 146.25 (Ar-C₁, d, J_{CF} = 10.41 Hz, no DEPT), 152.4 (Ar-C₂, d, J_{CF} = 250.9 Hz, no DEPT). ¹⁹F NMR (282MHz, CDCl₃, TMS) δ: -131.589 (IF, t, ArC₂-F, ³J_{HF} = 9.5 Hz, collapses to singlet at-131.59 ppm on decoupling). FT/IR (film/ATR/ZnSe) µₘₐₓ: - 2988, 2945, 2871, 1498, 1372, 1264, 1204, 1144, 1059, 866, 810 cm⁻¹.

***1-{(2,2-dimethyl)-1,(S)-2-dioxolan-4-yl}ethoxy-4-bromo-3-fluorobenzene (18)*** A similar procedure to that used for compound **5a** was used. Quantities used: compound **2a/b** (8.80 g, 60.3 mmol), triphenylphosphine (10.54 g, 40.1 mmol), 3-fluoro-4-bromophenol **(15)** (7.67 g, 40.1 mmol), diethylazodicarboxylate (7.00 g, 40.1 mmol) and tetrahydrofuran (145 ml). Purified by flash chromatography [fine mesh silica gel: 10% ethyl acetate in hexane] to give a colourless oil. Dried *in vacuo* (P₂O₅, RT, 0.5 mbar, 18h). Yield = 6.81 g (53%); ¹H NMR (300MHz, CDCl₃, TMS) δ: 1.36 (3H, s, O₂CCH₃), 1.42 (3H, s, O₂CCH₃), 2.04 (2H, q, OCH₂CH₂CH), 3.64 (1H, q, CHCH₂O), 4.04 (1H, t, OCH₂CH₂CH), 4.11(1H, m, CHCH₂O), 4.28 (1H, q, OCH₂CH₂CH), 6.63 (1H, m, Ar-H₆, ³J_{HH} = 8.88 Hz), 6.71 (1H, m, Ar-H₅, ³J_{HH} = 10.41 Hz), 7.39 (1H, m, Ar-H₂, ³J_{HF} = 8.81 Hz). ¹³C NMR (75MHz, CDCl₃, TMS) δ: 25.70 (O₂CCH₃, +ve DEPT), 26.98 (O₂CCH₃, +ve DEPT), 33.33 (OCH₂CH₂CH, -ve DEPT), 65.32 (OCH₂CH₂CH, -ve DEPT), 69.45 (CHCH₂O, -ve DEPT), 73.11 (OCH₂CH₂CH, +ve DEPT), 99.3 (Ar-C₄, d, J_{CF} = 21.2 Hz, no DEPT), 102.37 (Ar-C₂, d, J_{CF} = 25.5 Hz, +ve DEPT), 108.97 (O₂-C-(CH₃)₂, no DEPT), 111.81 (Ar-C₆, d, J_{CF} = 3.00 Hz, +ve DEPT), 133.34 (Ar-C₅, d, J_{CF} = 2.0 Hz, +ve DEPT), 159.37 (Ar-C₁, d, J_{CF} = 10 Hz, no DEPT), 159.48 (Ar-C₃, d, J_{CF} = 246 Hz, no DEPT). ¹⁹F NMR (282MHz, CDCl₃, TMS) δ: -105.65 (1F, t, ArC₃-F, ³J_{HF} = 9.24 Hz, collapses to singlet at -105 ppm on decoupling). FT/IR (film/ATR/ZnSe) µₘₐₓ: 2985, 2933, 2871, 1603, 1583, 1488, 1470, 1379, 1321, 1291, 1259, 1239, 1165, 1059, 982, 833 cm⁻¹.

***4-Bromo-2-fluoro-1-(1,(S)-2-dihydroxybutoxy)benzene (19)*** This compound was prepared in a manner similar to compound **6a.** Quantities used: p-toluenesulfonic acid (500 mg), compound **17** (8.00 g, 25.0 mmol) and methanol (460 ml). Purified by flash chromatography [fine mesh silica gel: dichloromethane (initially) then 1:1 dichloromethane: ethyl acetate (finally)]. Dried *in vacuo* (P₂O₅, 10 mbar, RT, 60 h). Yield = 5.25g (75%). ¹H.NMR (300 MHz, CDCl₃, TMS) δ : 1.91 (2H, m, OCH₂CH₂*CH), 3.13 (1H, broad s, *CHCH₂OH), 3.43 (1H, broad s, *CHOH), 3.52 (1H, t, *CHCH₂OH), 3.70 (1H, s, *CHCH₂OH), 3.98 (1H, s, OCH₂CH₂*CH), 4.15 (2H, m, OCH₂CH₂-*CH), 6.83 (1H, t, Ar-H₆, ³J_{HH} = 9.25 Hz), 7.16 (1H, m, Ar-H₅, ³J_{HH} = 8.12 Hz), 7.21 (1H, dd, Ar-H₃, ³J_{HF} = 13.22 Hz). ¹³C NMR (75MHz, CDCl₃, TMS) δ: 32.33 (OCH₂CH₂*CH, -ve DEPT), 66.53 (*CHCH₂OH, -ve DEPT), 66.63 (OCH₂CH₂*CH, -ve DEPT), 69.60 (OCH₂CH₂*CH, -ve DEPT), 112.33 (Ar-C₄, d, J_{CF} = 8.818 Hz, no DEPT), 115.88 (Ar-C₆, d, J_{CF} = 2.05 Hz, +ve DEPT), 119.67 (Ar-C₃, d, J_{CF} = 21.19 Hz, +ve DEPT), 127.29 (Ar-C₅, d, J_{CF} = 4.037 Hz, +ve DEPT), 146.01 (Ar-C₁, d, J_{CF} = 10.69 Hz, no DEPT), 152.38 (Ar-C₂, d, J_{CF} = 250.41 Hz, no DEPT). ¹⁹F NMR (282 MHz, CDCl₃, TMS) δ: -131.90 (1F, t, ArC₂-F, J_{HF} = 9.44 Hz, collapses to a singlet at -131.90 ppm on decoupling). FT/IR (KBr/Drift) µₘₐₓ: 3251, 1508, 1466, 1306, 1269, 1213, 1130, 1079, 968, 869, 804 cm⁻¹.

***4-Bromo-3-fluoro-1-(1,(S)-2-dihydroxybutoxy)benzene (20)*** This compound was prepared in a manner similar to compound **6a.** Quantities used: p-toluene sulphonic acid (500 mg), compound **18** (6.00 g, 18.8 mmol) in methanol (460 ml). Dried *in vacuo* (P₂O₅, 0.4 mbar, RT, 18 h). Yield = 4.94 g (94%). ¹H NMR (300MHz, CDCl₃, TMS) δ: 1.91(2H, m, OCH₂CH₂CH), 2.49 (1H, Broad, s, CHOH), 2.85 (1H, Broad, s, CHCH₂OH), 3.52 (1H, dd, CHCH₂OH), 3.71 (1H, dd, CHCH₂OH), 3.98 (1H, q, OCH₂CH₂CH), 4.10 (2H, m, OCH₂CH₂CH), 6.60 (1H, m, Ar-H₆, ³J_{HH} = 8.85 Hz), 6.69 (1H, dd, Ar-H₂, ³J_{HF} = 10.3 Hz), 7.40 (1H, t, Ar-H₅, ³J_{HH} = 8.44 Hz). ¹³C NMR (75MHz, CDCl₃, TMS) δ: 32.70 (OCH₂CH₂CH, -ve DEPT), 65.83 (OCH₂CH₂CH, -ve DEPT), 69.92 (OCH₂CH₂CH, +ve DEPT), 99.94 (Ar-C₄, d, J_{CF} = 21.1 Hz, no DEPT), 103.78 (Ar-C₂, d, J_{CF} = 25.5 Hz, +ve DEPT), 112.18 (Ar-C₆, d, J_{CF} = 2.882 Hz, +ve DEPT), 133.78 (Ar-C₅, d, J_{CF} = 2.165 Hz, +ve DEPT), 159.58 (Ar-C₁, d, J_{CF} = 9.45 Hz, no DEPT), 159.85 (Ar-C₃, d, J_{CF} = 247.2 Hz, no DEPT). ¹⁹F NMR (282 MHz, CDCl₃, TMS) δ: -105.46 (1F, t, ArC₃-F, ³J_{HF} = 9.2 Hz, collapses to singlet at -105.46 ppm on decoupling). FT/IR (KBr/Drift) µₘₐₓ: 3244, 2939, 2906, 2880, 2833, 1613, 1593, 1487, 1463, 1321, 1263, 1237, 1171, 1130, 1075, 985, 961, 919, 824 cm⁻¹.

***4-Bromo-2-fluoro-1-(1,(S)-2-dipentoxybutoxy)benzene (21)*** This compound was prepared in a manner similar to compound **8a.** Quantities used: 1-bromopentane (7.31g, 48.4 mmol), compound **19** (4.50 g, 16.1 mmol), sodium hydride (2.58 g, 60% dispersion in oil *ca.*, 64 mmol) and dimethylformamide (50 ml). Purified by flash chromatography [fine mesh silica gel: dichloromethane] to give a colourless oil. Dried *in vacuo* (P₂O₅, 10 mbar, RT, 24 h). Yield = 3.43 g (51%). ¹H NMR (300MHz; CDCl₃, TMS) δ: 0.87 (6H, m, OC₄CH₃'s), 1.30 (8H, m, OCH₂CH₂CH₂CH₂CH₃), 1.55 (4H, m, OCH₂CH₂C₃), 1.93 (1H, m, ArOCH₂CH₂*CH), 2.04 (1H, m, ArOCH₂CH₂*CH), 3.44 (4H + 1H, m, O-CH₂C₄ + ArOCH₂CH₂*CH), 3.65 (2H, m, *CHCH₂OC₅), 4.13 (2H, m, ArOCH₂CH₂*CH), 6.85 (1H, t, Ar-H₆, ³J_{HH} = 8.70 Hz), 7.16 (1H, dd, Ar-H₅, ³J_{HH} = 9.71 Hz), 7.21 (1H, dd, Ar-H₃, ³J_{HH} = 10.47 Hz). ¹³C NMR (75MHz, CDCl₃, TMS) δ: 14.41 (OC₄CH₃'s, +ve DEPT), 22.88 (OC₃CH₂CH₃, -ve DEPT), 28.71 (ArOCH₂CH₂*CH, -ve DEPT), 29.75 (OC₂CH₂C₂, -ve DEPT), 31.19 (OCH₂CH₂C₃, -ve DEPT), 66.37 (ArOCH₂CH₂*CH /-ve), 71.38 (OCH₂C₄, -ve DEPT), 73.39 (*CHCH₂OC₅, -ve DEPT), 75.31 (ArOCH₂CH₂*CH, +ve DEPT), 112.17 (Ar-C₄, d, J_{CF} = 8.2 Hz, no DEPT), 116.26 (Ar-C₆, d, J_{CF} = 2.2 Hz, +ve DEPT), 120.0 (Ar-C₃, d, J_{CF} = 21.2 Hz, +ve DEPT), 127.5 (Ar-C₅, d, J_{CF} = 4.1 Hz, +ve DEPT), 146.9 (Ar-C₁, d, J_{CF} = 10.7 Hz, no DEPT), 152.9 (Ar-C₂, d, J_{CF} = 250.7 Hz, no DEPT). ¹⁹F NMR (282 MHz, CDCl₃, TMS) δ: -131.69 (1F, t, ArC₂-F, J_{HF} = 9.59 Hz, collapses to singlet at -131.69 on decoupling). FT/IR (film/ATR/ZnSe) µₘₐₓ: - 2955, 2930, 2859, 1501, 1304, 1264, 1206, 1116, 1107, 872, 810 cm⁻¹.

***4-Bromo-3-fluoro-1-(1,(S)-2-dipeutoxybutoxy)benzene (22)*** This compound was prepared in a manner similar to compound **8a.** Quantities used: 1-bromopentane (6.49 g, 43 mmol), compound **20** (4.00 g, 14.3 mmol), sodium hydride (2.29 g, 60% dispersion in oil, *ca.*, 57 mmol) and dimethylformamide (50 ml). This was purified by flash chromatography [fine mesh silica gel: 10% ethyl acetate in hexane] to give a colourless oil. Dried *in vacuo* (P₂O₅, 0.8 mbar, RT, 16 h). Yield = 3.60 g (60%). ¹H NMR (300MHz, CDCl₃, TMS) δ : 0.87 (6H, m, OC₄CH₃), 1.30 (8H, m, OC₂CH₂CH₂CH₃), 1.55 (4H, m, OCH₂CH₂CH₃), 1.96 (2H, m, OCH₂CH₂*CH), 3.43 (4H + 1H, m, OCH₂C₄ + *CHCH₂OC₅), 3.63 (1H + 1H, m, OCH₂CH₂*CH + CHCH₂OC₅), 4.05 (2H, m, OCH₂CH₂*CH), 6.60 (1H, ddd, Ar-H₆, ³J_{HH} = 8.8 Hz), 6.70 (1H, dd, Ar-H₂, ³J_{HF} = 10.49 Hz), 7.38 (1H, t, Ar-H₅, ³J_{HH} = 8.69 Hz). ¹³C NMR (75MHz, CDCl₃, TMS) δ: 14.4 (OC₄CH₃, +ve DEPT), 22.9 (OC₃CH₂CH₃, -ve DEPT), 28.71 (OCH₂CH₂*CH, -ve DEPT), 29.96 (OCH₂CH₂CH₂CH₂CH₃, -ve DEPT), 32.25 (OCH₂CH₂C₃, -ve DEPT), 65.22 (OCH₂CH₂*CH,-ve DEPT), 71.44 (*CHCH₂OC₅, -ve DEPT), 73.46 (OCH₂C₄, -ve DEPT), 75.45 (OCH₂CH₂*CH, +ve DEPT), 99.4 (Ar-C₄, d, J_{CF} = 21.42 Hz, no DEPT), 103.71 (Ar-C₂, d, J_{CF} = 25.45 Hz, +ve DEPT), 112.21 (Ar-C₆, d, J_{CF} = 3.24 Hz, +ve DEPT), 133.63 (Ar-C₅, d, J_{CF} = 1.90 Hz, +ve DEPT), 159.84 (Ar-C₃, d, J_{CF} = 246.25 Hz, no DEPT), 160.02 (Ar-C₁, d, J_{CF} = 9.927 Hz, no DEPT). ¹⁹F NMR (282 MHz, CDCl₃, TMS) δ: -105.86 (IF, t, ArC₃-F, J_{HF} = 8.65 Hz, collapses to a singlet at -105.86 ppm on decoupling). FT/IR (Film/ATR/ZnSe) µₘₐₓ: 2955, 2930, 2859, 1604, 1583, 1489, 1466, 1321, 1291, 1167, 1141, 1120, 1109 cm⁻¹.

***1-Bromo-3-fluoro-4-octyloxybenzene (23)*** Bromooctane (9.93 g, 51.44 mmol) in acetone (50 ml) was added dropwise to a stirred, refluxing solution of compound **15** (6.55 g, 34.29 mmol), potassium carbonate (9.47 g, 68.58 mmol) and acetone (50 ml). The solution was then allowed to reflux for 5 h. TLC analysis showed some starting material present, so a small amount of potassium carbonate and bromooctane were added. The solution was then allowed to reflux for a further 10 h. TLC analysis showed no starting material left, so the mixture was allowed to cool to room temperature overnight. The solution was then filtered, and the solid residue washed with dichloromethane. The filtrate was then evaporated to a colourless oil which was redissolved in dichloromethane (200 ml). This solution was washed with water (50 ml), 10% (v/v) sodium hydroxide (50 ml) and finally water (50 ml). This was then dried (MgSO₄), filtered, evaporated to a colourless oil and purified by flash chromatography [coarse silica gel: dichloromethane]. Dried *in vacuo* (P₂O₅, 0.1mbar, RT, 18 h) to give a colourless oil. Yield = 10.73 g (>100%); ¹H NMR (300MHz, CDCl₃, TMS) δ: 0.88 (3H, t, ArOC₇CH₃), 1.35 (10H, m, ArOC₂CH₂CH₂CH₂CH₂CH₂CH₃), 1.81 (2H, m, ArOCH₂CH₂C₆), 3.91 (2H, t, ArOCH₂C₇), 6.59 (1H, ddd, ArH₆, ³J_{HH} = 6.84 Hz), 6.67 (1H, dd, ArH₂, ³J_{HF} = 10.52 Hz), 7.39 (1H, t, ArH₅, ³J_{HH} = 8.44 Hz). FT/IR (film/ATR/ZnSe) µₘₐₓ: 2955, 2924, 2854, 2869, 1604, 1583, 1488, 1467, 1321, 1291, 1166, 1143 cm⁻¹.

***1-Bromo-2-fluoro-4-octyloxybenzene (24)*** Prepared using a similar procedure to compound **23.** Quantities used: 1-bromooctane (7.58 g, 39.3 mmol), compound **16** (5.00 g, 26.2 mmol), potassium carbonate (7.24 g, 52.4 mmol) and butanone (160 ml). Purified by flash chromatography [fine mesh silica gel: dichloromethane] and then dried *in vacuo* (P₂O₅, 0.2 mbar, RT, 18 hrs) Yield = 9.18 g (>100%); ¹H NMR (300MHz, CDCl₃, TMS) δ: 0.88 (3H, t, CH₃C₇O), 1.30 (8H, m, CH₃CH₂CH₂CH₂CH₂C₃O), 1.42 (2H, m, C₅CH₂CH₂CH₂O), 1.75 (2H, q, C₆CH₂CH₂O), 3.89 (2H, t, C₇CH₂O), 6.58 (1H, ddd, ArC₅-H, ³J_{HH} = 8.86 Hz), 6.67 (1H, dd, ArC₃-H, ³J_{HF} = 10.53 Hz), 7.37 (1H, t, ArC₆-H, ³J_{HH} = 8.46 Hz). ¹³C NMR (300MHz, CDCl₃, TMS) δ: 14.49 (CH₃C₇O, +ve DEPT), 23.06 (CH₃CH₂C₆O, -ve DEPT), 29.42 (C₃CH₂C₄O, -ve DEPT), 29.62 (C₄CH₂C₃O, -ve DEPT), 29.71 (C₆CH₂CH₂O, -ve DEPT), 32.20 (CH₃CH₂CH₂C₅O, -ve DEPT), 68.98 (C₇CH₂O, -ve DEPT), 99.28 (Ar-C₁, d, J_{CF} = 21.5 Hz, no DEPT), 103.64 (Ar-C₃, d, J_{CF} = 25.4 Hz, +ve DEPT), 112.28 (Ar-C₅, d, J_{CF} = 3.00 Hz, +ve DEPT), 133.60 (Ar-C₆, d, J_{CF} = 1.91 Hz, +ve DEPT), 159.84 (Ar-C₂, d, J_{CF} = 246.14 Hz, no DEPT), 160.16 (Ar-C₄, d, J_{CF} = 9.92 Hz, no DEPT). ¹⁹F NMR (282MHz, CDCl₃, TMS) δ:-105.89 (1F, t, ArC₂-F, J_{HF} = 9.30 Hz, collapses to a singlet at -105.89 on decoupling). FT/IR (film/ATR/ZnSe) µₘₐₓ: - 3383, 3198, 2954, 2856, 1607, 1471, 1397, 1341, 1287, 1267, 1162, 1124 cm⁻¹.

***2-Fluoro-4-octyloxyphenylboronic acid (25)*** Butyllithium (8.0 ml, 2.5 M in hexanes, 20 mmol) was added dropwise *via* syringe and septum to a stirred solution of compound **23** (5.00 g, 16.5 mmol) in dry tetrahydrofuran (150 ml) at -78°C under a dry nitrogen atmosphere. Once this addition had occurred the solution was allowed to stir at this cooled temperature for a further 2 h. To this, trimethylborate (3.58 g, 34.5 mmol) in dry tetrahydrofuran (20ml) was added dropwise ensuring that the temperature did not exotherm over -70°C. The solution was allowed to stir at this cooled temperature for a further hour before being allowed to warm to room temperature overnight. To this now warmed solution 10% (v/v) hydrogen chloride solution (50 ml) was added dropwise and the solution allowed to stir for a further 30 min. The product was extracted by washing with diethyl ether (3 x 75 ml). These extracts were combined, dried (MgSO₄), filtered and evaporated to give a brown oil. Dried *in vacuo* (P₂O₅, 0.1mbar, RT, 60 h) to form a brown solid. Yield = 3.99 g (90%); FT/IR (KBr/Drift) µₘₐₓ: 3509, 3344, 3218, 2943, 2866, 1618, 1573, 1431, 1375, 1289, 1135, 1036, 1003, 851, 791, 659 cm⁻¹.

***3-Fluoro-4-octyloxyphenylboronic acid (26)*** Prepared using a similar procedure to compound **25**. Quantities used: Butyllithium (2 ml, 10.0 M in hexanes, 20 mmol), compound **24** (5.00 g, 16.5 mmol) and dry tetrahydrofuran (150 ml). Dried *in vacuo* (P₂O₅, 0.7mbar, RT, 72 h) to give a brown oil. Yield = 4.82 g (>100%); FT/IR (film/ATR/ZnSe) µₘₐₓ: 3204, 2925, 2852, 2359, 2334, 1616, 1456, 1430, 1420, 1352, 1323, 1292, 1230, 1125 cm⁻¹.

***4-(2'-Fluoro-4'-octyloxyphenyl)-4-bromobenzene (28)*** This compound was prepared in a manner similar to compound **12.** Quantities used: compound **25** (3.50 g, 13.1 mmol), compound **27** (2.95 g, 10.5 mmol), palladium(0) *tetrakis*(triphenylphosphine) (0.43 g, 0.37 mmol), 2M sodium carbonate (45 ml), and 1,2-dimethoxyethane (70 ml). Purified by flash chromatography [fine mesh silica gel: 5% dichloromethane in hexane] to give a pale oil. Dried *in vacuo* (P₂O₅, 0.1mbar, RT, 72 h) to give a white crystalline solid. Yield = 2.00 g (51%); ¹H NMR (300MHz, CDCl₃, TMS) δ: 0.89 (3H, t, CH₃C₇O), 1.33 (8H, m, CH₃CH₂CH₂CH₂CH₂C₃O), 1.45 (2H, m, C₅CH₂CH₂CH₂O), 1.78 (2H, q, C₆CH₂CH₂O), 3.95 (2H, t, C₇CH₂O), 6.69 (1H, dd, Ar'C₃H, ³J_{HF} = 12.58 Hz), 6.74 (1H, dd, Ar'C₅H, ³J_{HH} = 8.58 Hz), 7.28 (1H, t, Ar'C₆H, ³J_{HH} = 8.8 Hz), 7.37 (2H, dd, ArH₂/H₆, ³J_{HH} = 8.52 Hz), 7.53 (2H, dd, ArH₃/H₅, ³J_{HH} = 6.6 Hz). ¹³C NMR (75MHz, CDCl₃, TMS) δ: 14.53 (CH₃C₇O, +ve DEPT), 23.08 (CH₃CH₂C₆O, -ve DEPT), 26.41 (C₅CH₂CH₂CH₂O, -ve DEPT), 29.51 (C₃CH₂C₄O, -ve DEPT), 29.65 (C₄CH₂C₃O, -ve DEPT), 29.74 (C₆CH₂CH₂O, -ve DEPT), 68.86 (C₇CH₂O, -ve DEPT), 102.94 (Ar'C₃, d, J_{CF} = 26.2 Hz, +ve DEPT), 111.36 (Ar'C₅, d, J_{CF} = 3.1 Hz, +ve DEPT), 120.26 (Ar'C₁, d, J_{CF} = 13.4 Hz, no DEPT), 121.61 (ArC₄, no DEPT), 130.72 (ArC₂/C₆, +ve DEPT) 130.76 (ArC₂/C₆, +ve DEPT), 131.05 (Ar'C₆, d, J_{CF} = 4.3 Hz, +ve DEPT), 131.92 (ArC₃/C₅, +ve DEPT), 135.13 (ArC₁, d, J_{CF} = 1.2 Hz, no DEPT), 160.48 (Ar'C₄, d, J_{CF} = 10.9 Hz, no DEPT), 160.54 (Ar'C₂, d, J_{CF} = 247.9 Hz, no DEPT). ¹⁹F NMR (282MHz, CDCl₃, TMS) δ: -116.013 (1F, t, Ar'C₂-F, ³J_{HF} = 9.67 Hz, collapses to singlet at -116.013 on decoupling). FT/IR (KBr/Drift) µₘₐₓ: 2925, 2860, 1620, 1473, 1460, 1327, 1235, 1129, 1030, 957, 840, 816, 725, 515, 462 cm⁻¹.

***4-(3'-Fluoro-4'-octyloxyphenyl)-4-bromobenzene (29)*** This compound was prepared in a similar manner to compound **12.** Quantities used: compound **26** (4.70 g, 17.5 mmol), compound **27** (3.96 g, 14.0 mmol), palladium(0) *tetrakis*(triphenylphosphine) (0.48 g, 0.42 mmol), 2M sodium carbonate (45 ml) and 1,2-dimethoxyethane (70 ml). Purified using flash chromatography [fine mesh silica gel: hexanes] to give a white solid and recrystallised using hexane. Dried *in vacuo* (P₂O₅, 0.1mbar, RT, 16 h). Yield = 1.39 g (26.2%); ¹H NMR (300MHz, CDCl₃, TMS) δ: 0.89 (3H, m, CH₃C₇O), 1.34 (8H, m, CH₃CH₂CH₂CH₂CH₂C₃O), 1.45 (2H, m, C₅CH₂CH₂CH₂O), 1.79 (2H, q, CH₆CH₂CH₂O), 3.93 (2H, t, CH₇CH₂O), 6.69 (1H, dd, Ar'C₃-H, ³J_{HF} = 12.58 Hz), 6.75 (1H, dd, Ar'C₅-H, ³J_{HH} = 8.37 Hz), 7.27 (1H, t, Ar'C₆-H, ³J_{HH} = 8.82 Hz), 7.37 (2H, dd, ArC_{2/6}-H, ³J_{HH} = 6.77 Hz), 7.53 (2H, dd, ArC_{3/5}-H, ³J_{HH} = 7.53 Hz). ¹³C NMR (75MHz, CDCl₃, TMS) δ: 14.51 (CH₃C₇O, +ve DEPT), 23.06 (CH₃CH₂C₆O, -ve DEPT), 26.39 (C₅CH₂CH₂CH₂O, -ve DEPT), 29.49 (C₃CH₂C₄O, -ve DEPT), 29.72 (C₆CH₂CH₂O, -ve DEPT), 32.20 (CH₃CH₂CH₂CH₅O, -ve DEPT), 68.86 (C₇CH₂O, -ve DEPT), 102.93 (Ar'-C₃, d, J_{CF} = 26.23 Hz, +ve DEPT), 111.36 (Ar'-C₆, d, J_{CF} = 3.01 Hz, +ve DEPT), 120.26 (Ar'-C₄, d, J_{CF} = 13.55 Hz, no DEPT), 121.59 (Ar-C₄, no DEPT), 130.72 (Ar-C₂, +ve DEPT), 130.76 (Ar-C₆, +ve DEPT), 131.05 (Ar'-C₅, d, J_{CF} = 5.35 Hz, +ve DEPT), 131.92 (Ar-C_{3/5}, +ve DEPT), 135.11 (Ar-C₁, no DEPT), 160.48 (Ar'-C₁, d, J_{CF} = 11 Hz, no DEPT), 160.54 (Ar'-C₂, d, J_{CF} = 247.94 Hz, no DEPT). ¹⁹F NMR (282MHz, CDCl₃, TMS) δ: -116.05 (IF, t, Ar'C₂-F, ³J_{HF} = 10.2 Hz, collapses to a singlet at -116.05 on decoupling). FT/IR (KBr/Drift) µₘₐₓ: 2924, 2856, 1619, 1486, 1467, 1327, 1305, 1269, 1234, 1172, 1028, 999, 957, 841, 833, 816 cm⁻¹. GC Analysis (inj = 300 °C, det = 300 °C, Oven = 120 °C hold 0.5min, then 40 °C min⁻¹ to 300 °C hold 4min) R_{T} = 7.02 (97.61 %) min.

***4-(2'-Fluoro-4-octyloxyphenyl)phenylboronic acid (30)*** Prepared using a similar procedure to compound **25.** Quantities used: Butyllithium (2 ml, 2.5 M in hexanes, 5 mmol), compound **28** (1.75 g, 4.62 mmol) and tetrahydrofuran (50 ml). Dried *in vacuo* (P₂O₅, 0.3mbar, RT, 16h) to give a viscous oil. Yield = 1.8 g (>100%); FT/IR (film/ATR/ZnSe) µₘₐₓ: 3205, 2924, 2855, 1607, 1532, 1477, 1398, 1340, 1309, 1286, 1230, 1161, 1123 cm⁻¹.

***4-(3'-Fluoro-4'-octyloxyphenyl)phenylboronic acid (31)*** Prepared using a similar procedure to compound **25.** Quantities used: Butyllithium (0.5 ml, 10.0 M in hexanes, 5 mmol), compound **29** (1.20 g, 3.17 mmol) and dry tetrahydrofuran (40 ml). Dried *in vacuo* (P₂O₅, 2.0 mbar, RT, 18 h) to give a viscous oil. Yield = 0.83 g (76%) FT/IR (film/ATR/ZnSe) µₘₐₓ: 3383, 3198, 2954, 2925, 2856, 1607, 1471, 1397, 1341, 1287, 1267, 1162, 1124 cm⁻¹.

***4-Octyloxyphenylboronic acid (33)*** Prepared using a similar procedure to compound **25.** Quantities used: Butyllithium (14.2 ml, 35.5 mmol), compound **32** (10.0 g, 35.1 mmol) and tetrahydrofuran (130ml). Dried *in vacuo* (P₂O₅, RT, 0.5mbar, 16 h).Yield = 8.89 g (>100%); FT/IR (KBr/Drift) µₘₐₓ: 3204, 3039, 2927, 2854, 1602, 1413, 1368, 1363, 1352, 1306, 1292, 1246, 1171, 838, 745, 688 cm⁻¹.

***4-(4'-Octyloxyphenyl)-bromo-2-fluorobenzene (35)*** This compound was prepared in a manner similar to compound **12.** Quantities used: compound **32** (4.00 g, 16 mmol), compound **34** (4.51 g, 15 mmol), palladium(0) *tetrakis*(triphenylphosphine) (0.58 g, 0.50 mmol), 2M sodium carbonate (50 ml) and 1,2-dimethoxyether (75 ml). Purified by flash chromatography [fine mesh silica gel: hexane] to give a white solid. GC analysis revealed a slight impurity which was removed by flash chromatography [fine mesh silica gel: hexane] to give a white crystalline solid. Dried *in vacuo* (P₂O₅, 0.5mbar, RT, 18 h). Yield = 1.52 g (27%); ¹H NMR (300MHz, CDCl₃, TMS) δ: 0.90 (3H, t, OC₇CH₃), 1.34 (8H, m, OC₃CH₂CH₂CH₂CH₂CH₃), 1.46 (2H, q, OC₂CH₂C₅), 1.80 (2H, q, OCH₂CH₂C₆), 3.99 (2H, t, OCH₂C₇), 6.96 (2H, dd, Ar'H₃/H₅, ³J_{HH} = 6.7 Hz), 7.26 (1H, m, ArH₃), 7.32 (2H, m, ArH₅/H₆), 7.43 (2H, dd, Ar'H₂/H₆, ³J_{HH} = 8.7 Hz). ¹³C NMR (75MHz, CDCl3, TMS) δ: 14.12 (OC₇CH₃, +ve DEPT), 22.67 (OC₆CH₂CH₃, -ve DEPT), 26.05 (OC₂CH₂C₅, -ve DEPT), 29.25 (OC₃CH₂CH₂C₃, -ve DEPT), 29.37 (OCH₂CH₂C₆, -ve DEPT), 31.82 (OC₅CH₂C₂, -ve DEPT), 68.06 (OCH₂C₇, -ve DEPT), 114.6 (Ar'-C₃/C₅, +ve DEPT), 119.6 (Ar-C₃, d, J_{CF} = 26.4 Hz, +ve DEPT), 120.5 (Ar-C₁, d, J_{CF} = 9.48 Hz, no DEPT), 126.8 (Ar'-C₁, d, J_{CF} = 1.4 Hz, no DEPT), 127.6 (Ar-C₅, d, J_{CF} = 3.72 Hz, +ve DEPT), 127.9 (Ar-C₄, d, J_{CF} = 4.17 Hz, no DEPT), 129.92 (Ar'-C₂/C₆, d, indistinguishable, +ve DEPT), 131.41 (Ar-C₆, d, J_{CF} = 4.17 Hz, +ve DEPT), 159.05 (Ar'-C₄, no DEPT), 159.43 (Ar-C₂, d, J_{CF} = 251.6 Hz, no DEPT). ¹⁹F NMR (282MHz, CDCl₃, TMS) δ: -115.684 (1F, t, ArC₂-F, ³J_{HF} = 8.9 Hz, collapses to singlet at -115.684 on decoupling). FT/IR (KBr/Drift) µₘₐₓ: 3078, 3045, 2925, 2860, 1558, 1527, 1474, 1391, 1250, 1123, 1036, 1010, 863, 821, 738, 572 cm⁻¹.

***4-(4'-Ocyloxyphenyl)-2-fluorophenyl boronic acid (36)*** Prepared using a similar procedure to compound **25.** Quantities used: Butyllithium (1.38 ml, 2.5M in hexanes, *ca*., 3.45 mmol), compound **35** (1.30 g, 3.40 mmol) and tetrahydrofuran (100 ml). Dried *in vacuo* (P₂O₅, 0.5 mbar, RT, 18 h), to give a red wax. Yield = 1.12g (95%). FT/IR (KBr/Drift) µₘₐₓ: 3350, 3204, 2924, 2853, 1607, 1522, 1398, 1374, 1363, 1347, 1253, 1182, 1036, 904, 819, 758, 678 cm⁻¹.

***4-(4'-Octyloxyphenyl)-3-fluorobenzene (38)*** This compound was prepared in a manner similar to compound **12.** Quantities used: compound **33** (4.2 g, 16.8 mmol), compound **37** (2.48 g, 11.2 mmol), palladium(0) *tetrakis*(triphenylphosphine) (0.43 g, 0.37 mmol), 2M sodium carbonate (50 ml) and 1,2-dimethoxyethane (75 ml). Purified by flash chromatography [fine mesh silica gel: hexanes] to give a colourless solid. Recrystallised (hexanes), filtered and dried *in vacuo* (P₂O₅, 0.4 mbar, RT, 5 h). Yield = 1.77 g (53%). ¹H NMR (300MHz, CDCl₃, TMS) δ: 0.89 (3H, t, CH₃C₇OAr), 1.30 (8H, m, CH₂'s), 1.45 (2H, m, CH₂), 1.80 (2H, q, CH₂), 3.98 (ArOCH₂C₇), 6.96 (3H, dd, Ar-H_{2/6} and Ar-H₂, ³J_{HH} 6.85 Hz), 7.24 (1H, dd, Ar-H_{4'}, ³J_{HF} 10.20 Hz), 7.33 (2H, m, Ar-H_{5'/6'}), 7.48 (2H, dd, Ar-H_{3/5}, ³J_{HH} 6.65 Hz). ¹³C NMR (75MHz, CDCl₃, TMS) δ: 14.18 (ArOC₇CH₃), 22.73 (ArOC₆CH₂CH₃, -ve DEPT), 26.12, 29.32, 29.43 (CH₂'s, -ve DEPT), 31.89 (ArOCH₂CH₂C₆, -ve DEPT), 68.14 (ArOCH₂C₇, -ve DEPT), 113.34, 113.50 (Ar-C_{2'} or C₄, d, J_{CF} 21.14 Hz and 21.93 Hz, +ve DEPT), 114.88 (Ar-C_{3/5}, +ve DEPT), 122.28 (Ar-C_{6'}, d, J_{CF} 2.72 Hz, +ve DEPT), 128.12 (Ar-C_{2/6}, +ve DEPT), 130.16 (Ar-C_{5'}, d, J_{CF} 8.36 Hz, +ve DEPT), 132.20 (Ar-C₁, d, J_{CF} 2.18 Hz, no DEPT), 143.21 (Ar-C_{1'}, J_{CF} 7.59 Hz, no DEPT), 159.18 (Ar-C₄, no DEPT), 163.26 (Ar-C₃, d, J_{CF} 245.20 Hz, no DEPT). ¹⁹F NMR (282MHz, CDCl₃, TMS) δ: -113.66 (1F, dt, ³J_{HF} 9.51 Hz, collapses to a singlet at -113.66 on decoupling). FT/IR (KBr/Drift) µₘₐₓ: 3072, 3039, 2954, 2933, 2856, 1590, 1521, 1473, 1291, 1265, 1188, 1123, 1036, 884, 835, 785, 692, 612, 533 cm⁻¹.

***4-(4-Octyloxyphenyl)-3-fluorophenylboronic acid (39)*** Prepared using a similar procedure to compound **25.** Quantities used: Butyllithium 2.02 ml, 2.5 M in hexanes, *ca.* 5.05 mmol), compound **38** (1.5 g, 5.00 mmol), trimethyl borate (1.04 g, 10.0 mmol) and dry tetrahydrofuran (115 ml). Dried *in vacuo* (P₂O₅, 0.6 mbar, RT, 18 h) to give a colourless solid. Yield = 1.61 g (94%). FT/IR (KBr/Drift) µₘₐₓ: 3211, 2955, 2922, 2857, 1590, 1522, 1488, 1473, 1447, 1394, 1291, 1265, 1191, 1123, 1030, 835, 785 cm⁻¹.

### EXAMPLE 4

***1,2-(S)-Dipentoxy-(4"-octyloxyterphenyl-3-fluoro-4-oxy)butane (40)*** This compound was prepared in a manner similar to compound **12.** Quantities used: Compound **11** (2.50 g, 7.7 mmol), compound **21** (2.14 g, 5.1 mmol), palladium(0) *tetrakis*(triphenylphosphine) (0.20 g, 0.17mmol), 2M sodium carbonate (25 ml) and 1,2-dimethoxyethane (35 ml). Purified by flash chromatography [fine mesh silica gel: 1:1 hexanes-dichloromethane] to give a v pale yellow solid. This was further purified [fine mesh silica gel: 5% ethyl acetate in hexanes] to give a white crystalline solid. This was further purified by flash chromatography [fine mesh silica gel: 4% ethyl acetate in hexane], then again [fine mesh silica gel: 4% ethyl acetate in hexanes] to give a white solid. This was further purified by recrystallisation, (hexanes x 4). Dried *in vacuo* (P₂O₅, RT, 0.1 mbar, 18 h). Yield = 0.25g, (8%). ¹H NMR (300MHz, CDCl₃, TMS) δ: 0.87 (9H, m, OC₄CH₃'s + ArOC₇CH₃), 1.31 (16H, m, OC₂CH₂CH₂CH₃'s + ArOC₃CH₂CH₂CH₂CH₂CH₃), 1.46 (2H, m, ArOC₂CH₂C₅), 1.58 (4H, m, OCH₂CH₂C₃'s), 1.81 (2H, q, ArOCH₂CH₂C₆), 1.98 (1H, m, ArOCH₂CH₂*CH), 2.08 (1H, m, ArOCH₂CH₂*CH), 3.44 (4H, m, OCH₂C₄'s), 3.50 (1H, m, *CHCH₂OC₅), 3.66 (1H, m, *CHCH₂OC₅), 3.73 (1H, m, ArOCH₂CH₂*CH), 3.99 (2H, t, ArOCH₂C₇), 4.21 (2H, m, ArOCH₂CH₂*CH), 6.98 (2H, d, Ar'-H₃/H₅, ³J_{HH} = 8.74 Hz), 7.05 (1H, t, Ar"-H₅, ³J_{HH} = 8.5 Hz), 7.35 (2H, dd, Ar"-H₂/H₆, ³J_{HF} = 10.70 Hz), 7.55 (2H, m, Ar'-H₂/H₆, ³J_{HH} = 8.68 Hz), 7.60 (4H, m, Ar-H₂/H₃/H₅/H₆). ¹³C NMR (75MHZ, CDCl₃, TMS) δ: 14.09 (OC₄CH₃'s + ArOC₇CH₃, +ve DEPT), 22.59 (OC₃CH₂CH₃'s + ArOC₆CH₂CH₃, -ve DEPT), 26.07 (ArOC₂CH₂C₅, -ve DEPT), 28.32 → 29.38 (CH₂'s, -ve DEPT), 29.81 (OCH₂CH₂C₃'s, -ve DEPT), 31.83 (ArOCH₂CH₂C₆, -ve DEPT), 31.93 (ArOCH₂CH₂*CH, -ve DEPT), 65.85 (ArOCH₂CH₂*CH, -ve DEPT), 68.07 (ArOCH₂C₇, -ve DEPT), 70.43 (OCH₂C₄'s, -ve DEPT), 72.38 (*CHCH₂OC₅, -ve DEPT), 75.04 (ArOCH₂CH₂*CH, +ve DEPT), 114.57 (Ar"-C₂, d, J_{CF} = 19.16 Hz, +ve DEPT), 114.80 (Ar'-C₂/C₅, +ve DEPT), 114.94 (Ar"-C₅, d, J_{CF} = 2.03 Hz, +ve DEPT), 122.40 (Ar"-C₆, d, J_{CF} = 3.18 Hz, +ve DEPT), 126.97 (Ar'-C₂/C₆, +ve DEPT). 127.94 (Ar-C₂/C₃/C₅/C₆, +ve DEPT), 132.97 (Ar'-C₁, no DEPT), 133.87 (Ar"-C₁, d, J_{CF} = 6.48 Hz, no DEPT), 137.93 (Ar-C₁, no DEPT), 139.66 (Ar-C₄, no DEPT), 146.4 (Ar"-C₄, d, J_{CF} = 10.86 Hz, no DEPT), 152.80 (Ar"-C₃, d, J_{CF} = 245.4 Hz, no DEPT), 158.8 (Ar'-C₄, no DEPT). ¹⁹F NMR (282MHz, CDCl3, TMS) δ: -134.71 (1F, t, Ar''C₃-F, ³J_{HF} = 8.54 Hz, collapses to singlet at -134.71 on decoupling). FT/IR (KBr/Drift) µₘₐₓ: 2955, 2928, 2860, 1506, 1497, 1466, 1300, 1273, 1244, 1135, 1118, 831, 806cm⁻¹. UV/Vis (0.005g, CHCl₃) λₘₐₓ: 292 (0.8486) nm. HPLC analysis (C18 column; 1 ml min⁻¹ 95:5 acetonitrile-tetrahydrofuran) R_{T} = 26.93 (98.83%) min.

### EXAMPLE 5

***1,2-(S)-Dipentoxy-(4"-octyloxyterphenyl-2-fluoro-4-oxy)butane (41)*** This compound was prepared in a manner similar to compound **12.** Quantities used: compound **11** (2.34 g, 7.2 mmol), compound **22** (2.00 g, 4.7 mmol), palladium(0) *tetrakis*(triphenylphosphine) (0.17 g, 0.14 mmol), 2M sodium carbonate (20 ml) and 1,2-dimethoxyethane (30 ml). Purified by flash chromatography [fine mesh silica gel, 1:1 hexanes-chloromethane then to give a pale yellow oil, which was further purified by flash chromatography [fine mesh silica gel: 5% ethyl acetate in hexanes] to give a viscous colourless fluid. Purified again [fine mesh silica gel: 1:1 hexanes-chloromethane] to give a viscous colourless fluid. Dried *in vacuo* (P₂O₅, RT, 0.1 mbar, 18 h). Yield = 1.06g (36%). ¹H NMR (300MHz, CDCl₃, TMS) δ: 0.88 (9H, m, OC₇CH₃ + OC₄CH₃), 1.45 (22H, m, OCH₂CH₂CH₂CH₂CH₃ + OCH₂CH₂CH₂CH₂CH₂CH₂CH₂CH₃), 1.81 (2H, q, OCH₂CH₂C₆), 2.03 (2H, m, OCH₂CH₂CH*), 3.47 (4H + 1H, m, *CHCH₂OC₅+ OCH₂C₄), 3.66 (1H + 1H, OCH₂CH₂CH* + *CHCH₂OC₅), 4.00 (2H, t, OCH₂C₇), 4.12 (2H, m, OCH₂CH₂CH*), 6.75 (2H, m, Ar"H₅/H₆), 6.98 (2H, m, Ar H₃/H₅), 7.38 (1H, t, Ar"H₃), 7.58 (6H, m, Ar H₂/H₆ + Ar'H₂/H₃/H₅/H₆). ¹³C NMR (75MHz, CDCl₃, TMS) δ: 14.42, 14.45, 14.50 (OC₇CH₃,+ve DEPT + OC₄CH₃, +ve DEPT) 22.90, 22.92, 23.06 (OC₆CH₂CH₃, -ve DEPT + OC₃CH₂CH₃, -ve DEPT), 26.46→32.33 (CH₂'s, -ve DEPT), 30.22 (OCH₂CH₂CH*, -ve DEPT), 65.21 (OCH₂CH₂CH*, -ve DEPT), 68.48 (OCH₂C₇, -ve DEPT), 70.87 (*CHCH₂OC₅, -ve DEPT), 72.09, 73.52 (OCH₂C₄, -ve DEPT), 75.51 (OCH₂CH₂CH*, +ve DEPT), 102.96 (Ar-C₃'', d, J_{CF} = 25 Hz, +ve DEPT), 111.21 (Ar-C₅'', d, J_{CF} = 3.6 Hz, +ve DEPT), 115.9 (Ar-C₃'/C₅', +ve DEPT), 121.25 (Ar-C₁'', d, J_{CF} = 13.1 Hz, no DEPT), 127.05 (Ar-C₂/C₃/C₅/C₆, +ve DEPT), 128.40 (Ar-C₂'/C₆', +ve DEPT), 129.45 (Ar-C₆", d, J_{CF} = 3 Hz, +ve DEPT), 131.20 (Ar-C₄, no DEPT), 134.47 (Ar-C₁', no DEPT), 134.97 (Ar-C₁, no DEPT), 159.17 (Ar-C₄', no DEPT), 160.01 (Ar C₄", d, J_{CF} = 10 Hz, no DEPT), 160.72 (Ar C₂", d, J_{CF} = 245.4 Hz, no DEPT). ¹⁹F NMR (282 MHz, CDCl₃, TMS) δ: 115.99 (1F, t, Ar C₂"-F, J_{HF} = 10.71 Hz, collapses to singlet at -115.99 on decoupling). FT/IR (Film/ATR/ZnSe) µₘₐₓ: -3853, 3744, 3648, 2954, 2929, 2857, 2361, 2336, 1666, 1560, 1491, 1295, 1249, 1123, 831, 818 cm⁻¹. UV/Vis (0.005g, CHCl₃)λₘₐₓ: 291.00 (1.1738) nm. HPLC analysis (C18 column; 1 ml min⁻¹ 95:5 acetonitrile-tetrahydrofuran) R_{T} = 26.09 (99.95%) min.

### EXAMPLE 6

***1,2-(S)-Dipentoxy-(2"-fluoro-4"-octyloxyterphenyl-4-oxy)butane (42)*** This compound was prepared in a manner similar to compound **12.** Quantities used: compound **30** (1.60 g, 4.65 mmol), compound **8a** (1.49 g, 3.72 mmol), palladium(0) *tetrakis*(triphenylphosphine) (0.18 g, 0.15 mmol), 2M sodium carbonate (25 ml) and 1,2-dimethoxyethane (30 ml). Purified by flash chromatography [fine mesh silica gel: 1:1 hexanes:dichloromethane] to give a pale yellow solid. Further purification [fine mesh silica gel: 10% ethyl acetate in hexanes] (twice) gave a white crystalline solid which was recrystallised (ethanol x 2) and dried *in vacuo* (P₂O₅, 0.3 mbar, 40°C, 16 h) to give a white crystalline solid. Yield = 0.56 g, (24%). ¹H NMR (300MHz, CDCl₃, TMS) δ: 0.88 (9H, m, OC₄CH₃'s + ArOC₇CH₃), 1.31 (16H, m, OC₂CH₂CH₂CH₃'s + ArOC₃CH₂CH₂CH₂CH₂CH₃), 1.46 (2H, m, ArOC₂CH₂C₅), 1.59 (4H, m, OCH₂CH₂C₃'s), 1.80 (2H, q, ArOCH₂CH₂C₆), 1.95 (1H, m, ArOCH₂CH₂*CH), 2.04 (1H, m, ArOCH₂CH₂*CH), 3.47 (4H + 1H, m, OCH₂C₄'s + *CHCH₂OC₅), 3.64 (1H, m, *CHCH₂OC₅), 3.70 (1H, m, ArOCH₂CH₂*CH), 3.97 (2H, t, ArOCH₂C₇), 4.14 (2H, m, ArOCH₂CH₂*CH), 6.72 (1H, dd, Ar'H₃, ³J_{HF} = 12.54 Hz), 6.77 (1H, dd, Ar'H₅, ³J_{HH} = 8.47 Hz), 6.99 (2H, dd, Ar"H_{3/}H₅, ³J_{HH} = 6.96 Hz), 7.38 (1H, t, Ar'H₆, ³J_{HH} = 8.8 Hz), 7.58 (4H + 2H, m, ArH₂/H₃/H₅/H₆ + Ar"H₂/H₆). ¹³C NMR (75MHz CDCl3, TMS) δ: 14.09(OC₄CH₃'s + ArOC₇CH₃, +ve DEPT), 22.59 (OC₃CH₂CH₃'s + ArOC₆CH₂CH₃, -ve DEPT), 26.01 (ArOC₂CH₂C₅, -ve DEPT), 28.34 (CH₂'s,-ve DEPT), 29.13 (ArOCH₂CH₂C₆, -ve DEPT), 29.32 (CH₂'s, -ve DEPT), 29.84 (OCH₂CH₂C₃'s, -ve DEPT), 31.82 (CH₂'s, -ve DEPT), 32.04 (ArOCH₂CH₂*CH, -ve DEPT), 64.6 (ArOCH₂CH₂*CH, -ve DEPT), 68.4 (ArOCH₂C₇, -ve DEPT), 70.5 (*CHCH₂OC₅, -ve DEPT), 72.44 (OCH₂C₄'s, -ve DEPT), 75.23 (ArOCH₂CH₂*CH, +ve DEPT), 102.49 (Ar'-C₃, d, J_{CF} = 26.52 Hz, +ve DEPT), 110.84 (Ar'-C₅, d, J_{CF} = 3.01 Hz, +ve DEPT), 114.78 (Ar"-C₃/C₅, +ve DEPT), 120.72 (Ar'-C₁, d, J_{CF} = 13.65 Hz, no DEPT), 126.65 (Ar-C₃/C₅, +ve DEPT), 128.01 (Ar"-C₂/C₆, +ve DEPT), 129.06 (Ar-C₂/C₆, d, indistinguishable, +ve DEPT), 130.79 (Ar'-C₆, d, J_{CF} = 5.44 Hz, +ve DEPT), 133.09 (Ar"-C₁, no DEPT), 134.1 (Ar-C₁, d, J_{CF} = 1.5 Hz, no DEPT), 139.54 (Ar-C₄, no DEPT), 158.63 (Ar"-C₄, no DEPT), 159.74 (Ar'-C₄, d, J_{CF} = 10.91 Hz, no DEPT), 160.32 (Ar'-C₂, d, J_{CF} = 247.38 Hz, no DEPT). ¹⁹F NMR (282MHz, CDCl3, TMS) δ: -116.00 (1F, t, Ar'C₂-F, ³J_{CF} = 10.73 Hz, collapses to a singlet at -116.00 on decoupling). FT/IR (KBr/Drift) µₘₐₓ: 2932, 2867, 1625, 1494, 1474, 1329, 1292, 1251, 1181, 1115, 841, 812 cm⁻¹. UV/Vis (0.005g, CHCl₃) λₘₐₓ: 291.5 (0.9197) nm. HPLC analysis (C18 column; 1 ml min⁻¹ 95:5 acetonitrile-tetrahydrofuran) R_{T} = 29.77 (99.66%) min.

### EXAMPLE 7

***1,2-(S)-Dipentoxy-(3"-fluoro-4"-octyloxyterphenyl-4-oxy)butane (43)*** This compound was prepared in a manner similar to compound **12.** Quantities used: compound **31** (0.75 g, 2.20 mmol), compound **8a** (0.70 g, 1.74 mmol), palladium(0) *tetrakis*(triphenylphosphine) (0.09 g, 0.07 mmol), 2M Sodium Carbonate (15ml) and 1,2-dimethoxyethane (20 ml). Purified by flash chromatography [Fine mesh silica gel: 6:4 hexane-dichloromethane] to give a pale oil. This was further purified by flash chromatography [fine mesh silica gel: 5% ethyl acetate in hexane] to give a white crystalline solid. This was further purified by repeated flash chromatography [fine mesh silica gel: 5% ethyl acetate in hexane] to give a viscous white liquid, then fine mesh silica gel: 6:4 hexane-dichloromethane and finally fine mesh silica gel: 12.5% diethyl ether in hexane. The resultant solid was then purified by repeated recrystallisation (ethanol x 3) to give a white crystalline solid. Dried *in vacuo* (P₂O₅, 0.2mbar, RT, 48 h). Yield = 0.10g (10%). ¹H NMR (300 MHz, CDCl₃, TMS) δ: 0.87 (9H, m, OC₄CH₃ + OC₇CH₃), 1.29 (16H, m, OC₂CH₂CH₂CH₃ + OC₃CH₂CH₂CH₂CH₂CH₃), 1.52 (6H, m, OC₂CH₂C₅ + OCH₂CH₂C₃), 1.80 (2H, q, OCH₂CH₂C₆), 1.95 (1H, m, ArOCH₂CH₂*CH), 2.05 (1H, m, ArOCH₂CH₂*CH), 3.46 (4H + 1H, m, OCH₂C₄ + *CHCH₂OC₅), 3.67 (1H + 1H, m, ArOCH₂CH₂*CH + *CHCH₂OC₅), 3.98 (2H, t, OCH₂C₇), 4.14 (2H, m, ArOCH₂CH₂*CH), 6.72 (1H, dd, Ar'-H₂, ³J_{HF} = 12.56 Hz), 6.77 (1H, dd, Ar'-H₅, ³J_{HH} = 8.49 Hz), 6.99 (2H, m, Ar"-H₃/H₅, ³J_{HH} = 8.77 Hz), 7.38 (1H, t, Ar'-H₆, ³J_{HH} = 8.82 Hz), 7.58 (6H, m, Ar-H₂,/H₃/H₅/H₆, + Ar"-H₂/H₆, ³J_{HH} = 8.84 Hz). 13C NMR (75 MHz, CDCl₃, TMS) δ: 14.48 (OC₄CH₃ + OC₇CH₃, +ve DEPT), 22.98 (CH₂'s, -ve DEPT), 26.41 (OCH₂CH₂CH₂C₅, -ve DEPT), 28.74 (CH₂'s, -ve DEPT), 29.53 (OCH₂CH₂C₅, -ve DEPT), 29.71 (CH₂'s, -ve DEPT), 30.24 (OCH₂CH₂C₃, -ve DEPT), 32.21 (ArOCH₂CH₂*CH, -ve DEPT), 64.83 (ArOCH₂CH₂*CH, -ve DEPT), 68.83 (OCH₂C₇, -ve DEPT), 70.88 (*CHCH₂OC₅, -ve DEPT), 72.85 (OCH₂C₄, -ve DEPT), 75.65 (ArOCH₂CH₂*CH, +ve DEPT), 102.91 (Ar'-C₂, d, J_{CF} = 26.34 Hz, +ve DEPT), 111.25 (Ar'-C₅, d, J_{CF} = 3.00 Hz, +ve DEPT), 115.19 (Ar"-C₅, +ve DEPT), 121.14 (Ar'-C₁, d, J_{CF} = 13.67 Hz, no DEPT), 127.05 (Ar-C₂/C₅, +ve DEPT), 128.41 (Ar"-C₂/C₆, +ve DEPT), 129.46 (Ar-C₂/C₆, +ve DEPT), 131.19 (Ar'-C₆, d, J_{CF} = 5.16 Hz, +ve DEPT), 134.5 (Ar"-C₁, no DEPT), 139.9 (Ar-C₁, no DEPT), 159.03 (Ar"-C₄, no DEPT), 160.15 (Ar'-C₃, d, J_{CF} = 247.1 Hz, no DEPT), 160.73 (Ar'-C₄, d, J_{CF} = 10.91 Hz, no DEPT). ¹⁹F NMR (282MHz, CDCl₃, TMS) δ: -116.02 (1F, t, Ar'C₃-F, ³J_{HF} = 10.8 Hz, collapses to singlet at -116.02 ppm on decoupling). FT/IR (KBr/Drift) µₘₐₓ: 2951, 2858, 1633, 1494, 1475, 1466, 1288, 1251, 1116, 838, 812 cm⁻¹. UV/Vis (0.005g, CHCl₃) λₘₐₓ: 291.50 (0.5916) nm. HPLC analysis (C18 column; 1 ml min⁻¹ 95:5 acetonitrile-tetrahydrofuran) R_{T} = 28.29 (100%) min.

### EXAMPLE 8

***1,2-(S)-Dipentoxy-(2'-fluoro-4"-octyloxyterphenyl-4-oxy)butane (45)*** This compound was prepared in a manner similar to compound **12.** Quantities used: compound **36** (1.0 g, 3.00 mmol), compound **8a** (0.8 g, 2.00 mmol), palladium(0) *tetrakis*(triphenylphosphine) (0.08 g, 0.07 mmol), 2M sodium carbonate (20 ml) and 1,2-dimethoxyethane (30 ml) under a dry nitrogen atmosphere. This was purified by flash chromatography [fine mesh silica gel, 1:1 hexane to dichloromethane] to give a pale yellow oil. A further column was then run [fine mesh silica gel, 1:1 hexane to dichloromethane to give a pale oil. This was further purified [fine mesh silica gel, 25% hexane in dichloromethane] to give a colourless oil. This was further purified by [fine mesh silica gel, 5% ethyl acetate in hexane] to give a white liquid crystal. Recrystallised [ethanol x 2, -78 °C] and then dried *in vacuo* (P₂O₅, 0.3 mbar, RT, 16 h). Yield = 0.07 g (6%). ¹H NMR (300 MHz, CDCl₃, TMS) δ: 0.88 (9H, m, ArOC₇CH₃ + OC₄CH₃'s), 1.30 (16H, m, ArOC₃CH₂CH₂CH₂CH₂CH₃ + OC₂CH₂CH₂CH₃'s), 1.47 (2H, m, ArOC₂CH₂C₅), 1.57 (4H, m, OCH₂CH₂C₃'s), 1.81 (2H, q, ArOCH₂CH₂C₆), 1.95 (1H, m, ArOCH₂CH₂*CH), 2.05 (1H, m, ArOCH₂CH₂*CH), 3.46 (4H + 1H, m, OCH₂C₄'s + *CHCH₂OC₅), 3.68 (1H + 1H, m, *CHCH₂OC₅ + ArOCH₂CH₂*CH), 4.00 (2H, t, ArOCH₂C₇), 4.14 (2H, m, ArOCH₂CH₂*CH), 6.98 (2H, d, ArH₃/H₅ + Ar"H₃/H₅, ³J_{HH} = 8.82 Hz), 6.99 (2H, d, ArH₃/H₅ + Ar"H₃/H₅, ³J_{HH} = 8.78 Hz), 7.33 (1H, dd, Ar'H₃, ³J_{HF} = 12.21 Hz), 7.38 (1H, dd, Ar'H₅, ³J_{HH} = 8.01 Hz), 7.45 (1H, t, Ar'H₆, ³J_{HH} = 8.05 Hz), 7.52 (2H, dd, Ar"H₂/H₆), 7.54 (2H, dd, ArH₂/H₆). ¹³C NMR (75 MHz, CDCl₃, TMS) δ: 14.05, 14.07, 14.13, (ArOC₇CH₃ + OC₄CH₃'s, +ve DEPT), 22.51, 22.54, 22.67 (ArOC₆CH₂CH₃ + OC₃CH₂CH₃, -ve DEPT), 26.07, 28.34, 29.27, 29.38, 29.84, 31.83 (CH₂'s, -ve DEPT), 32.03 (ArOCH₂CH₂*CH, -ve DEPT), 64.46 (ArOCH₂CH₂*CH, -ve DEPT), 68.04 (ArOCH₂C₇, -ve DEPT), 70.49, 71.68 (OCH₂C₄, -ve DEPT), 73.19 (*CHCH₂OC₅, -ve DEPT), 75.21 (ArOCH₂CH₂*CH, +ve DEPT), 113.98 (Ar'C₃, d, J_{CF} = 23.86 Hz, +ve DEPT), 114.49, 114.80 (ArC₃/C₅ + Ar''C₃/C₅, indistinguishable doublet), 122.32 (Ar'C₅, d, J_{CF} = 2.9 Hz, +ve DEPT), 126.7 (Ar'C₁, d, J_{CF} = 13.26 Hz, no DEPT), 127.69 (Ar"C₁, broad singlet, no DEPT), 127.91 (ArC₂/C₆, +ve DEPT), 129.99 (Ar''C₂/C₆, indistinguishable doublet, J_{CF} = 2.96 Hz, +ve DEPT), 130.59 (Ar'C₆, d, J_{CF} = 4.21 Hz, +ve DEPT), 131.93 (ArC₁, no DEPT), 141.34 (Ar'C₄, d, J_{CF} = 8.1 Hz, no DEPT), 158.75, 158.96 (Ar''C₄/ArC₄, indistinguishable doublet, no DEPT), 159.99 (Ar'C₂, d, J_{CF} = 246.4 Hz, no DEPT). ¹⁹F NMR (282 MHz, CDCl₃, TMS) δ:-118.62 (1F, dd, Ar'C₂-F, ³J_{HF} = 11.83 Hz, collapses to a singlet at -118.62 on decoupling). FT/IR (film/ATR/ZnSe) µₘₐₓ: 2954, 2927, 2856, 1609, 1545, 1487, 1468, 1292, 1247, 1180, 1111, 1045, 817 cm⁻¹. UV/Vis (0.005 g, CHCl₃) λₘₐₓ: 294.0 (0.4512) nm. HPLC analysis (C18 column; 1 ml min⁻¹ 95:5 acetonitrile-tetrahydrofuran) RT = 29.01 (99.02%) min.

### C. 1,(S)-Dipentoxy-(4"-octyloxy-di-fluoro-terphenyl-4-oxy)butanes (Examples 9 to 11)

### Summary

The routes employed are outlined in schemes 7, 8 and 9 below and the chemical steps are essential similar to those previously described with respect to schemes 1 to 6.

All intermediates and final materials (compounds **53, 58** and **63**) were rigorously purified by flash chromatography and repeated recrystallization. Purities and structural assignments were determined using a combination of HPLC, GC, NMR spectroscopy (¹H, ¹³C, ¹³C DEPT, COSY, CHCORR and ¹⁹F-{¹H}), IR spectroscopy and UV-Vis spectroscopy. The liquid crystal properties were determined using a combination of optical microscopy and DSC analysis.

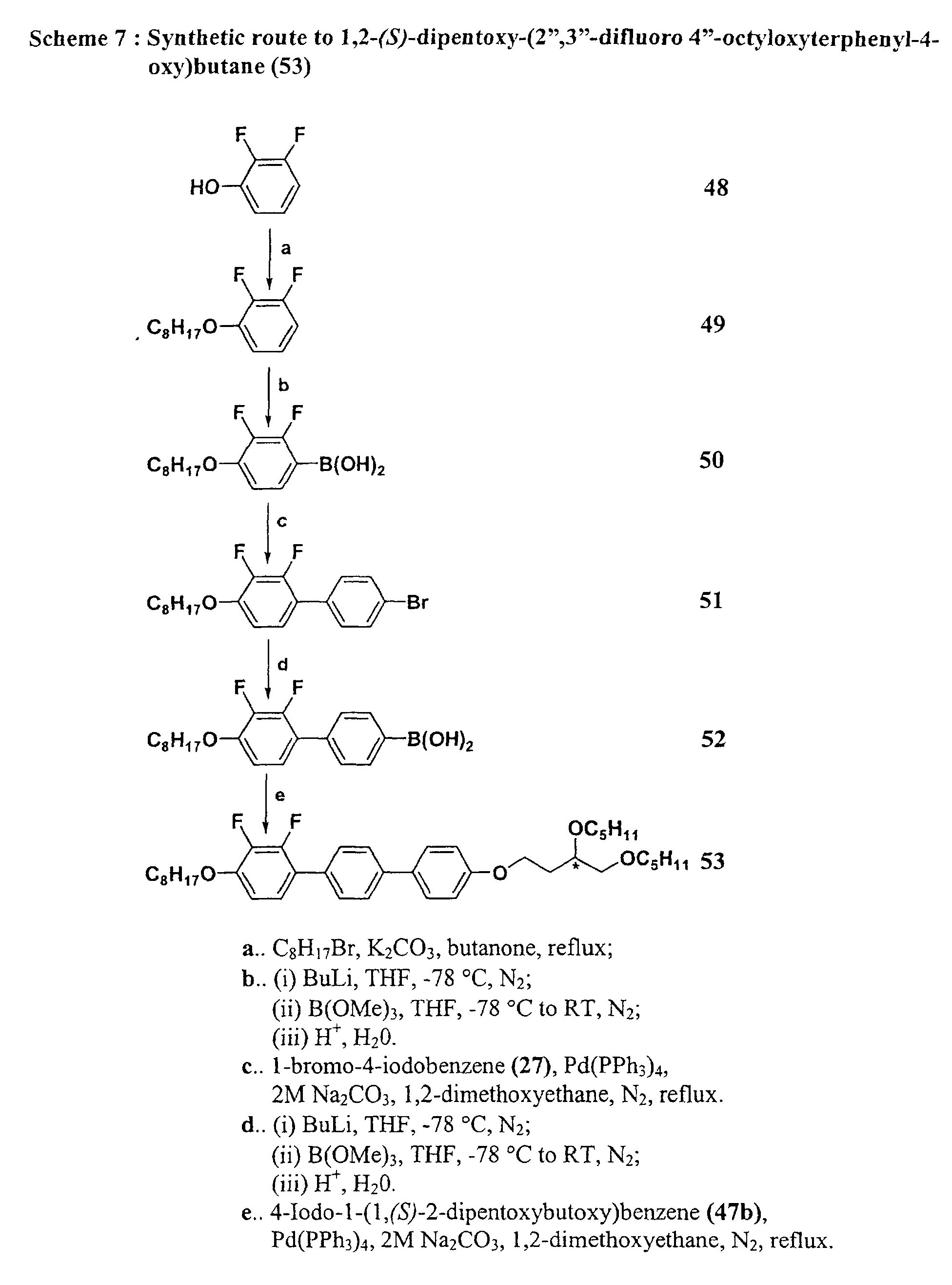

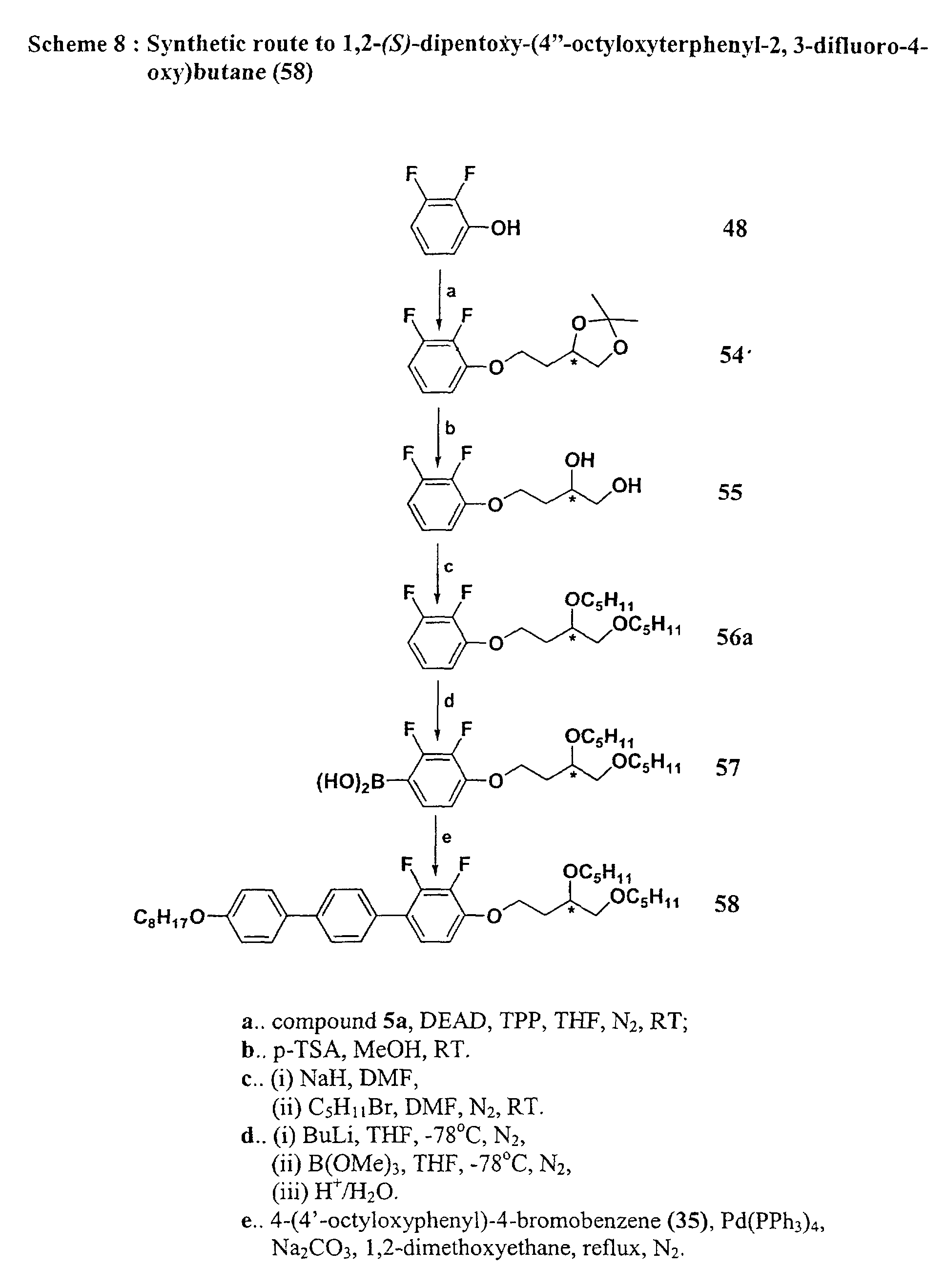

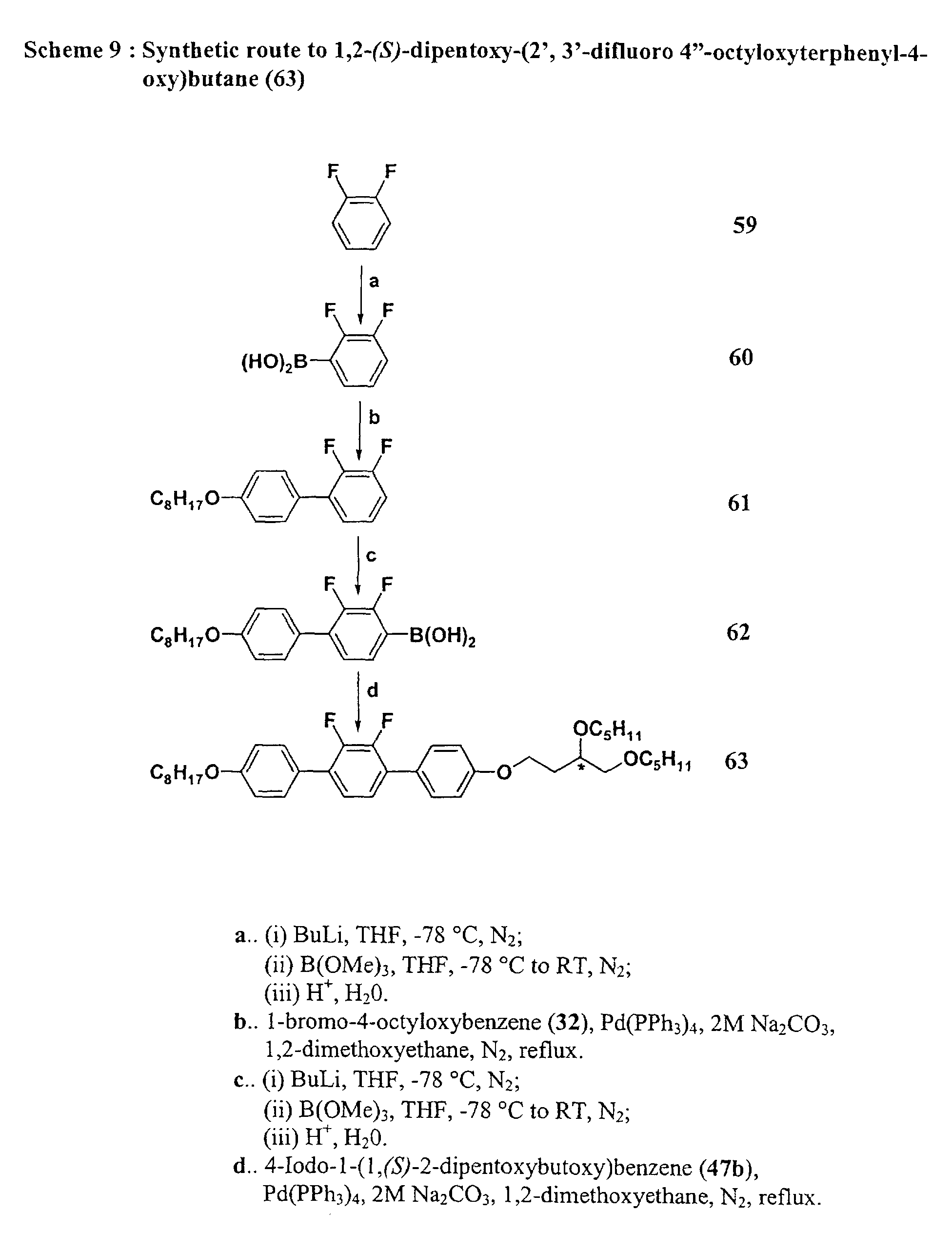

### Experimental Details

Compound **47b** is made using a route and procedures very similar to that described for scheme 2, the only difference being the use of 4-iodophenol instead of 4-bromophenol (**4**) as starting material. The route ultimately affording 4-iodo-1,(1,*(S)*-2-dihydroxybutoxy)benzene, prior to alkylation to give the dipentoxy compound **47b.** Characterising data is given only for compound **47b**

***4-Iodo-1-(1,(S)-2-dipentoxybutoxy)benzene (47b)*** A similar procedure to that used for compound **8a** was used. Quantities used: 1-bromopentane (4.70 g, 31.2 mmol), 4-iodo-1,(1,*(S)*-2-dihydroxybutoxy)benzene (3.20 g, 10.4 mmol), sodium hydride (1.66 g, 60% dispersion in oil, *ca.,* 41.5 mmol) and dimethylformamide (50 ml). Purified by flash chromatography [fine mesh silica gel, dichloromethane] to give a colourless oil. Dried *in vacuo* (P₂O₅, 0.3 mbar, RT, 18 h). Yield = 2.30 g, (49%); ¹H NMR (300 MHz, CDCl₃, TMS) δ: 0.87 (6H, m, OC₄CH₃'s), 1.29 (8H, m, OC₂CH₂CH₂CH₃'s), 1.55 (4H, m, OCH₂CH₂C₃'s), 1.91 (1H, m, ArOCH₂CH₂*CH), 2.00 (1H, m, ArOCH₂CH₂*CH), 3.43 (4H + 1H, m, OCH₂C₄'s + *CHCH₂OC₅), 3.64 (1H + 1H, m, *CHCH₂OC₅ + ArOCH₂CH₂*CH), 4.05 (2H, m, ArOCH₂CH₂*CH), 6.68 (2H, dd, ArH₂/H₆, ³J_{HH} = 6.86 Hz), 7.53 (2H, dd, ArH₃/H₅, ³J_{HH} = 6.71 Hz). ¹³C NMR (75 MHz, CDCl₃, TMS) δ: 14.04, 14.07 (OC₄CH₃'s, +ve DEPT), 22.48, 22.52 (OC₃CH₂CH₃'s, -ve DEPT), 28.32, 29.36, 29.80 (CH₂'s, -ve DEPT), 31.90 (ArOCH₂CH₂*CH, -ve DEPT), 64.44 (ArOCH₂CH₂*CH, -ve DEPT), 70.42, 71.56 (OCH₂C₄'s, -ve DEPT), 73.11 (*CHCH₂OC₅, -ve DEPT), 75.09 (ArOCH₂CH₂*CH, +ve DEPT), 82.47 (ArC₄, no DEPT), 116.89 (ArC₂/C₆, +ve DEPT), 138.12 (ArC₃/C₅, +ve DEPT), 158.86 (ArC₁, no DEPT). FT/IR (film/ATR/ZnSe) µₘₐₓ: 2954, 2929, 2858, 1587, 1486, 1467, 1282, 1242, 1174, 1109, 999, 928, 818, 732 cm⁻¹.

***2,3-Difluoro-4-octyloxybenzene (49)*** A similar procedure to that used for compound **24** was used. Quantities used: 1-Bromooctane (22.26 g, 115.30 mmol), 2,3-difluorophenol (**48**) (10 g, 76.86 mmol), potassium carbonate (21.25 g, 153.72 mmol) and butanone (160 ml). Purified by flash chromatography [fine mesh silica gel, 1:1 dichloromethane to hexanes] to give a colourless oil. Dried *in vacuo* (P₂O₅, 0.7 mbar, RT, 22 h). Yield = 15.83 g (85%); ¹H NMR (300MHz, CDCl₃, TMS) δ: 0.88 (3H, t, ArOC₇CH₃), 1.33 (8H, m, ArOC₃CH₂CH₂CH₂CH₂CH₃), 1.84 (2H, m, ArOCH₂CH₂C₆), 4.02 (2H, t, ArOCH₂C₇), 6.72 (1H, m, Ar-H₄), 6.97 (2H, m, Ar-H₅/₆). FT/IR (ZnSe/film/ATR) µₘₐₓ: 2954, 2926, 2873, 2866, 1617, 1513, 1418, 1317, 1291, 1253, 1074, 764. 1706 cm⁻¹.

***2,3-Difluoro-4-octyloxyphenylboronic acid (50)*** A similar procedure to that used for compound **25** was used. Quantities used: Butyllithium (16.6 ml, 2.5M in hexanes, 41.5 mmol), compound **49** (10.00 g, 41.3 mmol), trimethylborate (8.58 g, 82.5 mmol) and dry tetrahydrofuran (130 ml). Dried *in vacuo* (P₂O₅, 0.3 mbar, RT, 20 h), to give a brown sludge. Yield = 10.50 g (89%); FT/IR (KBr/Drift) µₘₐₓ: 3451, 3321, 3198, 2964, 2925, 2855, 1624, 1520, 1467, 1359, 1290, 1211, 1078 cm⁻¹.

***4-(2',3'-Difluoro-4'-octyloxyphenyl)-4-bromobenzene (51)*** A similar procedure to that used for compound **28** was used. Quantities used: compound **50** (7.00 g, 24.4 mmol), 4-bromo-1-iodobenzene **(27)** (4.61 g, 16.3 mmol), palladium(0) *tetrakis*(triphenylphosphine) (0.63 g, 0.54 mmol), 2M sodium carbonate (70 ml) and 1,2-dimethoxyethane (100 ml). Purified by flash chromatography [fine mesh silica gel, hexanes] to give a off white solid. This was then purified by recrystalisation [hexanes x 2] to give a colourless crystalline solid. Dried *in vacuo* (P₂O₅, 0.3 mbar, RT, 84 h). Yield = 2.20 g (34%); ¹H NMR (300 MHz, CDCl₃, TMS) δ: 0.89 (3H, t, ArOC₇CH₃), 1.31 (8H, m, ArOC₃CH₂CH₂CH₂CH₂CH₃), 1.47 (2H, m, ArOC₂CH₂C₅), 1.82 (2H, q, ArOCH₂CH₂C₆), 4.06 (2H, t, ArOCH₂C₇), 6.78 (1H, dt, Ar'H₅, ³J_{HH} = 9.10 Hz), 7.04 (1H, dt, Ar'H₆, ³J_{HH} = 9.63 Hz), 7.36 (2H, dd, Ar-H₂/H₆, ³J_{HH} = 8.50 Hz), 7.55 (2H, dd, Ar-H₃/H₅, ³J_{HH} = 6.65 Hz). ¹³C NMR (75 MHz, CDCl₃, TMS) δ: 14.12 (ArOC₇CH₃, +ve DEPT), 22.67 (ArOC₆CH₂CH₃, -ve DEPT), 25.88, 29.11, 29.23, 29.31, 31.81 (CH₂'s, -ve DEPT), 69.85 (ArOCH₂C₇, -ve DEPT), 109.53 (Ar'C₅, d, J_{CF} = 3.4 Hz, +ve DEPT), 121.65 (Ar'C₁, d, J_{CF} = 10.9 Hz, no DEPT), 121.83 (ArC₄, no DEPT), 123.35 (Ar'C₆, t, J_{CF} = 4.06 Hz, +ve DEPT), 130.29 (Ar-C₂/C₆, indistinguishable doublet, J_{CF} = 2.95 Hz, +ve DEPT), 131.70 (Ar-C₃/C₅, +ve DEPT), 133.84 (Ar-C₁, broad doublet, J_{CF} = 2.0 Hz, no DEPT), 141.73 (Ar'C₃, dd, J_{CF} = 247.5 Hz, no DEPT), 148.23 (Ar'C₄, dd, J_{CF} = 8.05 Hz, no DEPT), 148.70 (Ar'C₂, dd, J_{CF} = 249.1 Hz, no DEPT). ¹⁹F NMR (282 MHz, CDCl₃, TMS) δ: -142.1 (1F, dd, Ar'F₂/F₃, ³J_{FF} = 19.5 Hz, collapses to a doublet at -142.1 on decoupling), -158.9 (1F, dd, Ar'F₂/F₃, ³J_{FF} = 19.8 Hz, collapses to a doublet at -158.9 on decoupling). FT/IR (KBr/drift) µₘₐₓ: 2959, 2921, 2854, 1521, 1494, 1468, 1314, 1300, 1199, 1110, 1073, 892, 833, 800, 746, 717, 500, 411, 405 cm⁻¹.

***4-(2',3'-Difluoro-4'-octyloxyphenyl)phenylboronic acid (52)*** A similar procedure to that used for compound **25** was used. Quantities used: Butyllithium (1.78 ml, 2.5M in hexanes, 4.45 mmol) compound **51** (1.75 g, 4.40 mmol), trimethylborate (0.92 g, 8.81 mmol) and dry tetrahydrofuran (115 ml). Dried *in vacuo* (P₂O₅, 0.3 mbar, RT, 20 h), to give a pale wax-like solid. Yield = 1.65 g (>100%); FT/IR (KBr/Drift) µₘₐₓ: 3337, 3211, 2955, 2924, 2855, 1607, 1521, 1467, 1401, 1315, 1198, 1078, 893, 836, 801, 765, 699 cm⁻¹.

### EXAMPLE 9

***1,2-(S)-Dipentoxy-(2",3"-difluoro-4"-octyloxyterphenyl-4-oxy)butane (53)*** A similar procedure to that used for compound **28** was employed. Quantities used: compound **52** (1.21 g, 3.34 mmol), compound **47b** (1.00 g, 2.23 mmol), palladium(0) *tetrakis*(triphenylphosphine) (0.08 g, 0.07 mmol), 2M sodium carbonate (20 ml) and 1,2-dimethoxyethane (30 ml). Purified by flash chromatography [fine mesh silica gel, 1:1 hexanes to dichloromethane] to give a off white solid. This was further purified by flash chromatography [fine mesh silica gel, 7.5% ethyl acetate in hexanes to give a colourless solid. This was again purified [7.5% ethyl acetate in hexanes]. Dried *in vacuo* (P₂O₅, 0.5 mbar, RT, 20 h). Yield = 0.40 g (28%); ¹H NMR (300 MHz, CDCl₃, TMS) δ: 0.88 (9H, m, ArOC₇CH₃ + OC₄CH₃'s), 1.32 (16H, m, ArOC₃CH₂CH₂CH₂CH₂CH₃ + OC₂CH₂CH₂CH₃'s), 1.48 (2H, m, ArOC₂CH₂C₅), 1.56 (4H, m, OCH₂CH₂C₃'s), 1.84 (2H, q, ArOCH₂CH₂C₆), 1.95 (1H, m, ArOCH₂CH₂*CH), 2.05 (1H, m, ArOCH₂CH₂*CH), 3.46 (4H + 1H, m, OCH₂C₄'s + *CHCH₂OC₅), 3.67 (1H + 1H, m, *CHCH₂OC₅ + ArOCH₂CH₂*CH), 4.07 (2H, t, ArOCH₂C₇), 4.14 (2H, m, ArOCH₂CH₂*CH), 6.80 (1H, ddd, Ar"H₅, ³J_{HH} = 7.63 Hz), 6.99 (2H, dd, Ar-H₃/H₅, ³J_{HH} = 6.79 Hz), 7.13 (1H, ddd, Ar"H₆, ³J_{HH} = 8.44 Hz), 7.55 (4H, dd, Ar'H₂/₃/₅/₆, ³J_{HH} = 6.77 Hz), 7.62 (2H, dd, Ar-H₂/H₆, ³J_{HH} = 6.52 Hz). ¹³C NMR (75 MHz, CDCl₃, TMS) δ: 14.43, 14.45, 14.50 (ArOC₇CH₃ + OC₄CH₃'s, +ve DEPT), 22.90, 22.94, 23.05 (ArOC₆CH₂CH₃ + OC₃CH₂CH₃'s, -ve DEPT), 26.28, 28.73, 28.75, 29.56, 29.61, 29.70, 29.79, 30.24, 32.20 (CH₂'s, -ve DEPT), 32.46 (ArOCH₂CH₂*CH, -ve DEPT), 64.86 (ArOCH₂CH₂*CH, -ve DEPT), 70.29 (ArOCH₂C₇, -ve DEPT), 70.87, 72.08 (OCH₂C₄'s, -ve DEPT), 73.63 (*CHCH₂OC₅, -ve DEPT), 75.66 (ArOCH₂CH₂*CH, +ve DEPT), 109.97 (Ar''C₅, d, J_{CF} = 2.53 Hz, +ve DEPT), 115.24 (Ar-C₃/C₅, +ve DEPT), 123.01 (Ar"C₁, d, J_{CF} = 10.89 Hz, no DEPT), 123.83 (Ar"C₆, t, J_{CF} = 4.08 Hz, +ve DEPT), 127.16 (Ar-C₂/C₆, +ve DEPT), 128.43 (Ar'C₃/C₅, +ve DEPT), 129.43 (Ar'C₂/C₆, indistinguishable doublet, J_{CF} = 3.03 Hz, +ve DEPT), 133.10 (Ar-C₁, no DEPT), 133.59 (Ar'C₁, broad singlet, no DEPT), 140.50 (Ar'C₄, no DEPT), 142.33 (Ar''C₃, dd, J_{CF} = 246.84 Hz, no DEPT), 148.24 (Ar''C₄, t, J_{CF} = 4.07 Hz, no DEPT), 149.31 (Ar''C₂, dd, J_{CF} = 248.76 Hz, no DEPT), 159.16 (Ar-C₄, no DEPT). ¹⁹F NMR (282 MHz, CDCl₃, TMS) δ: -142.11 (1F, dd, Ar"C₂/C₃-F, ³J_{FF} = 19.69 Hz, collapses to a doublet at -142.11 {³J_{FF} = 19.69 Hz} on decoupling), -159.13 (1F, dd, Ar"C₂/C₃-F, ³J_{FF} = 19.81 Hz, collapses to a doublet at -159.13 {³J_{FF} = 19.66 Hz} on decoupling). FT/IR (film/ATR/ZnSe) µ µₘₐₓ: 2954, 2928, 2857, 1607, 1533, 1497, 1469, 1290, 1247, 1178, 1108, 1076, 897, 824, 798, 736 cm⁻¹. UV/Vis (0.5 mg, CHCl₃) λₘₐₓ: 290.5 (0.6988) nm. HPLC analysis (C₁₈ column; 1 ml min⁻¹ 95:5 acetonitrile to tetrahydrofuran) R_{T} = 21.49 (100%) min.

***1-{(2,2-Dimethyl)-1,(S)-2-dioxolan-4-yl)ethoxy-2,3-difluorobenzene (54)*** A similar procedure was used to that for compound **5a** was used. Quantities used: Compound **2a/b** (9.07 g, 62.1 mmol), triphenylphosphine (10.83 g, 41.3 mmol), 2,3-difluorophenol (**48**) (5.39 g, 41.3 mmol), diethyl azodicarboxylate (7.20 g, 41.3 mmol) and tetrahydrofuran (145 ml). Purified by flash chromatography [fine mesh silica gel, 10% ethyl acetate in hexanes] to give a pale oil. Dried *in vacuo* (P₂O₅, 10 mbar, R.T, 3 h) Yield = 6.74 g (63%); ¹H NMR (300 MHz, CDCl₃, TMS) δ: 1.36 (3H, s, CH₃CO₂), 1.42 (3H, s, CH₃CO₂), 2.08 (2H, m, ArOCH₂CH₂*CH), 3.66 (1H, t, *CHCH₂O), 4.18 (2H +1H, m, ArOCH₂CH₂*CH + *CHCH₂O), 4.27 (1H, ArOCH₂CH₂*CH), 6.76 (2H, m, ArH₅/H₆), 6.97 (1H, m, ArH₄). ¹³C NMR (75 MHz, CDCl₃, TMS) δ: 25.68, 26.96 (O₂CCH₃'s, +ve DEPT), 33.47 (ArOCH₂CH₂*CH, -ve DEPT), 66.48 (ArOCH₂CH₂*CH, -ve DEPT), 69.52 (*CHCH₂O, -ve DEPT), 73.21 (ArOCH₂CH₂*CH, -ve DEPT), 108.91 (O₂C(CH₃)₂, no DEPT), 109.27 (ArC₄, d, J_{CF} = 17.6 Hz, no DEPT), 109.71 (ArC₆, d, J_{CF} = 2.9 Hz, +ve DEPT), 123.17 (ArC₅, dd, J_{CF} = 8.6 Hz, +ve DEPT), 141.41 (ArC₂, dd, J_{CF} = 247.3 Hz, no DEPT), 148.53 (ArC₁, dd, J_{CF} = 10.93 Hz, no DEPT), 151.45 (ArC₃, dd, J_{CF} = 246.7 Hz, no DEPT). FT/IR (film/ATR/ZnSe) µₘₐₓ: 2986, 2938, 2882, 1620, 1513, 1481, 1380, 1317, 1292, 1252, 1219, 1159, 1071, 856, 765, 706 cm⁻¹.

***2,3-Difluoro-1-(1,(S)-2-dihydroxybutoxy)benzene (55)*** A similar procedure to that used for compound **6a** was used. Quantities used: *p*-toluenesulfonic Acid (500 mg), compound **54** (5.25 g, 18.9 mmol) and methanol (460 ml). Dried *in vacuo* (5 mbar, 30°C, 18 h). Yield = 4.62 g (86%); ¹H NMR (300MHz, CDCl₃, TMS) δ: 1.95 (2H, m, OCH₂CH₂*CH), 2.74 (1H, broad s, *CHCH₂OH), 3.12 (1H, broad s, *CHOH), 3.57 (1H, m, *CHCH₂OH), 3.70 (1H, m, *CHCH₂OH), 4.01 (1H, m, ArOCH₂CH₂*CH), 4.18 (2H, m, ArOCH₂CH₂*CH), 6.74 (2H, m, ArH₄/H₅), 6.94 (1H, m, ArH₃). ¹³C NMR (75MHz, CDCl₃, TMS) δ: 32.44 (ArOCH₂CH₂*CH,-ve DEPT), 66.69 (ArOCH₂CH₂*CH, -ve DEPT), 66.85 (*CHCH₂OH, -ve DEPT), 69.71 (ArOCH₂CH₂*CH, +ve DEPT), 109.45 (Ar-C₄, d, J_{CF} = 17.6 Hz, +ve DEPT), 109.56 (Ar-C₆, d, J_{CF} = 2.90 Hz, +ve DEPT), 123.28 (Ar-C₅, dd, J_{CF} = 8.55 Hz, +ve DEPT), 141.39 (Ar-C₂, dd, J_{CF} = 246.9 Hz, no DEPT), 148.35 (Ar-C₁, t, J_{CF} = 3.96 Hz, no DEPT), 151.38 (Ar-C₃, dd, J_{CF} = 247.1 Hz, no DEPT). ¹⁹F NMR (282 MHz, CDCl₃, TMS) δ: -137.38 (1F, ddd, ArC₂/₃-F, ³J_{FF} = 19.78, collapses to doublet at -137.77 {³J_{HH} = 19.94 Hz} on decoupling), -160.17 (1F, ddd, ArC₂/₃-F, ³J_{FF} = 19.75, collapses to doublet at -160.21 {³J_{HH} = 19.95 Hz} on decoupling). FT/IR (KBr/Drift) µₘₐₓ:- 3277, 2979, 2952, 2926, 2873, 1627, 1509, 1481, 1318, 1085, 1058, 764, 697 cm⁻¹.

***2,3-Difluoro-1-(1,(S)-2-dipentoxybutoxy)benzene (56a)*** A similar procedure to that used for compound **8a** was used. Quantities used: 1-bromopentane (6.84 g, 22.6 mmol), compound **55** (4.02 g, 18 mmol), sodium hydride (2.72 g, 60% dispersion in oil *ca.,* 68 mmol) and dimethylformamide (50 ml). Purified by flash chromatography [fine mesh silica gel, 8:2 dichloromethane-hexanes]. Dried *in vacuo* (P₂O₅, 0.7 mbar, RT, 16 h) to give 2 compounds; Compound **56a,** Yield = 2.95 g (45%) and compound **56b,** Yield = 1.15 g. Compound **56a;** ¹H NMR (300 MHz, CDCl₃, TMS) δ: 0.83 (6H, t, OC₄CH₃'s), 1.25 (8H, m, OC₂CH₂CH₂CH₃'s), 1.55 (4H, m, OCH₂CH₂C₃'s), 1.94 (2H, m, ArOCH₂CH₂*CH), 3.44 (4H + 1H, m, OCH₂C₄'s + *CHCH₂OC₅), 3.65 (1H + 1H, m, *CHCH₂OC₅ + ArOCH₂CH₂CH) 4.15 (2H, m, ArOCH₂CH₂*CH), 6.74 (2H, m, Ar-H₂/₃), 6.93 (1H, m, Ar-H₄). ¹³C NMR (75 MHz, CDCl₃, TMS) δ: 14.34, 14.39 (OC₄CH₃'s, +ve DEPT), 22.84, 22.89 (OC₃CH₂CH₃'s, -ve DEPT) 28.70, 29.75, 30.08, 30.16 (CH₂'s, -ve DEPT), 32.26 (ArOCH₂CH₂*CH, -ve DEPT), 66.57 (ArOCH₂CH₂*CH, -ve DEPT), 70.75 (*CHCH₂OC₅, -ve DEPT), 72.02, 73.40 (OCH₂C₄, -ve DEPT), 75.33 (ArOCH₂CH₂*CH, +ve DEPT), 109.29 (Ar-C₄, d, J_{CF} = 13.68 Hz, +ve DEPT), 110.10 (Ar-C₆, d, J_{CF} = 2.94 Hz, +ve DEPT), 123.44 (Ar-C₅, dd, J_{CF} = 8.72 Hz, +ve DEPT), 141.81 (Ar-C₂, dd, J_{CF} = 247.1 Hz, no DEPT), 149.12 (Ar-C₁, t, J_{CF} = 5.42 Hz, no DEPT), 151.81 (Ar-C₃, dd, J_{CF} = 246.3 Hz, no DEPT). FT/IR (film/ATR/ZnSe) µₘₐₓ: 2955, 2927, 2857, 1514, 1481, 1472, 1466, 1253, 1108, 1074 cm⁻¹. **2,3-Difluoro-1-(1-pentoxy-*(S)*-2-hydroxybutoxy)benzene (56b)** ¹H NMR (300 MHz, CDCl₃, TMS) δ: 0.87 (3H, t, OC₄CH₃), 1.29 (4H, m, OC₂CH₂CH₂CH₃), 1.56 (2H, m, OCH₂CH₂C₃) 1.92 (2H, m, OCH₂CH₂CH), 2.80 (1H, Broad, s, *CHOH), 3.36 (1H, m, CHCH₂OC₅), 3.46 (2H + 1H, m, CHCH₂OC₅ + OCH₂C₄), 4.04 (1H, m, CHCH₂OC₅), 4.17 (2H, m, OCH₂CH₂CH), 6.73 (2H, m, Ar-H₅/H₆), 6.91 (1H, m, ArH₄). ¹³C NMR (75 MHz, CDCl₃, TMS) δ: 14.36 (OC₄CH₃, +ve DEPT), 22.87 (OC₃CH₂CH₃,-ve DEPT), 28.63 (OC₂CH₂CH₂CH₃, -ve DEPT), 29.66 (OCH₂CH₂C₃, -ve DEPT), 33.12 (OCH₂CH₂CH, -ve DEPT), 66.86 (OCH₂CH₂CH, -ve DEPT), 67.88 (OCH₂CH₂CH, +ve DEPT), 71.92 (OCH₂C₄, -ve DEPT), 75.23 (CHCH₂OC₅, -ve DEPT), 109.44 (ArC₄, +ve DEPT), 110.09 (ArC₆, +ve DEPT), 123.52 (ArC₅, +ve DEPT), 141.73 (ArC₂, no DEPT), 148.96 (ArC₁, no DEPT), 151.51 (ArC₃, no DEPT), FT/IR (Film/ATR/ZnSe) µₘₐₓ:- 3443, 2972, 2933, 2863, 1620, 1513, 1481, 1317, 1292, 1253, 1075, 1068, 765 cm⁻¹.

***2,3-Difluoro-1-(1,(S)-2-dipentoxybutoxy)phenyl-4-boronic acid*** *(57)* A similar procedure to that used for compound **25** was used. Quantities used: Butyllithium (2.5 ml, 2.5M in hexanes, 6.25 mmol), compound **56a** (2.22 g, 6.20 mmol), trimethylborate (1.29 g, 12.40 mmol) and dry tetrahydrofuran (115 ml). Dried *in vacuo* (P₂O₅, 0.4 mbar, RT, 96 h). Yield = 2.41 g (97%); FT/IR (film/ATR/ZnSe) µₘₐₓ: 3203, 2964, 2927, 2858, 1625, 1514, 1481, 1377, 1349, 1294, 1254, 1230, 1076, 761 cm⁻¹.

### EXAMPLE 10

***1,2-(S)-Dipentoxy-(4"-octyloxyterphenyl-2,3-difluoro-4-oxy)butane (58)*** A similar procedure to that used for compound **28** was employed. Quantities used: compound **57** (1.21 g, 3.34 mmol), compound **35** (1.37 g, 3.81mmol), palladium(0) *tetrakis*(triphenylphosphine) (0.13 g, 0.12 mmol), 2M sodium carbonate (20 ml) and 1,2-dimethoxyethane (30 ml). Purified by flash chromatography [fine mesh silica gel, 1:1 hexanes to dichloromethane] to give a off white solid. This was further purified by flash chromatography [fine mesh silica gel, 2.5% ethyl acetate in hexanes], then again with [fine mesh silica gel, 2.5% ethyl acetate in hexanes]. Dried *in vacuo* (P₂O₅, 0.3 mbar, RT, 22 h). Yield 0.40 g (16 %); ¹H NMR (300 MHz, CDCl₃, TMS) δ: 0.88 (9H, m, ArOC₇CH₃ + OC₄CH₃'s), 1.32 (16H, m, ArOC₃CH₂CH₂CH₂CH₂CH₃ + OC₂CH₂CH₂CH₃'s), 1.47 (2H, m, ArOC₂CH₂C₅), 1.57 (4H, m, OCH₂CH₂C₃'s), 1.79 (2H, q, ArOCH₂CH₂C₆), 1.99 (1H, m, ArOCH₂CH₂*CH), 2.10 (1H, m, ArOCH₂CH₂*CH), 3.46 (4H + 1H, m, OCH₂C₄'s + *CHCH₂OC₅), 3.68 (1H + 1H, m, *CHCH₂OC₅ + ArOCH₂CH₂*CH), 4.00 (2H, t, ArOCH₂C₇), 4.32 (2H, m, ArOCH₂CH₂*CH), 6.83 (1H, ddd, Ar-H₅, ³J_{HH} = 7.66 Hz), 6.98 (2H, dd, Ar"H₃/H₅, ³J_{HH} = 6.79 Hz), 7.13 (1H, ddd, Ar-H₆, ³J_{HH} = 8.44 Hz), 7.55 (4H, dd, Ar'H₂/₃/₅/₆, ³J_{HH} = 6.67 Hz), 7.62 (2H, dd, Ar"H₂/H₆, ³J_{HH} = 6.57 Hz). ¹³C NMR (75 MHz, CDCl₃, TMS) δ: 14.03, 14.07, 14.12 (ArOC₇CH₃ + OC₄CH₃'s, +ve DEPT), 22.51, 22.54, 22.68 (ArOC₆CH₂CH₃ + OC₃CH₂CH₃'s, -ve DEPT), 26.09, 28.35, 28.37, 29.31, 29.39, 29.53, 31.84 (CH₂'s, -ve DEPT), 31.91 (ArOCH₂CH₂*CH, -ve DEPT), 66.27 (ArOCH₂CH₂*CH, -ve DEPT), 68.10 (ArOCH₂C₇,-ve DEPT), 70.44, 71.70 (OCH₂C₄'s, -ve DEPT), 73.04 (*CHCH₂OC₅, -ve DEPT), 74.97 (ArOCH₂CH₂*CH, +ve DEPT), 109.53 (Ar-C₅, broad s, +ve DEPT), 114.84 (Ar"C₃/C₅, +ve DEPT), 122.7 (Ar-C₁, d, J_{CF} = 10.96 Hz, no DEPT), 123.49 (Ar-C₆, t, J_{CF} = 4.08 Hz, +ve DEPT), 126.77 (Ar"C₂/C₆, +ve DEPT), 128.04 (Ar'C₂/C₆, +ve DEPT), 129.04 (Ar'C₃/C₅, indistinguishable doublet, J_{CF} = 2.98 Hz, +ve DEPT), 132.83 (Ar"C₁, no DEPT), 133.17 (Ar'C₄, broad singlet, no DEPT), 140.14 (Ar'C₁, no DEPT), 141.8 (Ar-C₃, dd, J_{CF} = 247.30 Hz, no DEPT), 147.7 (Ar-C₄, t, J_{CF} = 4.12 Hz, no DEPT), 148.9 (Ar-C₂, dd, J_{CF} = 248.80 Hz, no DEPT), 158.9 (Ar" C₄, no DEPT). ¹⁹F NMR (282 MHz, CDCl₃, TMS) δ: -142.14 (1F, dd, Ar-C₂/C₃-F, ³J_{FF} = 19.71 Hz, collapses to a doublet at -142.14 {³J_{FF} = 19.68 Hz} on decoupling),-159.15 (1F, dd, Ar-C₂/C₃-F, ³J_{FF} = 19.81 Hz, collapses to a doublet at -159.15 {³J_{FF} = 19.69 Hz} on decoupling). FT/IR (film/ATR/ZnSe) µₘₐₓ: 2954, 2928, 2857, 1653, 1558, 1533, 1506, 1471, 1458, 1289, 1248, 1105, 1075, 824, 799cm⁻¹. UV/Vis (0.5 mg, CHCl₃) λₘₐₓ: 291.50 (0.7201) nm. HPLC analysis (C₁₈ column; 1 ml min⁻¹ 95:5 acetonitrile to tetrahydrofuran) R_{T} = 23.07 (99.84%) min.

***2,3-Difluorophenylboronic acid (60)*** A similar procedure to that used for compound **25** was used. Quantities used: Butyllithium (10M, 9 ml, 90 mmol), 2,3-difluorobenzene (**59**) (10.07 g, 88.3 mmol), trimethylborate (18.34 g, 176.5 mmol) and tetrahydrofuran (145 ml). Dried *in vacuo* (P₂O₅, 0.3 mbar, RT, 16 h) to give a waxy solid. Yield = 10.94g (79%); FT/IR (KBr/Drift) µₘₐₓ: 3330, 1624, 1474, 1386, 1375, 1363, 1312, 1263, 1064, 927, 907, 826, 792, 740, 696, 677, 557, 552 cm⁻¹.

***4-(4'-Octyloxyphenyl)-2,3-difluorobenzene (61)*** A similar procedure to that used for compound **28** was employed. Quantities used: compound **60** (4.93 g, 31.3 mmol), 1-bromo-4-octyloxybenzene **(32)** (7.12 g, 25 mmol), palladium(0) *tetrakis*(triphenylphosphine) (0.87 g, 0.75 mmol), 2M sodium carbonate (75 ml) and 1,2-dimethoxyethane (105 ml). Purified by flash chromatography [fine mesh silica gel, hexanes then 10% dichloromethane in hexanes] to give a colourless oil, dried *in vacuo* (P₂O₅, 0.1 mbar, RT, 24 h) to give a waxy solid. Yield = 4.76g (59%); ¹H NMR (300 MHz, CDCl₃, TMS) δ: 0.89 (3H, t, ArOC₇CH₃), 1.32 (8H, m, ArOC₃CH₂CH₂CH₂CH₂CH₃), 1.49 (2H, m, ArOC₂CH₂C₆), 1.80 (2H, m, ArOCH₂CH₂C₆), 3.99 (2H, m, ArOCH₂C₇), 6.97 (2H, dd, Ar'-H₃/₅, ³J_{HH} = 6.71 Hz), 7.11 (3H, m, Ar-H₄/H₅/H₆), 7.46 (2H, dd, Ar'-H₂/₆, ³J_{HH} = 8.80 Hz). ¹³C NMR (75 MHz, CDCl₃, TMS) δ: 14.08 (ArOC₇CH₃, +ve DEPT), 22.65, 26.05, 29.24, 39.35, 31.81 (CH₂'s, -ve DEPT), 68.05 (ArOCH₂C₇, -ve DEPT), 114.55 (Ar'C₃/C₅, +ve DEPT), 115.34 (ArC₄, d, J_{CF} = 17.40 Hz, +ve DEPT), 123.89 (ArC₆, dd, J_{CF} = 7.26 Hz, +ve DEPT), 125.00 (ArC₅, t, J_{CF} = 2.68 Hz, +ve DEPT), 126.77 (Ar'C₁, d, J_{CF} = 2.93 Hz, no DEPT), 130.04 (Ar'C₂/C₆, indistinguishable doublet, J_{CF} = 2.95 Hz, +ve DEPT), 131.00 (ArC₁, d, J_{CF} = 10.23 Hz, no DEPT), 147.80 (ArC₂, dd, J_{CF} = 246.7 Hz, no DEPT), 151.24 (ArC₃, dd, J_{CF} = 245.4 Hz, no DEPT), 159.14 (Ar'C₄, no DEPT). FT/IR (KBr/Drift) µₘₐₓ: 2955, 2934, 2855, 1610, 1519, 1472, 1291, 1258, 1186, 1096, 895, 837, 810, 781, 732, 663, 577 cm⁻¹.

***4-(4'-octyloxyphenyl)-2-3-difluorophenylboronic acid (62)*** A similar procedure to that used for compound **25** was employed. Quantities used: Butyllithium (1.5 ml, 10M in hexanes, 15 mmol), compound **61** (4.14 g, 13.0 mmol), trimethylborate (2.70 g, 26 mmol) and tetrahydrofuran (160 ml). Dried *in vacuo* (P₂O₅, 0.5 mbar, RT, 24 h). Yield = 4.38g (93%); FT/IR (KBr/Drift) µₘₐₓ: 3344, 2935, 2853, 1521, 1452, 1407, 1394, 1388, 1362, 1257, 1043, 924, 824 cm⁻¹.

### EXAMPLE 11

***1,2-(S)-Dipentoxy-(2',3'-difluoro-4"-octyloxyterphenyl-4-oxy)butane (63)*** A similar procedure to that used for compound **28** was employed. Quantities used: compound **62** (1.08 g, 3.00 mmol), compound **47b** (0.90 g, 2.00 mmol), palladium(0) *tetrakis*(triphenylphosphine) (0.07 g, 0.06 mmol), 2M sodium carbonate (20 ml) and 1,2-dimethoxyethane (30 ml). Purified by flash chromatography [fine mesh silica gel, 1:1 hexanes to dichloromethane] to give a yellow solid. This was further purified by flash chromatography [fine mesh silica gel, 4 % ethyl acetate in hexanes]. A further purification was then carried out [fine mesh silica gel, 7.5% ethylacetate in hexanes]. Dried *in vacuo* (P₂O₅, 0.5 mbar, RT, 22 h). Yield = 0.74 g (58%); ¹H NMR (300 MHz, CDCl₃, TMS) δ: 0.88 (9H, m, ArOC₇CH₃ + OC₄CH₃'s), 1.31 (16H, m, ArOC₃CH₂CH₂CH₂CH₂CH₃ + OC₂CH₂CH₂CH₃'s), 1.46 (2H, m, ArOC₂CH₂C₅), 1.57 (4H, m, OCH₂CH₂C₃'s), 1.81 (2H, q, ArOCH₂CH₂C₆), 1.95 (1H, m, ArOCH₂CH₂*CH), 2.05 (1H, m, ArOCH₂CH₂*CH), 3.46 (4H + 1H, m, OCH₂C₄'s + *CHCH₂OC₅), 3.67 (1H + 1H, m, *CHCH₂OC₅ + ArOCH₂CH₂*CH), 4.00 (2H, t, ArOCH₂C₇), 4.15 (2H, m, ArOCH₂CH₂*CH), 6.98 (2H, dd, Ar"H₃/H₅ + Ar-H₃/H₅, ³J_{HH} = 4.15 Hz), 7.00 (2H, dd, Ar"H₃/H₅ + Ar-H₃/H₅, ³J_{HH} = 4.15 Hz), 7.20 (2H, dd, Ar'H₅/H₆, ³J_{HH} = 3.47 Hz), 7.51 (4H, d, Ar"H₂/H₆ + Ar-H₂/H₆, ³J_{HH} = 8.68 Hz). ¹³C NMR (75 MHz, CDCl₃, TMS) δ: 14.43, 14.46, 14.50 (ArOC₇CH₃ + OC₄CH₃'s, +ve DEPT), 22.91, 22.94, 23.07 (ArOC₆CH₂CH₃ + OC₃CH₂CH₃'s, -ve DEPT), 26.46, 28.75, 29.66, 29.77, 29.79, 30.25, 32.23, (CH₂'s, -ve DEPT), 32.43 (ArOCH₂CH₂*CH, -ve DEPT), 64.85 (ArOCH₂CH₂*CH, -ve DEPT), 68.48 (ArOCH₂C₇, -ve DEPT), 70.88, 72.07 (OCH₂C₄'s,-ve DEPT), 73.61 (*CHCH₂OC₅, -ve DEPT), 75.63 (ArOCH₂CH₂*CH, +ve DEPT), 115.01 (Ar"C₃/C₅ + Ar-C₃/C₅, +ve DEPT), 124.69 (Ar'C₅/₆, t, J_{CF} = 3.63 Hz, +ve DEPT), 127.28 (Ar"C₁ + Ar-C₁, d, J_{CF} = 7.06 Hz, no DEPT), 129.30 (Ar'C₁/₄, t, J_{CF} = 3.63 Hz, no DEPT), 130.35 (Ar"C₂/C₆ + Ar-C₂/C₆, +ve DEPT), 148.84 (Ar'C₂/₃, dd, J_{CF} = 250.0 Hz, no DEPT), 159.40, 159.53 (Ar"C₄ + Ar-C₄, indistinguishable doublet, no DEPT). ¹⁹F NMR (282 MHz, CDCl₃, TMS) δ: -143.998 (2F, d, Ar'C₂-F + Ar'C₃-F, ⁵J_{HF} = 3.36 Hz, collapses to a singlet at-143.998 on decoupling). FT/IR (film/ATR/ZnSe) µₘₐₓ: 2954, 2927, 2856, 1610, 1527, 1533, 1486, 1475, 1459, 1290, 1250, 1180, 1102, 1044, 905, 841, 829, 526 cm⁻¹. UV/Vis (0.5 mg, CHCl₃) λₘₐₓ: 290.0 (0.6467) nm. HPLC analysis (C₁₈ column; 1 ml min⁻¹ 95:5 acetonitrile to tetrahydrofuran) R_{T} = 26.10 (100%) min.

The present invention will now be described in further detail and with reference to the accompanying drawings in which:-
Figure 1 is a cooling DSC scan for compound 13a,
Figure 2 is a pitch length - temperature dependence graph for compounds 13a, 41, 42, 43 and 45,
Figure 3 is a pitch length - temperature dependence graph for compounds 53, 58 and 63, and
Figure 4 is a schematic section through an electro-optical liquid crystal device according to the present invention

### Liquid Crystal Behaviour

### A. 1,(S)-2-(4"-Octyloxyterphenyl-4-oxy)dialkoxybutanes (Examples 1 to 3)

All three of compounds **12, 13a,** and **14** showed complicated and unique liquid-crystalline behaviour and were characterised by optical microscopy and differential scanning calorimetry, the results are listed in table 1 below. Although all the materials show enantiotropic liquid-crystalline phase behaviour, the phase behaviour of the three Examples is best described on cooling from the isotropic liquid:-

**Example 1:** Cooling compound **12** from the isotropic liquid results in the formation of shimmering grey focal-conic fans which rapidly give way to homeotropic black texture *via* a highly mobile filaments (vermis-like), the appearance of which suggests a transition sequence of I-TGB A*-S_{A}*. On continued cooling of the homeotropic texture to 176.5 °C, highly mobile, grey circular domains appear which quickly coalesce to form a conventional *schlieren* S_{C}* texture containing 4-brush singularities. The sample crystallises at 161.1 °C. DSC analysis of this material confirms the presence of these events which are clearly resolved during a cooling scan rate of 1 °C min⁻¹. This material is believed to show a I-TGB A*-S_{A}-TGB C*-S_{C}*-K phase sequence, which has been observed previously in a number of other unrelated chiral materials (A. Bouchta et. al., **Liquid Crystals,** 1992, **12(4),** 575; H.T. Nguyen et al. **J. Phys. II France,** 1992, **2**, 1889; L. Navailles et. al., **Liquid Crystals,** 1993, **15(4)**, 479; N. Isaert et. al., **J. Phys. II France,** 1994, 4, 1501; and A. Bouchta at al., **J. Mater. Chem.,** 1995, **5(2)**, 2079). The presence of the two TGB phases is confirmation of the highly twisting nature of this material.

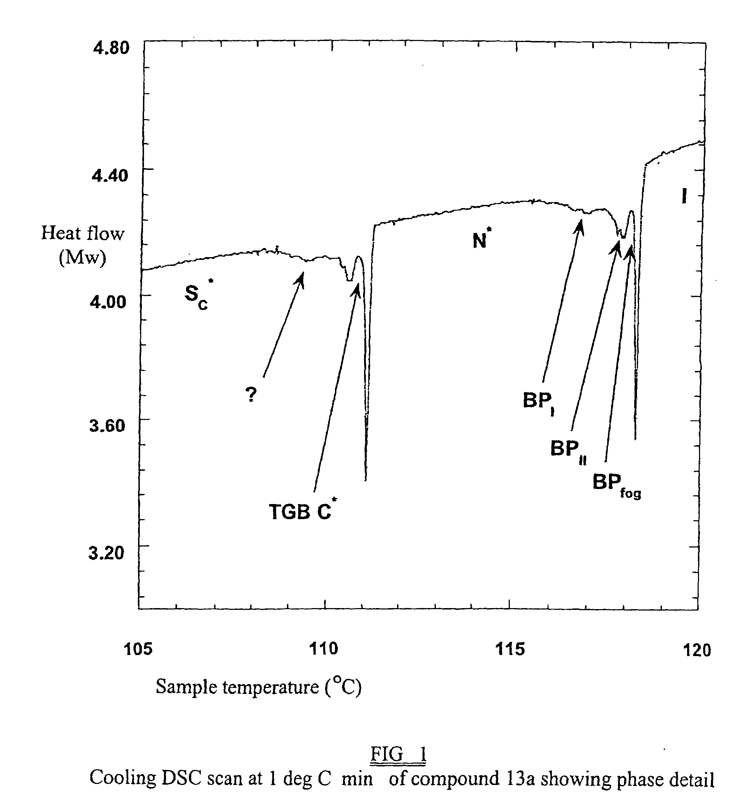

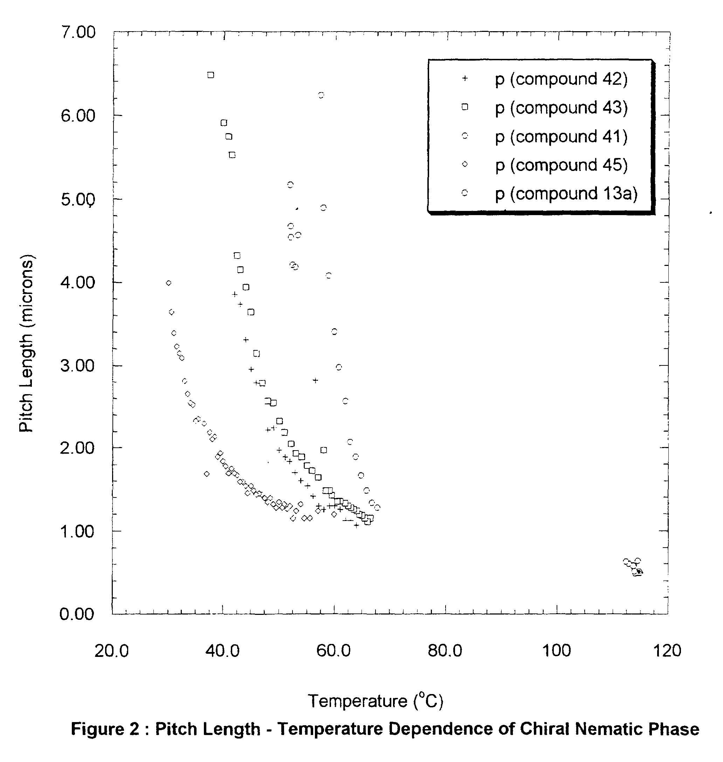

**Example 2:** Cooling compound **13a** from the isotropic liquid gives way to the formation of blue-green platelets at 118.4 °C which rapidly give way to a *pseudo*-focal conic texture. If the cover slip is mechanically disturbed the material briefly adopts a planar *Grandjean* type texture before reverting to the undisturbed form, clearly confirming this as a nematic phase. Further cooling to around 111.2 °C results in the formation of a highly coloured planar texture of a smectic C* phase. If the hot stage is held just on the transition then fine filaments (or vermis) are seen penetrating the S_{C}* texture from the undisturbed N* state. This is believed to be a TGB C* phase and exists only for a range of approximately 0.4 °C. Further cooling of the S_{C}* phase results in the formation of a higher order smectic phase (denoted S_{X}*) before crystallisation occurs. Surprisingly, all of these phase transitions are observed during DSC analysis (Figure 1); at a cooling scan rate of 1 °C min⁻¹, the BP "event" is resolved in three transitions, presumably Iso-BP_{fog}-BP_{II}-BP₁. Furthermore, the N*-TGB C*-S_{C}* is also clearly resolved (this also shows an unidentified change in heat capacity (labelled as ? in Figure 2) at *ca* 109 °C . The S_{C}*-S_{X}* transition is a weakly first order, its enthalpy change is *ca.,* 0.21 kJ mol⁻¹.

Compound **13a**'s racemate, compound **13b,** as might be expected gave a far simpler phase sequence. Cooling from the isotropic liquid results in the formation of N droplets, which coalesce to give areas of *schlieren* texture (containing 2- and 4-brush singularities) interspersed with homeotropically aligned material. The homeotropic regions seem to predominate until at 108.9 °C they give way to a bright S_{C} *schlieren* texture containing 4-brush singularities only. Further cooling to this achiral S_{C} phase results in the formation of an unidentified higher order smectic phase (S_{X}*). This transition is somewhat difficult to observe (the texture appears to become feathery) and is best observed by DSC. Further cooling of compound **13b** results only in crystallisation.

**Example 3:** Cooling compound **14** slowly (0.5 °C min⁻¹) from the isotropic liquid causes the formation of droplets which develop a grey *Grandjean* texture with highly mobile pitch lines (rotation of the microscope's upper polariser gives a red dispersion in an anticlockwise rotation *i.e.*, right handed twist sense). Further cooling of this texture results in a blue planar texture (with slowly moving domains) which slowly sweeps across the field of view. This is believed to be a S_{C}* phase (with identical twist sense to the N* phase). Further cooling results only in recrystallisation of the sample at around 140.6 °C. These events are confirmed by DSC studies, particularly at low cooling scan rates (0.5 °C min⁻¹) when the I-N*-S_{C}* appears slightly better resolved.

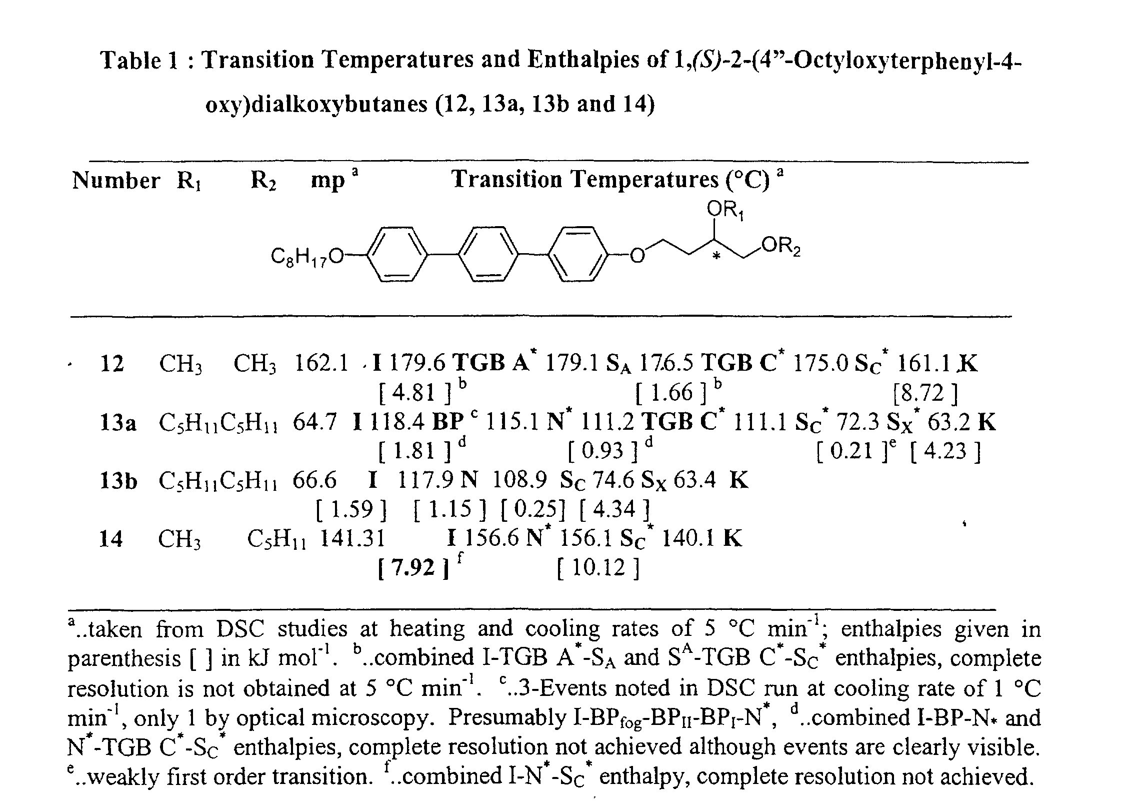

### B. The Mono-Fluoro-Substituted 1,(S)-2-(4"-octyloxyterphenyl-4-oxy)dialkoxy-butanes (Examples 4 to 8)

All five materials synthesised showed enantiotropic liquid crystalline behaviour, showing N* or S_{C}* phases with relatively low melting and clearing temperatures, which are listed in table 2 below. However, the optical microscopy of the individual compounds revealed a number of unexpected facts about the phase behaviour that was not at all apparent from the DSC studies.

**Example 4:** Compound **40** has a moderately low melting point (51 °C). Cooling from the isotropic liquid resulted in the formation of a S_{A} phase characterised by the presence of regions of broken focal-conic fan and darker homeotropic regions, the focal-conic regions gradually becoming homeotropic as the temperature dropped. At around 84 °C, this mixed texture gave way to a greyish *schlieren* texture which contained small regions of focal-conics. Analysis of the *schlieren* domains revealed the presence of 4-brush singularities, meaning that this phase could be assigned as S_{C}* (rotation of the microscope's upper polariser resulted in an anticlockwise colour dispersion). At around 46 °C, the focal-conic regions became 'feathery' in appearance. DSC confirmed this as another phase transition and is probably a higher order phase S_{X}*.

**Example 5:** Compound **41** proved remarkably'different to compound **40.** Here the influence of the 2-fluoro-substituent in the inter annular position drastically reduces the melting point to ca. 28 °C and the clearing point to 73 °C, corresponding to depressions of 36 and 45 °C respectively over its parent compound **13a.** Cooling compound **41** from its isotropic liquid resulted in a N* phase with of a blue *Grandjean* texture (rotation of the microscope's upper polariser gives an anticlockwise colour dispersion). Continued cooling causes the texture to become highly mobile, rippled and then *schlieren-*like. At a temperature of 58.3 °C, the texture appears to be completely *schlieren* (Brownian motion and shimmering in the texture is clearly visible). If cooling is continued, this *schlieren* texture appears to reform into a *Grandjean* texture at approximately 56.6 °C. This phenomenon is a twist inversion: the chiral nematic phase is experiencing a N*-N*_{∞}-N* sequence in which the pitch unwinds to infinite pitch and then rewinds. The handedness of the helix is opposite above and below the unwound N*_{∞} state as shown by rotation of the microscope's upper polariser; it is anticlockwise above and clockwise below. If cooling is continued the N* phase undergoes a transition to a S_{C}* phase at 48.4 °C. DSC analysis showed this phase transition to be 1^{st} order in nature (ΔH = 1.13 kJ mol⁻¹). This material eventually crystallises at 4 °C, indicating potential use in formulating room temperature mixtures.

**Example 6:** Compound **42** shows some similarities in both structure and behaviour to compound **41.** Like compound **41,** the 2"-fluoro-substituent results in dramatic depression of the melting point and clearing point when compared to the parent compound **13a.** Compound **42** forms a blue *Grandjean* textured N* phase on cooling from the isotropic liquid, further cooling results in the steady unwinding of the phases helix until a shimmering, *schlieren* texture is observed at 35.1 °C (the N*_{∞}). This never actually rewinds because the phase undergoes a transition to an S_{C}* phase at 30.5 °C, before crystallising at 14.4 °C.

**Example 7:** Compound **43,** has similar properties to both **41** and **42,** having a similar low melting point of 34 °C. It forms a N* phase on cooling from the isotropic liquid at 70.9 °C. Just like compound **41,** the blue *Grandjean* texture gradually gives way to an unwound N*_{∞} state at *ca.*, 36.3-34.7 °C before the *Grandjean* texture reforms at 30.7 °C. Once again the handedness of the helix above and below the twist inversion was checked by rotating the microscope's upper polariser and observing the colour dispersion; anticlockwise above the N*_{∞} state and clockwise below the N*_{∞} state. The N^{*} eventually gives way to a S_{C}* phase before recrystallising at 14.8 °C.

**Example 8:** Compound **45,** with its interannular fluorosubstituent might be expected to give both low thermal stability and melting point and this is indeed the case. This material has a melting point (as determined by DSC) of 2.6 °C some 62 °C lower than its non fluoro-substituted parent compound **13a.** Cooling compound **45** from the isotropic liquid results in the formation of a *Grandjean* texture characteristic of a N* phase. As the temperature is cooled to around 26.2 °C, the texture shows every indication of unwinding and the texture becomes *schlieren-like* in nature. Unlike compounds **41** and **43** no inversion is observed because at 25.6 °C the texture undergoes a transition to a planar S_{C}* phase. Crystallisation was not observed by microscopy or by DSC, but does occur after standing at -30 °C for 5 mins.

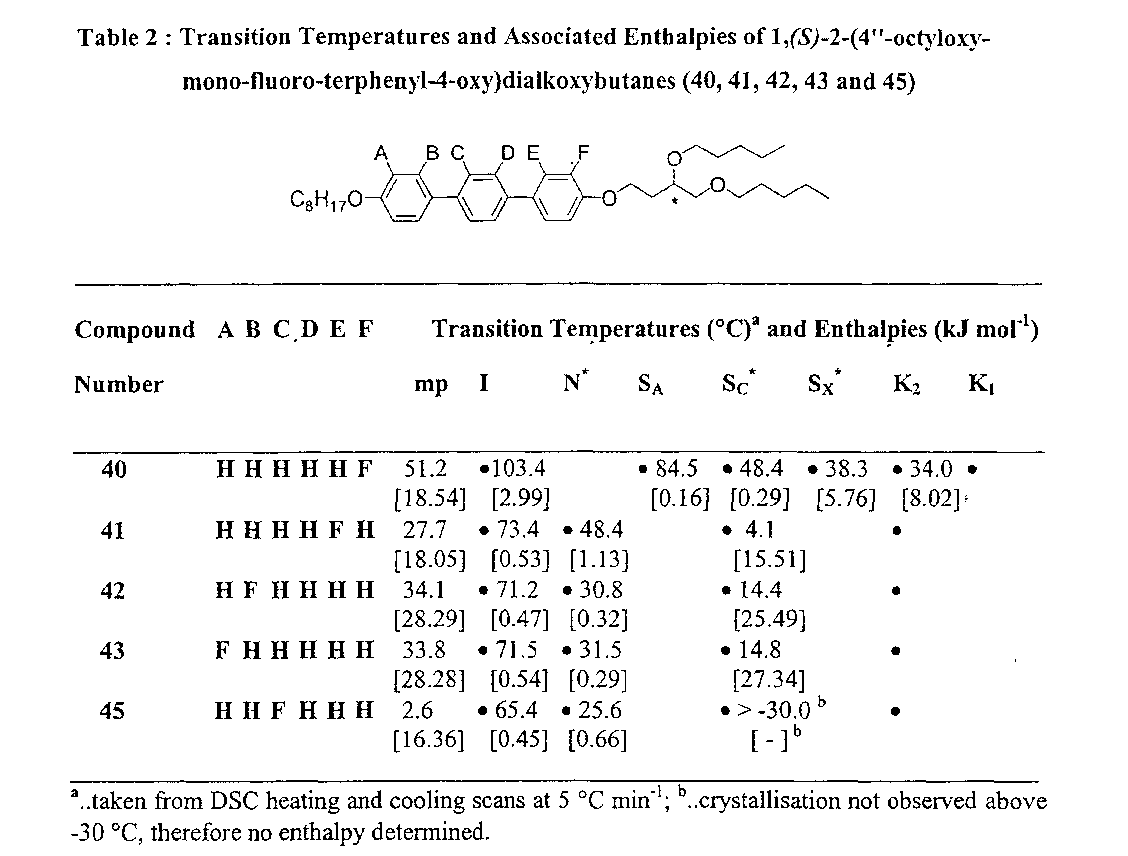

### C. The di-fluoro-substituted 1,(S)-2-dipentoxy-(4"-octyloxyterphenyl-4-oxy)-butanes (Examples 9 to 11)

All three of these synthesised compounds showed enantiotropic liquid-crystalline behaviour, displaying both N* and S_{C}* phases. More useful was the fact that the melting and clearing points had been drastically reduced from these of their parent non-fluorinated compound. The transitions and related enthalpies are shown in table 3 below.

Compound **53** (Example 9) was seen to display, at 15.9 °C by far the lowest melting point of these three difluoro-substituted compounds. Cooling the compound from an isotropic liquid resulted in the formation of an N* at 69.0 °C. This phase showed a typical *Grandjean* texture which gave an anticlockwise dispersion of colour on rotation of the microscopes upper polariser. Continued cooling results in the formation of a S_{C}* phase at 49.1 °C, eventually crystallising at-2.0 °C.

Compound **63** (Example 11) had a moderately low melting point of 50.7 °C and cooling from an isotropic liquid resulted in the formation of a N* phase at 62.7 °C (rotation of the microscopes upper polariser gave an anticlockwise colour dispersion). This texture became a S_{C}* at 21.0 °C before crystallising at -14.9 °C.

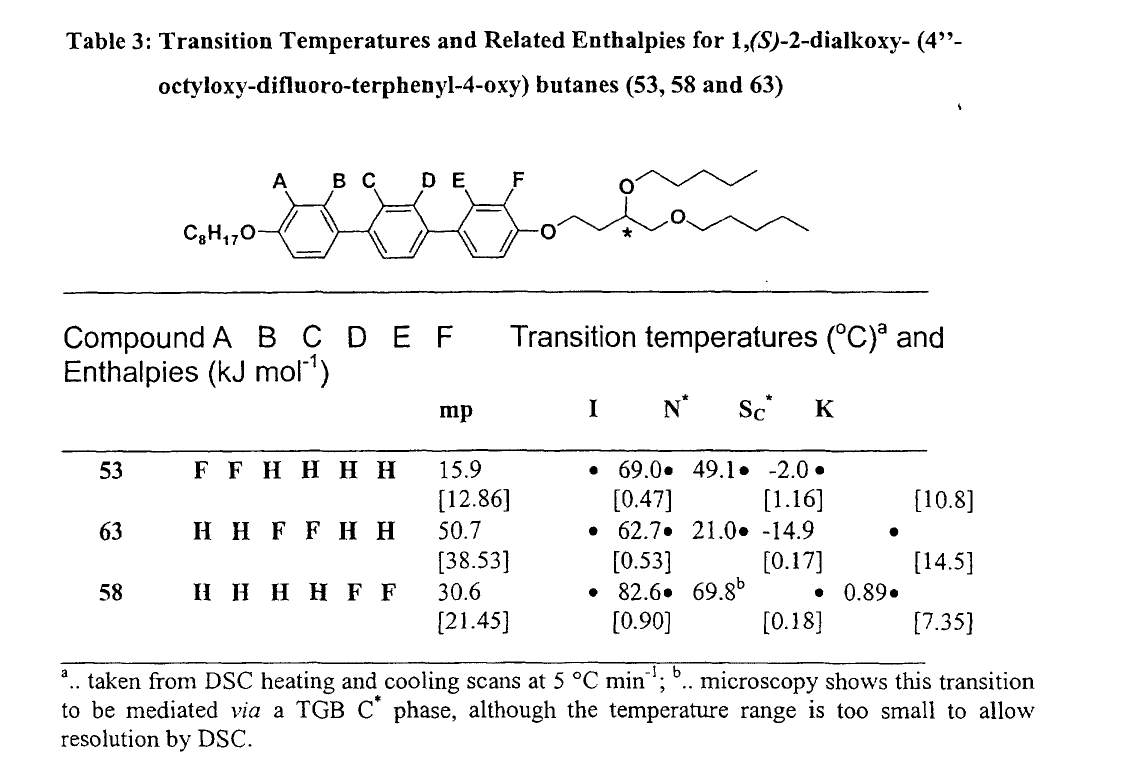

Of these 3 compounds, the derivative **58** proved to be the most interesting because of its enormous S_{C}* range (approximately 68 °C). With a melting point of 30.6 °C, this compound, on cooling formed a N* phase at 82.6 °C. This phase showed a mobile *Grandjean* texture which, on rotation of the microscopes upper polariser had an anticlockwise dispersion of colour. This phase appeared to briefly form a TGB C* phase before becoming S_{C}* (difficult to observe on cooling). This manifests itself in the formation of TGB-like filaments just prior to fully forming the smectic C* phase at the N*-S_{C}* transition. Unfortunately DSC analysis of this transition at a variety of scan rates (10, 5, 2 and 1 °C min⁻¹) failed to resolve any event other than the N*-S_{C}* transition. At below 69.8 °C the texture became *schlieren* in nature and closer analysis revealed 4-brush singularities meaning that the phase could be unambiguously assigned as S_{C}*. Continued cooling resulted only in the crystallisation of the material at 0.89 °C.

### Helical Pitch Measurements of Chiral Nematic Phases

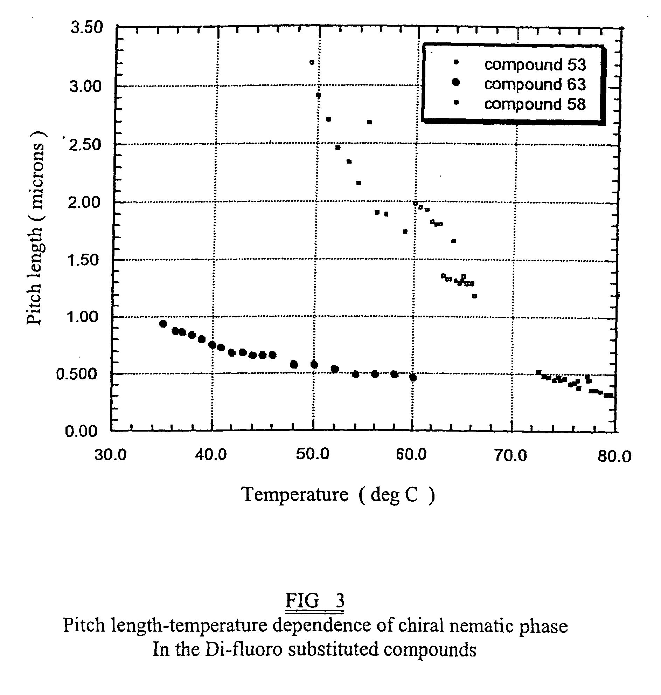

These were performed using the Cano wedge method (M.F. Grandjean, **C.R. Acad. Sci.,** 1921, 172, 71; and R.R. Cano, **Bull. Soc. Miner. Cryst.,** 1968, 91, 20) using prefabricated EHC wedge cells (average wedge angle of 0.47 degrees), the distance between the disclination lines being measured using a calibrated graticule and eyepiece. The results are plotted in Figures 2 and 3 as a function of temperature.

Referring to Figure 2, all the monofluoro-substituted materials (compounds **41, 42, 43,** and **45**) show pitch lengths which vary between 1.06 to 6.48 microns (the plotted curves do not necessarily show the pitch over the full chiral nematic range, as the disclination lines could not always be observed). Of the two twist inverting materials **41** and **43,** only compound **41** shows the inversion phenomenon clearly. Here the pitch tends to infinity at *ca* 58 °C before dropping to pitch length of between **4** and **5** microns before entering the underlying S_{C}* phase. Materials **42, 43** and **45** all show similarly shaped curves which tend to infinity (an unwound state) before reaching their underlying S_{C}* phases. Interestingly, the parent compound **13a,** shows a much shorter pitch, *ca* 0.49 - 0.62 microns which probably explains why the material shows both Blue Phases and the TGB C* phases only observed in highly chiral compounds.

Referring to Figure 3, the three synthesised difluoro compounds (**53**, **58** and **63**) showed a pitch length ranging from 0.3 to 3.3 microns. However the plotted curves may not show the pitch over the complete N* range as disclination lines could not always be easily observed. All three compounds show an increase in pitch as the temperature decreases before transforming into the underlying S_{C}* phase. Compound **58** showed a relatively small pitch length, a factor that is often associated with highly chiral compounds; which suggests that the 'suspected' TGB C* phase noted earlier might be a genuine observation. It is also noticeable that compound **53** shows a much higher pitch length than either **58** or **63.**

### Optical Purity

Although twist inversion phenomena in liquid crystals are now relatively well known, examples of materials which show this behaviour with one chiral centre are rare (A.J. Slaney et. al., **J. Mater. Chem.,** 1992, **2,** 805 and C. Loubser et. al., **J. Mater. Chem.,** 1994, **4,** 71). As the materials **41** and **43** fall into both these categories, it became imperative that their optical purity be determined or in some way estimated so that the possibility of an opposite enantiomer present as an undetectable 'impurity' causing a compensation of helical twist can be discounted. The starting butanetriol (purchased from Fluka) is claimed to have an *ee* of >96%, so in order to determine this, and to discount racemisation of the chiral centre during the synthetic route (schemes 2 and 3) a series of Mosher's esters were prepared from various intermediates **(2a/b, 3a/b** and **6a)** using *(R)*-(-)-α-methoxy-(trifluoromethyl)phenylacetylchloride (MTPACl), as outlined in table 4 below. The resulting diastereoisomeric Mosher's esters were then analysed by ¹⁹F-{¹H} NMR (J.A. Dale et. al., **J. Org. Chem.,** 1969, 34, 2543).

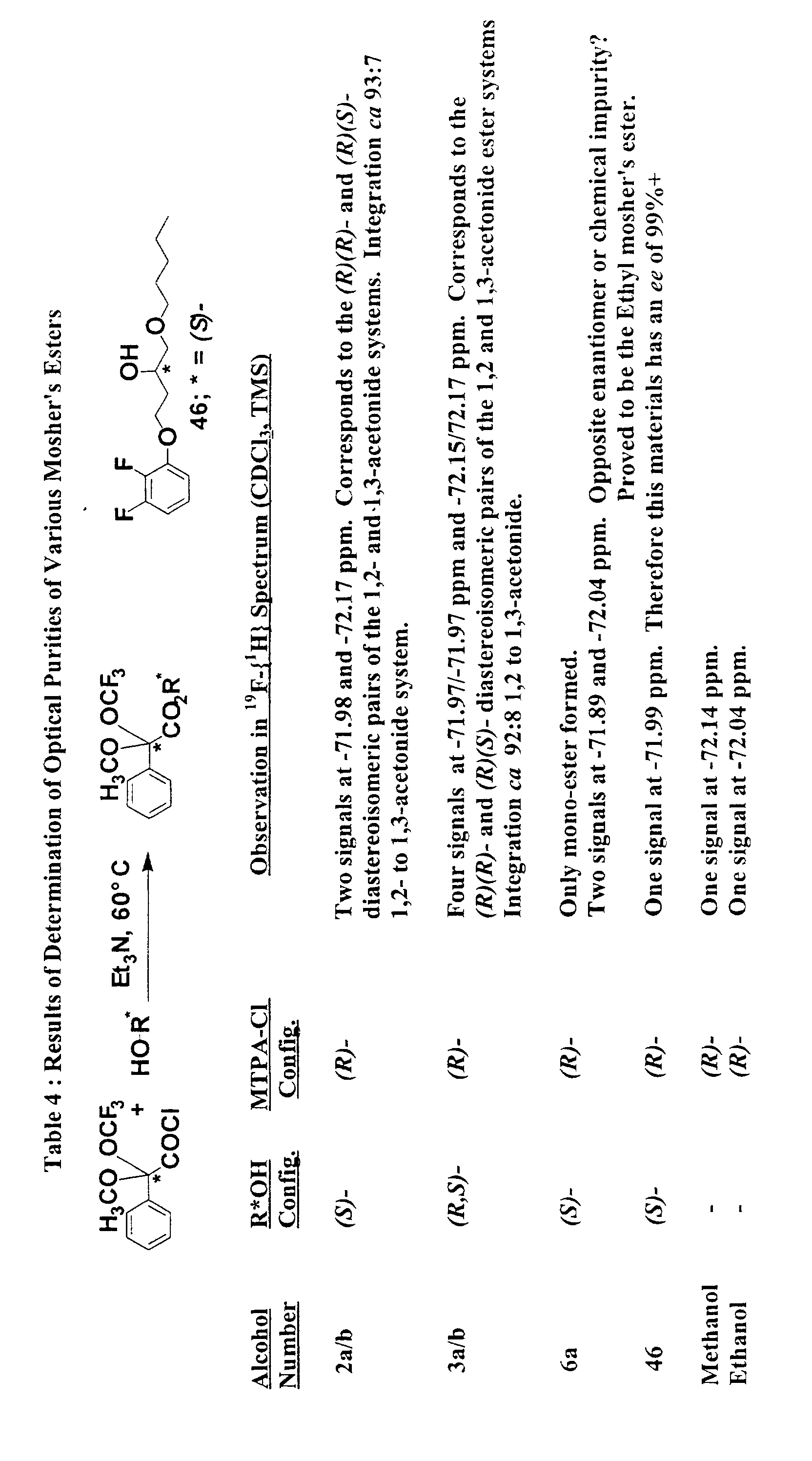

The method was first tested using the racemic acetonide **3a/b,** here four signals were observed corresponding to the *(R)(R)-* and *(R)(S)*-diastereoisomeric pairs of both the 1,2-acetonide (5-membered ring) and 1,3-acetonide (6-membered ring) at -71.97, -71.97 ppm and -72.15, -72.17 ppm. Integration of the signals revealed a ratio of 92:8 1,2- to 1,3- which corresponds well with the result determined by ¹H NMR. This was repeated for the optically active variant **2a/b** and two signals at -71.98 and -72.17 ppm observed, again corresponding to a 93:7 ratio of *(R)(S)*-1,2-acetonide and *(R)(S)*-1,3-acetonide. The lack of any other signals indicates that no racemization takes place durirrg the formation of the acetonide, which is in accordance with the published literature.

However, it was a concern that racemization might occur during the deprotection step using p-toluenesulfonic acid in methanol, so the Mosher's ester of compound **6a** was prepared. Here the position was clouded by the observation of two peaks at -71.89 and -72.04 ppm. The former peak was undoubtedly the *(R)(S)*-diastereoisomer but the second peak could be the *(R)(R)*-diastereoisomer indicating a lower optical purity of **6a** or an impurity peak such as the Mosher's acid anhydride (unlikely) or a reaction product of MTPACl and methanol or ethanol impurity in the triethylamine. To test this, the methyl and ethyl esters of MTPACl were prepared; the ¹⁹F-{¹H} NMR spectra of these show peaks at -72.14 and -72.04 ppm respectively. A sample of the ethyl Mosher's ester was 'spiked' into an NMR sample of the Mosher's ester of **6a.** The ¹⁹H-{¹H} NMR spectrum showed the 'impurity' peak to increase in height over the *(R)(S)*-diastereoisomer peak at -72.04 ppm. This conclusively proves that the 'impurity' in the **6a** Mosher's ester is the ethyl Mosher's ester, meaning that compound **6a** is essentially optically pure and that no racemization occurs during the deprotection reaction using p-toluenesulfonic acid in methanol.

This result was finally checked by preparing the Mosher's ester 2,3-difluoro-1-(1-pentoxy-*(S)*-2-hydroxybutoxy)benzene (synthesised from 2,3-difluorophenol in three steps using similar methods to those used in the preparation of compound **5a** in scheme 2), the extraction being performed with freshly distilled and dried triethylamine. The ¹⁹F-{¹H} NMR spectra showed one peak at -72.04 ppm corresponding to the *(R)(S)*-diastereoisomer only, indicating that the product is essentially optically pure (*ee* >99%). From this it can reasonably be assumed that the final targets are all enantiopure (possibly enriched due to the further purification in later synthetic steps) and that the twist inversion phenomenon that occurs in compound **41** and **43** is not due to a compensation due to the opposite enantiomer.

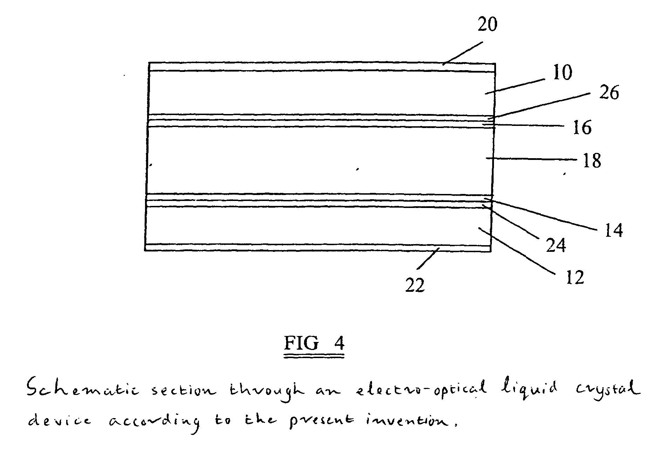

Referring now to Figure **4,** the device illustrated therein comprises first and second spaced glass substrates 10 and 12 between which is defined a liquid crystal cell. The mutually facing surfaces of the substrates 10 and 12 are provided with respective rubbed alignment layers 14 and 16 between and in contact with which is disposed a layer 18 of a liquid crystal composition including compound 13a.

Linear polarisers 20 and 22 are provided on the outer surfaces of the substrate 10 and the substrate 12, respectively. The polariser 20 has its polarisation axis aligned with the rubbing directions of the alignment layers 14 and 16 which are mutually parallel, whilst the linear polariser 22 has its polarisation axis perpendicular to that of the polariser 20 and the rubbing directions of the alignment layers 14 and 16. The liquid crystal molecules in the chiral smectic phase within the liquid crystal composition in the layer 18 are sensitive to changes in electrical field applied across the layer 18 via the electrodes 24 and 26. Thus, in a manner known per se, the layer 18 can be switched such that light transmission through the device is enabled at one voltage and prevented at another voltage so that the device can act as a fast-operating optical shutter.

The following are given as some representative examples of possible industrial applications for the compounds of the present invention:-
1. Compensating dopants which utilise the twist inversion phenomenon.
2. Components in ferroelectric mixtures, since the Examples have room temperature S_{c}* phase ranges.
3. S_{A}-suppressing dopants for ferroelectric mixtures.
4. Twist compensating dopants for use in ferroelectric mixtures. Unwinding a preceding nematic phase can improve the subsequent alignment of the underlying S_{c}* phase.
High twist dopants (low melting point).

## Claims

1. A terphenyl compound having the general formula [1]:- 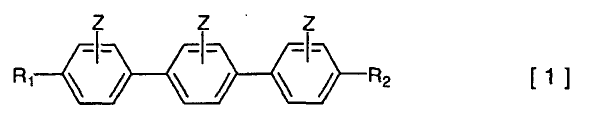 where each of R₁ and R₂ is the same or different and is selected from the group consisting of:-alkyl, alkyloxy, alkenyl, alkenyloxy, alkynyl, alkynyloxy, alkylthio, alkenylthio and alkynylthio 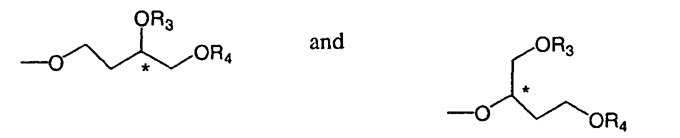 where * indicates an asymmetric carbon atom,
provided that at least one of R₁ and R₂ is 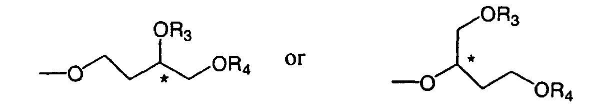 and where each of R₃ and R₄ is the same or different and is an alkyl, alkenyl or alkynyl group; Z is independently selected from hydrogen and fluorine at each terphenyl carbon and R₁, R₂, R₃,
and R₄ contain no more than 16 carbon atoms.

2. A compound as claimed in claim 1, wherein the group represented by R₁ or R₂ is a C₁- to C₁₆ n-alkyl group.

3. A compound as claimed in claim 2, wherein the group represented by R₁ or R₂ is a C₇- to C₁₂ n-alkyl group.

4. A compound as claimed in any preceding claim, wherein the group represented by R₃ or R₄ is a C₁- to C₁₆ n-alkyl group.

5. A compound as claimed in claim 4, wherein the group represented by R₃ or R₄ is a C₁- to C₅ n-alkyl group.

6. A compound as claimed in any preceding claim having the general formula [2] or the general formula [3]:- 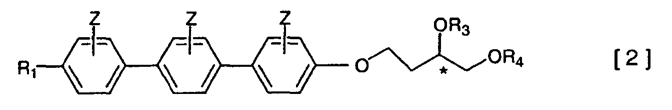 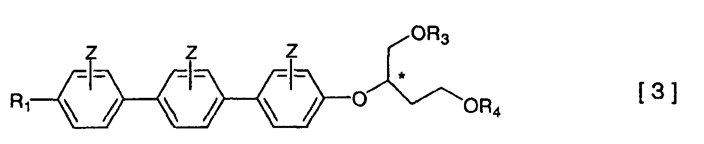 wherein R₁, R₃, R₄ and Z are as defined above.

7. A compound as claimed in claim 6 having the general formula [4] or the general formula [5]:- 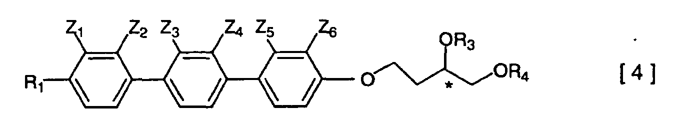 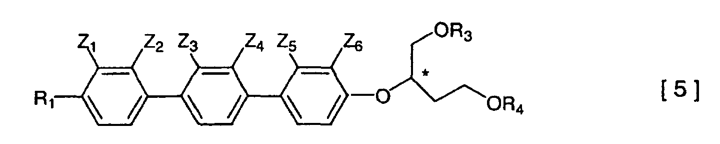 wherein R₁, R₃ and R₄ are as defined above, and either
(i) one of Z₁ to Z₆ is F, the remaining Z, to Z₆ being H, or
(ii) Z, and Z₂, or Z₃ and Z₄, or Z₅ and Z₆ are F, the remaining Z₁ to Z₆ being H.

8. A compound as claimed in any preceding claim, wherein R₁ is n-octyl.

9. A compound as claimed in any preceding claim wherein R₃ and R₄ are independently selected from methyl and n-pentyl.

10. A compound selected from the following group:-
1,2-(S)-dimethoxy-(4"-octyloxyterphenyl-4-oxy)butane,
1,2-(S)-dipentoxy(4"-octyloxyterphenyl-4-oxy)butane,
1-pentoxy-2-(S)-methoxy-(4"-octyloxyterphenyl-4-oxy)butane,
1,2-(S)-dipentoxy-(4"-octyloxyterphenyl-3-fluoro-4-oxy)butane,
1,2-(S)-dipentoxy-(4"-octyloxyterphenyl-2-fluoro-4-oxy)butane,
1,2-(S)-dipentoxy-(2"-fluoro-4"-octyloxyterphenyl-4-oxy)butane,
1,2-(S)-dipentoxy-(3"-fluoro-4"-octyloxyterphenyl-4-oxy)butane,
1,2-(S)-dipentoxy-(2'-fluoro-4"-octyloxyterphenyl-4-oxy)butane,
1,2-(S)-dipentoxy-(2",3"-difluoro-4"-octyloxyterphenyl-4-oxy)butane,
1,2-(S)-dipentoxy-(4"-octyloxyterphenyl-2,3-difluoro-4-oxy)butane, and
1,2-(S)-dipentoxy-(2',3'-difluoro-4"-octyloxyterphenyl-4-oxy)butane.

11. A liquid crystal composition containing at least one compound according to any one of claims 1 to 10.

12. An electro-optical liquid crystal device including a liquid crystal cell having a layer of a liquid crystal composition according to claim 11, and means for applying an electrical field across said layer.

## Patentansprüche

1. Terphenylverbindung mit der allgemeinen Formel [1]: 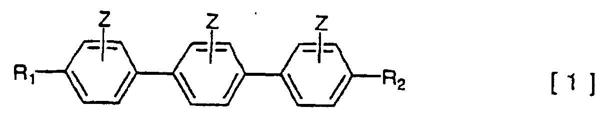 wobei jedes R₁ und R₂ gleich oder verschieden ist und ausgewählt ist aus der Gruppe, bestehend aus Alkyl, Alkyloxy, Alkenyl, Alkenyloxy, Alkinyl, Alkinyloxy, Alkylthio, Alkenylthio und Alkinylthio, 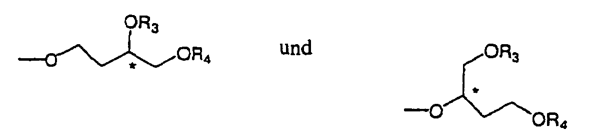 wobei * ein asymmetrisches Kohlenstoffatom bezeichnet,
vorausgesetzt dass wenigstens eines von R₁ und R₂ 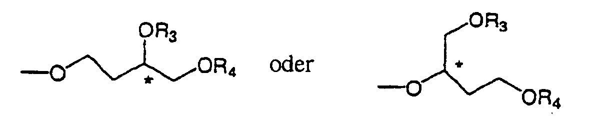 ist und wobei jedes R₃ und R₄ gleich oder verschieden ist und eine Alkyl-, Alkenyl- oder Alkinylgruppe darstellt; Z unabhängig ausgewählt ist aus Wasserstoff und Fluor für jeden Terphenylkohlenstoff und R₁, R₂, R₃ und R₄ nicht mehr als 16 Kohlenstoffatome enthalten.

2. Verbindung nach Anspruch 1, worin die durch R₁ oder R₂ dargestellte Gruppe eine C₁bis C₁₆-n-Alkylgruppe ist.

3. Verbindung nach Anspruch 2, worin die durch R₁ oder R₂ dargestellte Gruppe eine C₇bis C₁₂-n-Alkylgruppe ist.

4. Verbindung nach irgendeinem der vorhergehenden Ansprüche, worin die durch R₃ oder R₄ dargestellte Gruppe eine C₁- bis C₁₆-n-Alkylgruppe ist.

5. Verbindung nach Anspruch 4, worin die durch R₃ oder R₄ dargestellte Gruppe eine C₁bis C₅-n-Alkylgruppe ist.

6. Verbindung nach irgendeinem der vorhergehenden Ansprüche mit der allgemeinen Formel [2] oder der allgemeinen Formel [3]: 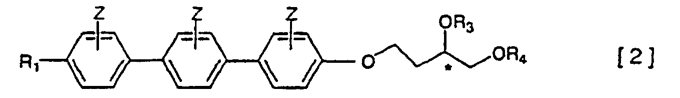 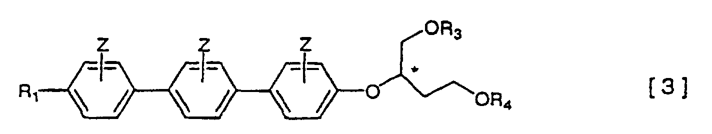 worin R₁, R₃, R₄ und Z wie oben definiert sind.

7. Verbindung nach Anspruch 6 mit der allgemeinen Formel [4] oder der allgemeinen Formel [5]: 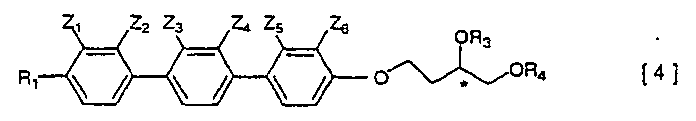 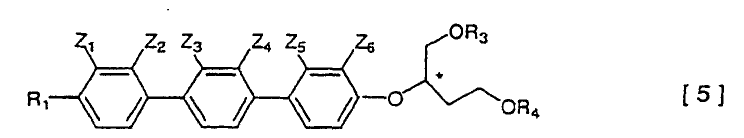 worin R₁, R₃ und R₄ wie oben definiert sind, und entweder
(i) eines von Z₁ bis Z₆ F ist, wobei die verbleibenden Z₁ bis Z₆ H sind, oder
(ii) Z₁ und Z₂, oder Z₃ und Z₄, oder Z₅ und Z₆ F sind, wobei die verbleibenden Z₁ bis Z₆ H sind.

8. Verbindung nach irgendeinem der vorhergehenden Ansprüche, worin R₁ n-Octyl ist.

9. Verbindung nach irgendeinem der vorhergehenden Ansprüche, worin R₃ und R₄ unabhängig voneinander ausgewählt sind aus Methyl und n-Pentyl.

10. Verbindung ausgewählt aus der folgenden Gruppe:
1,2-(S)-Dimethoxy-(4"-octoyloxyterphenyl-4-oxy)butan,
1,2-(S)-Dipentoxy-(4"-octoyloxyterphenyl-4-oxy)butan,
1-Pentoxy-2-(S)-methoxy-(4"-octoyloxyterphenyl-4-oxy)butan,
1,2-(S)-Dipentoxy-(4"-octoyloxyterphenyl-3-fluor-4-oxy)butan,
1,2-(S)-Dipentoxy-(4"-octoyloxyterphenyl-2-fluor-4-oxy)butan,
1,2-(S)-Dipentoxy-(2"-fluor-4"-octoyloxyterphenyl-4-oxy)butan,
1,2-(S)-Dipentoxy-(3"-fluor-4"-octoyloxyterphenyl-4-oxy)butan,
1,2-(S)-Dipentoxy-(2'-fluor-4"-octoyloxyterphenyl-4-oxy)butan,
1,2-(S)-Dipentoxy-(2",3"-difluor-4"-octoyloxyterphenyl-4-oxy)butan,
1,2-(S)-Dipentoxy-(4"-octoyloxyterphenyl-2,3-difluor-4-oxy)butan,
1,2-(S)-Dipentoxy-(2',3'-difluor-4"-octoyloxyterphenyl-4-oxy)butan.

11. Flüssigkristallzusammensetzung, enthaltend mindestens eine der Verbindungen nach irgendeinem der Ansprüche 1 bis 10.

12. Elektro-optische Flüssigkristallvorrichtung, umfassend eine Flüssigkristallzelle mit einer Schicht einer Flüssigkristallzusammensetzung nach Anspruch 11, und Mitteln zur Anwendung eines elektrischen Feldes über der Schicht.

## Revendications

1. Composé terphényle ayant la formule générale [1] : 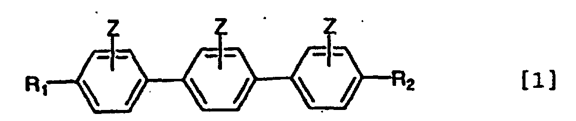 dans laquelle chacun de R₁ et R₂ est identique ou différent et est choisi dans le groupe constitué par : alkyle, alkyloxy, alcényle, alcényloxy, alcynyle, alcynyloxy, alkylthio, alcénylthio et alcynylthio 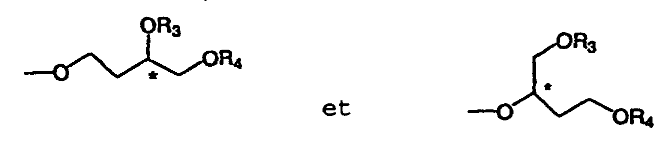 où * indique un atome de carbone asymétrique,
sous réserve qu'au moins l'un de R₁ et R₂ est 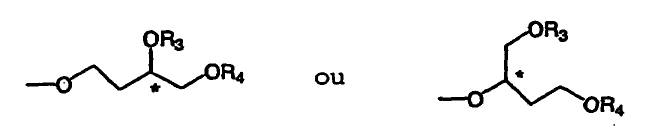 et où chacun de R₃ et R₄ est identique ou différent et est un groupe alkyle, alcényle ou alcynyle ; Z est indépendamment choisi parmi hydrogène et fluor sur chaque atome de carbone du terphényle et R₁, R₂, R₃ et R₄ ne contiennent pas plus de 16 atomes de carbone.

2. Composé tel que revendiqué dans la revendication 1, dans lequel le groupe représenté par R₁ ou R₂ est un groupe n-alkyle en C₁-C₁₆.

3. Composé tel que revendiqué dans la revendication 2, dans lequel le groupe représenté par R₁ ou R₂ est un groupe n-alkyle en C₇-C₁₂.

4. Composé tel que revendiqué dans n'importe quelle revendication précédente, dans lequel le groupe représenté par R₃ ou R₄ est un groupe n-alkyle en C₁-C₁₆.

5. Composé tel que revendiqué dans la revendication 4, dans lequel le groupe représenté par R₃ ou R₄ est un groupe n-alkyle en C₁-C₅.

6. Composé tel que revendiqué dans n'importe quelle revendication précédente ayant la formule générale [2] ou la formule générale [3] : 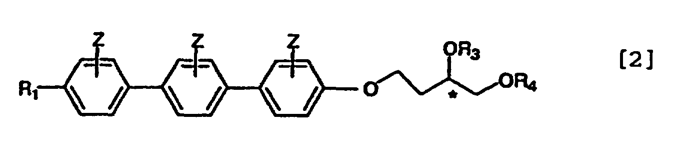 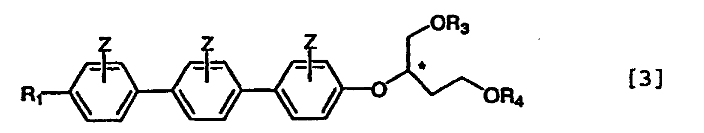 dans lesquelles R₁, R₃, R₄ et Z sont tels que définis ci-dessus.

7. Composé tel que revendiqué dans la revendication 6 ayant la formule générale [4] ou la formule générale [5] : 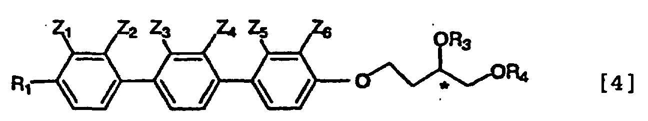 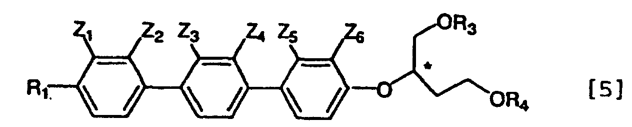 dans lesquelles R₁, R₃ et R₄ sont tels que définis ci-dessus, et soit
(i) l'un de Z₁ à Z₆ est F, les Z₁ à Z₆ restants étant H, soit
(ii) Z₁ et Z₂, ou Z₃ et Z₄, ou Z₅ et Z₆ sont F, les Z₁ à Z₆ restants étant H.

8. Composé tel que revendiqué dans n'importe quelle revendication précédente, dans lequel R₁ est n-octyle.

9. Composé tel que revendiqué dans n'importe quelle revendication précédente, dans lequel R₃ et R₄ sont indépendamment choisis parmi méthyle et n-pentyle.

10. Composé choisi dans le groupe suivant :
1,2-(S)-diméthoxy-(4''-octyloxyterphényl-4-oxy)butane,
1,2-(S)-dipentoxy-(4''-octyloxyterphényl-4-oxy)butane, 1-pentoxy-2-(S)-méthoxy-(4''-octyloxyterphényl-4-oxy)butane,
1,2-(S)-dipentoxy-(4''-octyloxyterphényl-3-fluoro-4-oxy)butane,
1,2-(S)-dipentoxy-(4''-octyloxyterphényl-2-fluoro-4-oxy)butane,
1,2-(S)-dipentoxy-(2''-fluoro-4''-octyloxyterphényl-4-oxy)butane,
1,2-(S)-dipentoxy-(3''-fluoro-4''-octyloxyterphényl-4-oxy)butane,
1,2-(S)-dipentoxy-(2'-fluoro-4''-octyloxyterphényl-4-oxy)butane,
1,2-(S)-dipentoxy-(2'',3''-difluoro-4''-octyloxyterphényl-4-oxy) butane,
1,2-(S)-dipentoxy-(4''-octyloxyterphényl-2,3-difluoro-4-oxy)butane, et
1,2-(S)-dipentoxy-(2',3'-difluoro-4''-octyloxyterphényl-4-oxy)butane.

11. Composition à cristaux liquides contenant au moins un composé selon l'une quelconque des revendications 1 à 10.

12. Dispositif à cristaux liquides électro-optique comprenant une cellule à cristaux liquides ayant une couche d'une composition à cristaux liquides selon la revendication 11, et des moyens pour appliquer un champ électrique à travers ladite couche.
